# EUROPEAN PATENT APPLICATION

(11) **EP 4 465 159 A1**
(43) Date of publication of application: **20.11.2024**
(21) Application number: 23795159.5
(22) Date of filing: 19.04.2023
(51) Int. Cl.: G06F 3/0484

(54) **EXERCISE GUIDANCE METHOD AND ELECTRONIC DEVICE**

(30) Priority: 24.04.2022 CN 202210435708
(71) Applicant: Huawei Technologies Co., Ltd., Shenzhen, Guangdong 518129 (CN)
(72) Inventor: JIANG, Jin, Shenzhen, Guangdong 518129 (CN); DIAO, Jun, Shenzhen, Guangdong 518129 (CN)
(74) Representative: Gill Jennings & Every LLP
(86) International application number: PCT/CN2023/089312
(87) International publication number: WO 2023/207726

(57) **Abstract**

An exercise guidance method and an electronic device are disclosed, and relate to the field of terminal technologies, so that a plurality of performance ranges can be recommended to a user, to effectively provide exercise guidance for the user. The method is applied to an electronic device, and the method includes: receiving a first operation input by a user, and displaying a first interface including information about a first exercise ability of the user; receiving a second operation input by the user, and displaying a second interface including one or more movement distances; and receiving a target movement distance input by the user on the second interface, and displaying a third interface, where the third interface includes a plurality of exercise levels and an exercise performance range corresponding to each exercise level, and the exercise performance range is determined based on the corresponding exercise level, the target movement distance, and the first exercise ability of the user.

## Description

This application claims priority to Chinese Patent Application No. 202210435708.4, filed with the China National Intellectual Property Administration on April 24, 2022 and entitled "EXERCISE GUIDANCE METHOD AND ELECTRONIC DEVICE", which is incorporated herein by reference in its entirety.

### TECHNICAL FIELD

This application relates to the field of terminal technologies, and in particular, to an exercise guidance method and an electronic device.

### BACKGROUND

At present, more users pay attention to physical exercise. In some schemes, a user may wear a wearable device such as a watch or a band, and collect exercise parameters of the user through the wearable device. For example, a heart rate and a step quantity during walking may be collected through a band. The user may learn about an exercise status of the user by using these exercise parameters.

In some high-intensity and professional exercise projects, relatively high exercise skills are usually required. It is difficult to provide targeted exercise guidance for the user by only recording the foregoing exercise parameters. As a result, various problems such as an injury and poor exercise performance occur in an exercise process of the user.

### SUMMARY

This application provides an exercise guidance method and an electronic device, to effectively guide a user to do exercise.

To achieve the foregoing objective, embodiments of this application provide the following technical solutions:

According to a first aspect, an exercise guidance method is provided. The method is applied to an electronic device, and the method includes:
receiving a first operation input by a user, and displaying a first interface, where the first interface includes information about a first exercise ability of the user;
receiving a second operation input by the user, and displaying a second interface, where the second interface includes one or more movement distances;
receiving a target movement distance input by the user on the second interface; and
displaying a third interface, where the third interface includes a plurality of exercise levels and an exercise performance range corresponding to each exercise level, and the exercise performance range is determined based on the corresponding exercise level, the target movement distance, and the first exercise ability.

In this solution, the electronic device may recommend a plurality of performance intervals to the user based on a stable exercise ability of the user in a period of time (the user may have exercised many times in this period of time). The performance interval is more in line with an exercise level of the user in this period of time, so that more accurate exercise guidance can be provided for the user. The user may select a performance interval of this exercise from the plurality of performance intervals. Because the performance interval selected by the user matches the exercise ability of the user, even some runners lacking exercise or race experience may perform intensity control properly in each stage of the exercise, thereby avoiding an overly aggressive or overly conservative race strategy.

In addition, a personalized exercise performance range is determined based on the exercise ability of the user, that is, the exercise performance range recommended by the electronic device for the user matches the ability of the user, so that the user is more likely to complete the exercise.

In a possible design, the method further includes: receiving a first target exercise level input by the user on the third interface, and displaying a first exercise interface, where the first target exercise level corresponds to a first exercise performance range.

In a possible design, after the displaying a first interface, the method further includes:
displaying a fourth interface, where the fourth interface includes a first control used to modify the first exercise ability; and
receiving an operation performed by the user on the first control, and displaying a fifth interface, where the fifth interface includes an option used to modify the first exercise ability.

In this way, the user can modify the exercise ability, so that the exercise ability is more accurate. Based on the accurate exercise ability, the electronic device can obtain a more accurate exercise performance range of a corresponding exercise level.

In a possible design, the first interface includes heart rate information, and the fourth interface includes a second control used to modify the heart rate information. In this way, the user can modify personal heart rate information, so that the exercise ability is more accurate. Based on the accurate exercise ability, the electronic device can obtain a more accurate exercise performance range of a corresponding exercise level.

In a possible design, the method further includes:
displaying a second exercise interface in response to a third operation input by the user, where the second exercise interface includes a current exercise parameter of the user, an annular indication region located on an interface edge, and a pointer; and the pointer points to the annular indication region, the annular indication region includes a first indication region, a second indication region, and a third indication region, a first end of the first indication region is connected to a second end of the second indication region, and a second end of the first indication region is connected to a first end of the third indication region, where
the first indication region includes an annular region corresponding to a first parameter range, and the first parameter range is a recommended range of the electronic device for the exercise parameter; and the second indication region includes an annular region greater than a maximum value of the first parameter range, the third indication region includes an annular region less than a minimum value of the first parameter range, and a location at which the pointer points to the annular indication region indicates whether the current exercise parameter of the user exceeds the first parameter range.

In this way, the user may quickly determine, based on a pointing direction of the pointer, that a current heart rate is too high or too low. Based on this, the user may reduce a pace or increase a pace, to successfully complete the exercise based on a specified performance goal.

In a possible design, when the current exercise parameter of the user exceeds the first parameter range, the pointer points to the second indication region or the third indication region, the second exercise interface further includes first prompt information, and the first prompt information is used to prompt the user that the current exercise parameter exceeds the first parameter range.

In this way, the electronic device may notify the user in a timely manner that the current exercise parameter is too high or too low, so that the user knows a current exercise status.

In a possible design, the second exercise interface further includes the first parameter range.

In this way, the user may determine, based on the first parameter range displayed on the second exercise interface, an exercise parameter range currently recommended by the electronic device.

In a possible design, the method further includes:
obtaining a first exercise parameter of the user in an exercise process; and
displaying a third exercise interface when the first exercise parameter exceeds the first parameter range for more than preset duration, where the third exercise interface is used to recommend the user to adjust a second exercise parameter.

It should be understood that, if an exercise parameter of the user exceeds a recommended value range for a short time, the electronic device may not present prompt information (for example, not display an interface used to prompt a pace adjustment, or not vibrate), to prevent ping-pong effect from frequently presenting prompt information to the user and disturbing the user.

In this way, the user may adjust the exercise parameter based on a suggestion provided by the electronic device, so that a physical status can be taken into account and a degree of exercise completion is higher.

In a possible design, the first exercise parameter includes a heart rate, and the second exercise parameter includes a pace; and
the displaying a third exercise interface when the first exercise parameter exceeds the first parameter range for more than preset duration includes:
displaying the third exercise interface when the heart rate is greater than the maximum value of the first parameter range, where the third exercise interface is used to recommend the user to reduce the pace; or
displaying the third exercise interface when the heart rate is less than the minimum value of the first parameter range, where the third exercise interface is used to recommend the user to increase the pace.

In this way, the suggestion provided by the electronic device is more in line with an actual situation of the user during the exercise, so that the suggestion obtained by the user is more specific and more operable.

In a possible design, the displaying a third exercise interface includes:
displaying the third exercise interface within first duration, and displaying a fifth exercise interface after the first duration, where user interface UI effect of the fifth exercise interface is different from UI effect of the third exercise interface.

In this way, during the exercise, after learning that the exercise parameter of the user exceeds the recommended value range, the user may leave the third exercise interface without performing any operation on the electronic device, and restore an original exercise interface of the user, which is more conducive to improving user experience.

In a possible design, after the displaying a third exercise interface, a sixth exercise interface is displayed in response to a fourth operation input by the user, where UI effect of the sixth exercise interface is different from UI effect of the third exercise interface.

In this way, during the exercise, after learning that the exercise parameter of the user exceeds the recommended value range, the user may control the electronic device, to enter an exercise interface expected by the user, thereby improving user experience.

In a possible design, the method further includes:
displaying a second exercise interface, where the second exercise interface includes a first parameter range, and the first parameter range is related to a first physical status of the user at a first moment in an exercise process; and
displaying a fourth exercise interface, where the fourth exercise interface includes a second parameter range, and the second parameter range is related to a second physical status of the user at a second moment in the exercise process, where the first parameter range is different from the second parameter range, and the first moment is different from the second moment.

In this solution, the electronic device dynamically monitors an exercise parameter of the user in real time, and may dynamically calculate a recommended parameter range based on a current physical status of the user, to provide real-time exercise guidance for the user.

In a possible design, the first physical status is determined based on an exercise parameter of the user at the first moment, and the second physical status is determined based on an exercise parameter of the user at the second moment.

In this way, exercise guidance is performed based on a real-time physical status of the user, so that the exercise guidance is more in line with the current physical status in the exercise, to avoid recommending too high or too low exercise intensity.

In a possible design, the exercise parameter includes a heart rate and/or a pace.

In a possible design, second prompt information is presented when a difference between the second parameter range and the first parameter range is greater than a threshold.

In this way, the electronic device prompts the user on the interface only when the recommended parameter range changes greatly, to prevent the user from adjusting exercise intensity improperly because the parameter range frequently changes.

In a possible design, the second exercise interface includes estimated duration for completing the target movement distance, and the estimated duration is determined based on the first physical status of the user, the target movement distance, and the first exercise performance range.

In the foregoing exercise guidance method, in the exercise process (for example, a running race) of the user, finish performance of the user may be dynamically estimated (the finish performance of the user may be updated in real time) based on an actual exercise parameter of the user (which may be used to represent a physical status of the user) and a preset performance goal. In some cases, if the physical status of the user is stable, the user may complete the current exercise based on the preset performance goal. In some other cases, if it is detected that the physical status of the user is unstable or a requirement of a specified goal is not met, the electronic device recommends, to the user based on the physical status of the user, a recommended parameter value range that is suitable for the current physical status and that minimizes a difference from the performance goal. In this way, finish performance that conforms to the physical status of the user can be obtained.

In a possible design, the second exercise interface includes one or more of the following: a current finished movement distance, current exercise duration, and a current exercise parameter.

In a possible design, the second exercise interface further includes a first icon, a second icon, and a progress bar, the first icon indicates, on the progress bar, a location of the current finished movement distance of the user in the target movement distance, and the second icon indicates, on the progress bar, a location that is of the user in the target movement distance and that is expected based on the first exercise performance range.

Through different progress bars, the user may intuitively learn about a current exercise progress and a distance difference between the user and a pacemaker. This helps the user adjust exercise intensity of the user based on the progress bar, to narrow the distance difference between the user and the pacemaker.

In a possible design, the method further includes:
displaying a sixth interface, where the sixth interface includes third prompt information, and the third prompt information is used to prompt the user to do exercise to obtain the first exercise ability.

In a possible design, the method further includes:
displaying a seventh interface, where the seventh interface includes the first target exercise level, and the first target exercise level corresponds to a second exercise performance range; the second exercise performance range is determined based on the first target exercise level, the target movement distance, and a second exercise ability of the user; and the second exercise performance range is different from the first exercise performance range, and the first exercise ability is different from the second exercise ability.

When the exercise ability of the user changes, the exercise performance range provided by the electronic device also changes accordingly, to provide exercise guidance that is more in line with an actual physical condition for the user.

In a possible design, the method further includes:
displaying a seventh exercise interface in the exercise process of the user in response to a fifth operation input by the user, where the seventh exercise interface includes a third control used to modify an exercise level. In a possible design, after the displaying a seventh exercise interface, the method further includes:
receiving an operation performed by the user on the third control, and displaying an eighth exercise interface, where the eighth exercise interface includes information about a plurality of exercise levels; and
receiving a second target exercise level input by the user on the eighth exercise interface, and displaying a ninth exercise interface, where the second target exercise level is different from the first target exercise level.

In this way, the electronic device allows the user to modify an exercise level in the exercise process, so that the user flexibly adjusts the current exercise.

In a possible design, after the receiving a first target exercise level input by the user on the third interface, the method further includes:
displaying fourth prompt information on the third interface, where the fourth prompt information is used to prompt the user to select a third target exercise level, and the third target exercise level is different from the first target exercise level.

When an exercise level selected by the user does not meet an exercise level obtained by the electronic device based on the exercise ability of the user, the user may be prompted to modify the exercise level, to provide more intuitive exercise guidance.

In a possible design, before the receiving a first operation input by a user, the method further includes:
displaying an eighth interface, where the eighth interface includes a plurality of exercise types; and
receiving a target exercise type input by the user on the eighth interface, and displaying a ninth interface, where
the receiving a first operation input by a user includes receiving the first operation that is input by the user and that is performed on the ninth interface.

In a possible design, the exercise includes any one of the following types of exercise: running, swimming, and cycling.

In a possible design, the exercise includes running, the first exercise ability is a running ability index, and the running ability index is related to an exercise record of the user.

In a possible design, the electronic device includes a wearable device or a mobile terminal device.

According to a second aspect, an exercise guidance apparatus is provided. The apparatus may be an electronic device or a component that can implement a function of the electronic device, and the apparatus includes:
an input unit, configured to receive a first operation input by a user; and
a display unit, configured to display a first interface, where the first interface includes information about a first exercise ability of the user, where
the input unit is further configured to receive a second operation input by the user;
the display unit is further configured to display a second interface, where the second interface includes one or more movement distances;
the input unit is further configured to receive a target movement distance input by the user on the second interface; and
the display unit is further configured to display a third interface, where the third interface includes a plurality of exercise levels and an exercise performance range corresponding to each exercise level, and the exercise performance range is determined based on the corresponding exercise level, the target movement distance, and the first exercise ability.

In a possible design, the input unit is further configured to receive a first target exercise level input by the user on the third interface; and
the display unit is further configured to display a first exercise interface, where the first target exercise level corresponds to a first exercise performance range.

In a possible design, the display unit is further configured to display a fourth interface, where the fourth interface includes a first control used to modify the first exercise ability;
the input unit is further configured to receive an operation performed by the user on the first control; and
the display unit is further configured to display a fifth interface, where the fifth interface includes an option used to modify the first exercise ability.

In a possible design, the first interface includes heart rate information, and the fourth interface includes a second control used to modify the heart rate information.

In a possible design, the display unit is further configured to display a second exercise interface in response to a third operation input by the user, where the second exercise interface includes a current exercise parameter of the user, an annular indication region located on an interface edge, and a pointer; and the pointer points to the annular indication region, the annular indication region includes a first indication region, a second indication region, and a third indication region, a first end of the first indication region is connected to a second end of the second indication region, and a second end of the first indication region is connected to a first end of the third indication region, where
the first indication region corresponds to a first parameter range; and the second indication region indicates a region greater than a maximum value of the first parameter range, the third indication region indicates a region less than a minimum value of the first parameter range, and a location at which the pointer points to the annular indication region indicates whether the current exercise parameter of the user exceeds the first parameter range.

In a possible design, the second exercise interface further includes first prompt information, the first prompt information is used to prompt the user that the current exercise parameter exceeds the first parameter range, and the pointer points to the second indication region or the third indication region.

In a possible design, the second exercise interface further includes the first parameter range.

In a possible design, the apparatus further includes a processing unit, configured to obtain a first exercise parameter of the user in an exercise process, where
the display unit is further configured to display a third exercise interface when the first exercise parameter exceeds the first parameter range for more than preset duration, where the third exercise interface is used to recommend the user to adjust a second exercise parameter.

In a possible design, the first exercise parameter includes a heart rate, and the second exercise parameter includes a pace; and
the displaying a third exercise interface when the first exercise parameter exceeds the first parameter range for more than preset duration includes:
displaying the third exercise interface when the heart rate is greater than the maximum value of the first parameter range, where the third exercise interface is used to recommend the user to reduce the pace; or
displaying the third exercise interface when the heart rate is less than the minimum value of the first parameter range, where the third exercise interface is used to recommend the user to increase the pace.

In a possible design, the displaying a third exercise interface includes:
displaying the third exercise interface within first duration, and displaying a fifth exercise interface after the first duration, where user interface UI effect of the fifth exercise interface is different from UI effect of the third exercise interface.

In a possible design, after the displaying a third exercise interface, a sixth exercise interface is displayed in response to a fourth operation input by the user, where UI effect of the sixth exercise interface is different from UI effect of the third exercise interface.

In a possible design, the display unit is further configured to display a second exercise interface, where the second exercise interface includes a first parameter range, and the first parameter range is related to a first physical status of the user at a first moment in an exercise process; and
the display unit is further configured to display a fourth exercise interface, where the fourth exercise interface includes a second parameter range, and the second parameter range is related to a second physical status of the user at a second moment in the exercise process, where the first parameter range is different from the second parameter range, and the first moment is different from the second moment.

In a possible design, the first physical status is determined based on an exercise parameter of the user at the first moment, and the second physical status is determined based on an exercise parameter of the user at the second moment.

In a possible design, the exercise parameter includes a heart rate and/or a pace.

In a possible design, the processing unit is further configured to control presentation of second prompt information when a difference between the second parameter range and the first parameter range is greater than a threshold.

In a possible design, the second exercise interface includes estimated duration for completing the target movement distance, and the estimated duration is determined based on the first physical status of the user, the target movement distance, and the first exercise performance range.

In a possible design, the second exercise interface includes one or more of the following: a current finished movement distance, current exercise duration, and a current exercise parameter.

In a possible design, the second exercise interface further includes a first icon, a second icon, and a progress bar, the first icon indicates, on the progress bar, a location of the current finished movement distance of the user in the target movement distance, and the second icon indicates, on the progress bar, a location that is of the user in the target movement distance and that is expected based on the first exercise performance range.

In a possible design, the display unit is further configured to display a sixth interface, where the sixth interface includes third prompt information, and the third prompt information is used to prompt the user to do exercise to obtain the first exercise ability.

In a possible design, the display unit is further configured to display a seventh interface, where the seventh interface includes the first target exercise level, and the first target exercise level corresponds to a second exercise performance range; the second exercise performance range is determined based on the first target exercise level, the target movement distance, and a second exercise ability of the user; and the second exercise performance range is different from the first exercise performance range, and the first exercise ability is different from the second exercise ability.

In a possible design, the displaying a third exercise interface includes:
displaying the third exercise interface within first duration, and displaying a fifth exercise interface after the first duration, where user interface UI effect of the fifth exercise interface is different from UI effect of the third exercise interface.

In a possible design, the display unit is further configured to display a sixth exercise interface in response to a fourth operation input by the user, where UI effect of the sixth exercise interface is different from UI effect of the third exercise interface.

In a possible design, the display unit is further configured to display a seventh exercise interface in the exercise process of the user in response to a fifth operation input by the user, where the seventh exercise interface includes a third control used to modify an exercise level.

In a possible design, the input unit is further configured to receive an operation performed by the user on the third control;
the display unit is further configured to display an eighth exercise interface, where the eighth exercise interface includes information about a plurality of exercise levels;
the input unit is further configured to receive a second target exercise level input by the user on the eighth exercise interface; and
the display unit is further configured to display a ninth exercise interface, where the second target exercise level is different from the first target exercise level.

In a possible design, the display unit is further configured to present fourth prompt information on the third interface, where the fourth prompt information is used to prompt the user to select a third target exercise level, and the third target exercise level is different from the first target exercise level.

In a possible design, the display unit is further configured to display an eighth interface, where the eighth interface includes a plurality of exercise types;
the input unit is further configured to receive a target exercise type input by the user on the eighth interface; and
the display unit is further configured to display a ninth interface, where
the receiving a first operation input by a user includes receiving the first operation that is input by the user and that is performed on the ninth interface.

In a possible design, the exercise includes any one of the following types of exercise: running, swimming, and cycling.

In a possible design, the exercise includes running, the first exercise ability is a running ability index, and the running ability index is related to an exercise record of the user.

In a possible design, the electronic device includes a wearable device or a mobile terminal device.

According to a third aspect, an embodiment of this application provides an exercise guidance apparatus. The apparatus has a function of implementing the method in any one of the foregoing aspects. The function may be implemented by hardware, or may be implemented by hardware executing corresponding software. The hardware or the software includes one or more modules corresponding to the foregoing function.

According to a fourth aspect, an apparatus is provided, including a processor and a memory. The memory is configured to store computer-executable instructions, and when the apparatus runs, the processor executes the computer-executable instructions stored in the memory, so that the apparatus performs the method in any one of the foregoing aspects.

According to a fifth aspect, an apparatus is provided, including a processor. The processor is coupled to a memory and is configured to: after reading instructions in the memory, perform the method in any one of the foregoing aspects according to the instructions.

According to a sixth aspect, a computer-readable storage medium is provided. The computer-readable storage medium stores instructions, and when the instructions are run on a computer, the computer may be enabled to perform the method in any one of the foregoing aspects.

According to a seventh aspect, a computer program product including instructions is provided. When the instructions are run on a computer, the computer may be enabled to perform the method in any one of the foregoing aspects.

According to an eighth aspect, a circuit system is provided. The circuit system includes a processing circuit, and the processing circuit is configured to perform the method in any one of the foregoing aspects.

According to a ninth aspect, a chip is provided. The chip includes a processor. The processor is coupled to a memory, and the memory stores program instructions, where when the program instructions stored in the memory are executed by the processor, the method in any one of the foregoing aspects is implemented.

According to a tenth aspect, a communication system is provided. The communication system includes the sending device in any one of the foregoing aspects and the receiving device in any one of the foregoing aspects.

For technical effects brought by any design manner of the second aspect to the tenth aspect, refer to the technical effects brought by different design manners of the first aspect. Details are not described herein again.

### BRIEF DESCRIPTION OF DRAWINGS

FIG. 1A is a schematic diagram of an architecture of a system according to an embodiment of this application;
FIG. 1B is a schematic diagram of an architecture of a system according to an embodiment of this application;
FIG. 1C is a schematic diagram of an architecture of a system according to an embodiment of this application;
FIG. 2 is a schematic diagram of a structure of an electronic device according to an embodiment of this application;
FIG. 3 is a schematic diagram of a structure of an electronic device according to an embodiment of this application;
FIG. 4 to FIG. 13(b) are interface diagrams according to embodiments of this application;
FIG. 14A is a schematic diagram of an exercise scenario according to an embodiment of this application;
FIG. 14B is a schematic diagram of a method for calculating a performance interval according to an embodiment of this application;
FIG. 15 and FIG. 16 are interface diagrams according to embodiments of this application;
FIG. 17 is a schematic diagram of a method for calculating a recommended parameter range according to an embodiment of this application;
FIG. 18 to FIG. 34 are interface diagrams according to embodiments of this application;
FIG. 35 and FIG. 36 are schematic diagrams of a prompt interval according to embodiments of this application;
FIG. 37 to FIG. 39 are interface diagrams according to embodiments of this application;
FIG. 40 is a schematic diagram of a method for calculating predicted finish performance according to an embodiment of this application;
FIG. 41 to FIG. 45 are interface diagrams according to embodiments of this application;
FIG. 46 is a flowchart of an exercise guidance method according to an embodiment of this application; and
FIG. 47 is a structural diagram of an apparatus according to an embodiment of this application.

### DESCRIPTION OF EMBODIMENTS

In embodiments of this application, the term "example" or "for example" is used to represent giving an example, an illustration, or a description. Any embodiment or design scheme described as an "example" or "for example" in embodiments of this application should not be explained as being more preferred or having more advantages than another embodiment or design scheme. Exactly, use of the term such as "example" or "for example" is intended to present a relative concept in a specific manner.

In embodiments of this application, "at least one" means one or more, and "a plurality of" means two or more. The term "and/or" describes an association relationship between associated objects and represents that three relationships may exist. For example, A and/or B may represent the following three cases: Only A exists, both A and B exist, and only B exists. A and B may be singular or plural. In the descriptions of this application, the character "/" usually indicates an "or" relationship between the associated objects.

Implementations of the embodiments of this application are described below in detail with reference to the accompanying drawings.

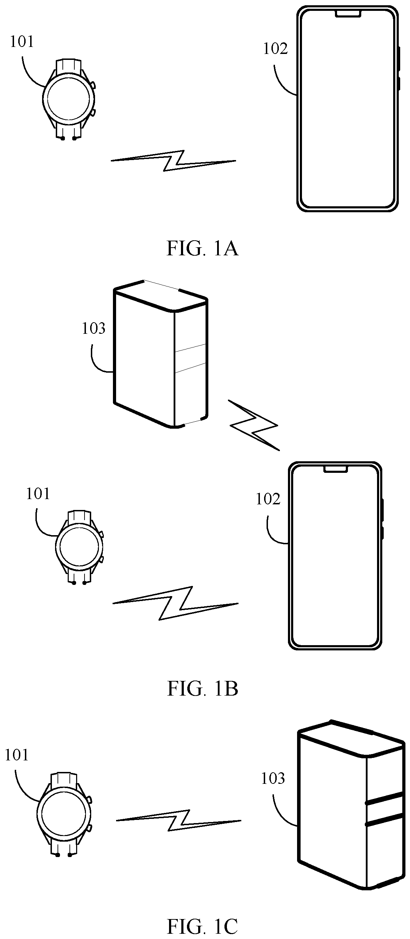

FIG. 1A is an example diagram of an architecture of a system to which embodiments of this application can be applied. As shown in FIG. 1A, the system includes a first electronic device 102 and a second electronic device 101.

The first electronic device 102 may be connected to the second electronic device 101 in a wireless manner or a wired manner. A wired or wireless connection manner is not limited. In FIG. 1A, for example, the first electronic device 102 is a mobile phone and the second electronic device 101 is a wearable device. A Bluetooth connection may be established between the mobile phone 102 and the wearable device 101, and communication may be performed based on the established Bluetooth connection.

In an embodiment, a user may wear the second electronic device 101 (or referred to as a wearable electronic device 101) at parts such as a wrist and/or an ankle in an exercise process. A sensor (for example, but not limited to an acceleration sensor, a gyroscope, a magnetometer, or a photoplethysmogram (photo plethysmo graphic, PPG) sensor) in the second electronic device 101 may obtain an exercise parameter of the user in the exercise process in real time, for example, but not limited to a step quantity, a stride length, a stride rate, a distance, time, a pace, a heart rate, a body temperature, blood oxygen, blood pressure, a heart rate variability (heart rate variability, HRV), a respiratory rate, and training load (or referred to as exercise load).

In another embodiment, in an exercise process, a user may alternatively wear or hold the mobile phone 102 to collect statistics on an exercise parameter. A sensor (for example, but not limited to an acceleration sensor, a gyroscope, or a magnetometer) in the mobile phone 102 may obtain an exercise parameter of the user in the exercise process in real time.

An exercise application may be installed in both the first electronic device 102 and the second electronic device 101. The user may configure the second electronic device 101 through the exercise application. Optionally, an exercise parameter of the user may be alternatively collected by the exercise application, to calculate a recommended heart rate range, an exercise ability, and the like by using the exercise parameter.

In some embodiments, FIG. 1B shows an architecture of another system to which technical solutions in embodiments of this application are applicable. The system may include a first electronic device 102, a second electronic device 101, and a cloud server 103. The first electronic device 102 may further upload the exercise parameter to the cloud server 103, to implement functions such as exercise parameter recording and processing.

FIG. 1C is a schematic diagram of another scenario to which embodiments of this application can be applied. Referring to FIG. 1C, the application scenario includes a second electronic device 101. The second electronic device 101 may collect an exercise parameter of a user through a sensor in the second electronic device 101, and may upload the exercise parameter to the cloud server 103, to implement functions such as exercise parameter recording and processing, and exercise ability calculation.

In some other embodiments, FIG. 1A, FIG. 1B, and FIG. 1C may further include another device and/or function component. A specific architecture of the system is not limited in the embodiments of this application.

In some embodiments, the first electronic device 102 may be a mobile terminal device, for example, a cellphone (or referred to as a "cellular" phone or a mobile phone (mobile phone)), a computer, or a data card, for example, may be a portable, pocket-sized, handheld, computer built-in, or in-vehicle mobile apparatus, which exchanges language and/or data with a radio access network, for example, a personal communication service (personal communication service, PCS) phone, a cordless phone, a personal digital assistant (personal digital assistant, PDA), or a tablet computer (Pad). The first electronic device 102 may be alternatively user equipment (user equipment, UE), a mobile terminal (mobile terminal, MT), or the like.

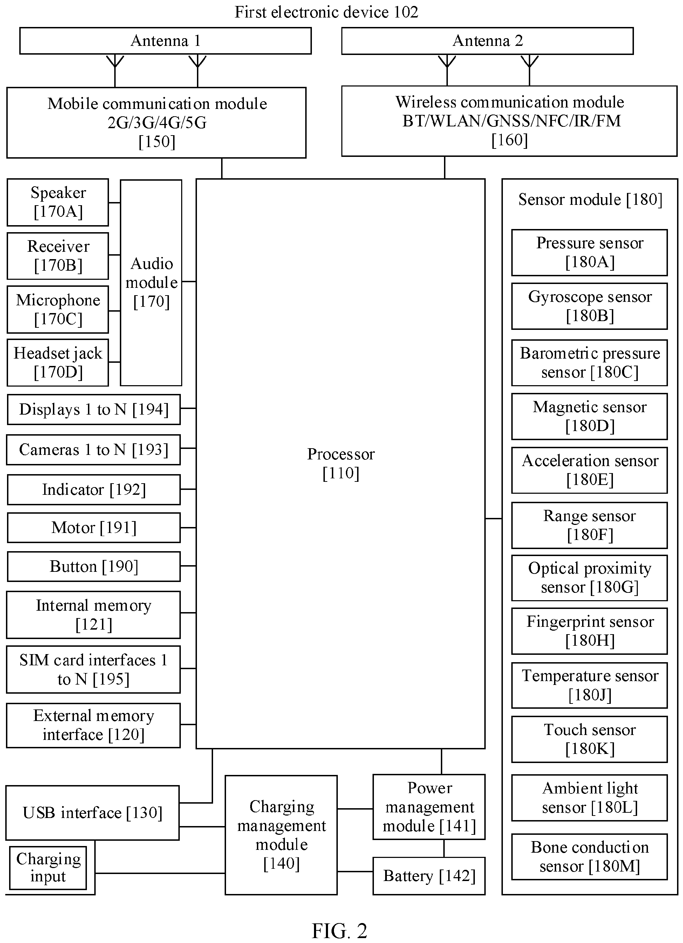

For example, the first electronic device 102 is a mobile phone. In this embodiment, a structure of the first electronic device 102 may be shown in FIG. 2. The first electronic device may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a sensor module 180, a button 190, a motor 191, an indicator 192, a camera 193, a display 194, a subscriber identity module (subscriber identity module, SIM) card interface 195, a video module 196 (not shown in FIG. 2), and the like.

It may be understood that the structure shown in this embodiment of this application does not constitute a specific limitation on the electronic device. In some other embodiments of this application, the electronic device may include more or fewer components than those shown in the figure, or combine some components, or split some components, or have a different component arrangement. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on an instruction operation code and a time sequence signal, to control instruction reading and instruction execution.

A memory may be further disposed in the processor 110, and is configured to store instructions and data. In some embodiments, the memory in the processor 110 is a cache. The memory may store instructions or data that has been used or cyclically used by the processor 110. If the processor 110 needs to use the instructions or the data again, the processor may directly invoke the instructions or the data from the memory. This avoids repeated access, reduces a waiting time period of the processor 110, and improves system efficiency.

In some embodiments, the processor 110 may include one or more interfaces. The interface may include an integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronization serial bus, and includes a serial data line (serial data line, SDA) and a serial clock line (serial clock line, SCL). In some embodiments, the processor 110 may include a plurality of groups of I2C buses. The processor 110 may be separately coupled to a touch sensor, a charger, a flash, the camera 193, and the like through different I2C bus interfaces. For example, the processor 110 may be coupled to the touch sensor through the I2C interface, so that the processor 110 communicates with the touch sensor through the I2C bus interface, to implement a touch function of the electronic device.

The MIPI interface may be configured to connect the processor 110 to a peripheral component such as the display 194 or the camera 193. The MIPI interface includes a camera serial interface (camera serial interface, CSI), a display serial interface (display serial interface, DSI), and the like. In some embodiments, the processor 110 communicates with the camera 193 through the CSI interface, to implement a photographing function of the electronic device. The processor 110 communicates with the display 194 through the DSIinterface, to implement a display function of the electronic device.

The USB interface 130 is an interface that conforms to a USB standard specification, and may be specifically a mini USB interface, a micro USB interface, a USB type-C interface, or the like. The USB interface 130 may be configured to connect to a charger to charge the electronic device, may be configured to transmit data between the electronic device and a peripheral device, or may be configured to connect to a headset for playing audio through the headset. The interface may be configured to connect to another electronic device such as an AR device.

It may be understood that, an interface connection relationship between the modules shown in this embodiment of this application is merely an example for description, and does not constitute a limitation on the structure of the electronic device. In some other embodiments of this application, the electronic device may alternatively use an interface connection manner different from that in the foregoing embodiment, or a combination of a plurality of interface connection manners.

The charging management module 140 is configured to receive a charging input from a charger. The charger may be a wireless charger or a wired charger. In some embodiments of wired charging, the charging management module 140 may receive a charging input of a wired charger through the USB interface 130. In some embodiments of wireless charging, the charging management module 140 may receive a wireless charging input through a wireless charging coil of the electronic device. The charging management module 140 may further supply power to the electronic device by using the power management module 141 when the battery 142 is charged.

The power management module 141 is configured to connect to the battery 142, the charging management module 140, and the processor 110. The power management module 141 receives an input of the battery 142 and/or the charging management module 140, and supplies power to the processor 110, the internal memory 121, the display 194, the camera 193, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same component.

A wireless communication function of the electronic device may be implemented by using the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna of the electronic device may be configured to cover one or more communication frequency bands. Different antennas may be multiplexed to improve antenna utilization. For example, the antenna 1 may be multiplexed as a diversity antenna in a wireless local area network. In some other embodiments, the antennas may be used in combination with a tuning switch.

The mobile communication module 150 may provide a wireless communication solution that is applied to the electronic device and that includes 2G/3G/4G/5G and the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave through the antenna 1 for radiation. In some embodiments, at least some function modules of the mobile communication module 150 may be disposed in the processor 110. In some embodiments, at least some function modules of the mobile communication module 150 may be disposed in a same device with at least some modules of the processor 110.

The modem processor may include a modulator and a demodulator. The modulator is configured to modulate a to-be-sent low-frequency baseband signal into a medium-high frequency signal. The demodulator is configured to demodulate the received electromagnetic wave signal into a low-frequency baseband signal. Then, the demodulator transmits the low-frequency baseband signal obtained through demodulation to the baseband processor for processing. After being processed by the baseband processor, the low-frequency baseband signal is transmitted to the application processor. The application processor outputs a sound signal by using an audio device, or displays an image or a video by using the display 194. In some embodiments, the modem processor may be an independent device. In some other embodiments, the modem processor may be independent of the processor 110, and is disposed in a same device as the mobile communication module 150 or another function module.

The wireless communication module 160 may provide a wireless communication solution that is applied to the electronic device and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The wireless communication module 160 may be one or more components integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the to-be-sent signal, and convert the signal into an electromagnetic wave through the antenna 2 for radiation.

In some embodiments, in the electronic device, the antenna 1 and the mobile communication module 150 are coupled, and the antenna 2 and the wireless communication module 160 are coupled, so that the electronic device can communicate with a network and another device by using a wireless communication technology. The wireless communication technology may include a global system for mobile communication (global system for mobile communication, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

In this embodiment of this application, the electronic device may perform positioning by using a GPS module, or perform positioning in another manner. A module used for positioning, such as a GPS, may be referred to as a positioning module.

In some embodiments of this application, the electronic device may further include a heart rate module, configured to measure a real-time heart rate of a user.

The electronic device implements a display function by using the GPU, the display 194, the application processor, and the like. The GPU is a microprocessor for image processing, and is connected to the display 194 and the application processor. The GPU is configured to perform mathematical and geometric computation, and render an image. The processor 110 may include one or more GPUs, and the GPUs execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, the electronic device may include one or N displays 194, where N is a positive integer greater than 1.

The camera 193 is configured to capture a static image or a video. An object generates, through a lens, an optical image to be projected to a photosensitive element. The photosensitive element may be a charge coupled device (charge coupled device, CCD) or a complementary metal-oxide-semiconductor (complementary metal-oxide-semiconductor, CMOS) optoelectronic transistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into a standard image signal in a format such as RGB or YUV. In some embodiments, the electronic device may include one or N cameras 193, where N is a positive integer greater than 1.

The external memory interface 120 may be configured to be connected to an external storage card, for example, a micro SD card, to extend a storage capability of the electronic device. The external memory card communicates with the processor 110 by using the external memory interface 120, to implement a data storage function. For example, files such as music and a video are stored in the external storage card.

The internal memory 121 may be configured to store computer-executable program code. The executable program code includes instructions. The internal memory 121 may include a program storage region and a data storage region. The program storage region may store an operating system, an application required by at least one function (for example, a voice play function or an image play function), and the like. The data storage region may store data (such as audio data and a phone book) created when the electronic device is used, and the like. In addition, the internal memory 121 may include a high-speed random access memory, and may further include a nonvolatile memory, for example, at least one magnetic disk storage device, a flash memory device, or a universal flash storage (universal flash storage, UFS). The processor 110 runs the instructions stored in the internal memory 121 and/or the instructions stored in the memory disposed in the processor, to execute various function applications of the electronic device and data processing.

The audio module 170 is configured to convert digital audio information into an analog audio signal for output, and is also configured to convert an analog audio input into a digital audio signal. The audio module 170 may be further configured to encode and decode an audio signal. In some embodiments, the audio module 170 may be disposed in the processor 110, or some function modules in the audio module 170 are disposed in the processor 110. The electronic device may use the audio module 170, for example, music playing or recording. The audio module 170 may include a speaker, a receiver, a microphone, a headset interface, an application processor, and the like to implement an audio function.

In some embodiments of this application, the video module 196 is configured to process a video frame. The processing includes, but is not limited to, encoding and/or decoding of a video frame. Optionally, the video module 196 may be implemented as a video encoder and/or a video decoder in the electronic device.

The sensor module 180 may include a pressure sensor, a gyroscope sensor, a barometric pressure sensor, a magnetic sensor, an acceleration sensor, a range sensor, an optical proximity sensor, a fingerprint sensor, a temperature sensor, a touch sensor, an ambient light sensor, a bone conduction sensor, and the like.

The button 190 includes a power button, a volume button, and the like. The button 190 may be a mechanical button, or may be a touch button. The electronic device may receive a button input, and generate a button signal input related to user settings and function control of the electronic device.

The motor 191 may generate a vibration prompt. The motor 191 may be configured to provide an incoming call vibration prompt or a touch vibration feedback. For example, touch operations performed on different applications (for example, photographing and audio playing) may correspond to different vibration feedback effect. The motor 191 may also correspond to different vibration feedback effect for touch operations performed on different regions of the display 194. Different application scenarios (for example, a time reminder, information receiving, an alarm clock, and a game) may also correspond to different vibration feedback effect. A touch vibration feedback effect may be customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device. The electronic device may support one or N SIM card interfaces, where N is a positive integer greater than 1.

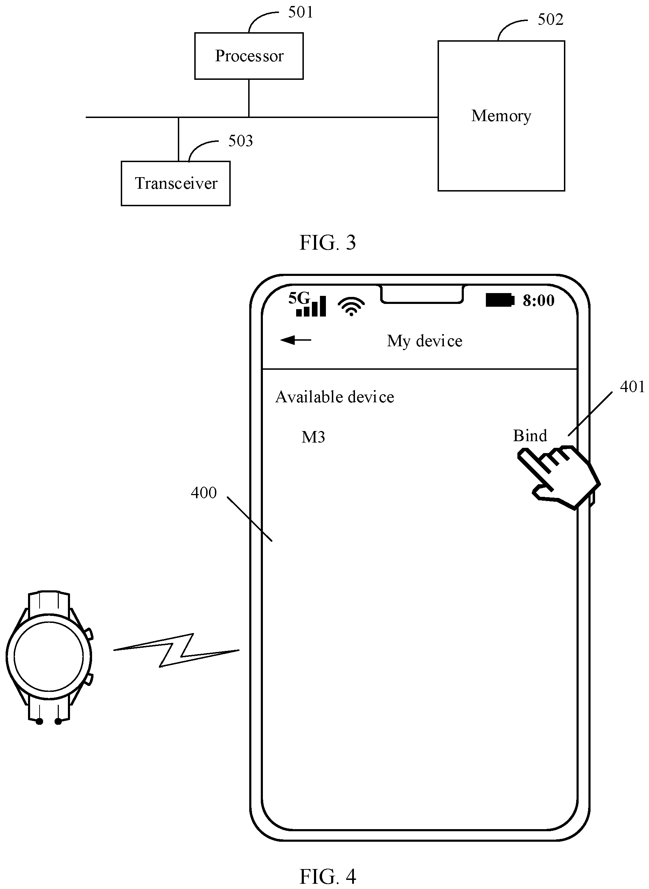

For example, FIG. 3 shows another example structure of a first electronic device. As shown in FIG. 3, the first electronic device includes a processor 501, a memory 502, and a transceiver 503. For implementation of the processor 501 and the memory 502, refer to implementation of the processor and the memory of the first electronic device shown in FIG. 3. The transceiver 503 is configured to interact between the first electronic device and another device. The transceiver 503 may be a device based on Wi-Fi, Bluetooth, or another communication protocol.

For a structure of the second electronic device, refer to the structure shown in FIG. 3. For example, there are more or fewer components than the structure shown in FIG. 3, or there is a different component layout. Alternatively, in some other examples, the second electronic device may have fewer components than the structure shown in FIG. 2, or may have a different component layout. A specific structure of the second electronic device is not limited in this embodiment of this application.

FIG. 3 and FIG. 2 are merely possible examples of the structure of the electronic device (including but not limited to the first electronic device and the second electronic device), and do not constitute a limitation on the structure of the electronic device.

In the embodiments of this application, in an exercise process of the user, the electronic device may perform positioning by using a positioning module (located in this device or another device), determine a location of the user based on a positioning result, and determine a movement distance and an exercise pace of the user based on the location of the user. The electronic device may further measure a heart rate of the user by using a heart rate module (located in this device or another device). In some examples, if it is determined that a real-time pace of the user exceeds a recommended pace range, the electronic device may dynamically present prompt information to the user, so that the user adjusts exercise intensity based on the prompt information, to achieve desired exercise effect. In some examples, if it is determined that a real-time heart rate of the user exceeds a recommended heart rate range, the electronic device may present prompt information to the user, so that the user adjusts exercise intensity based on the prompt information. This process may be referred to as a process of executing an exercise strategy.

An example in which the first electronic device is a mobile phone and the second electronic device is a smartwatch is used below to describe an exercise guidance method provided in the embodiments of this application.

An exercise application may be installed in the mobile phone. The exercise application may be an original system application or a third-party application. A specific form and a name of the application are not limited in the embodiments of this application.

For example, to enable the mobile phone to communicate with the watch, a communication connection needs to be established between the mobile phone and the watch. Specifically, FIG. 4 is a schematic diagram of establishing a Bluetooth communication connection between the mobile phone and the watch. As shown in FIG. 4, the mobile phone displays a user interface 400. Optionally, the user interface 400 may be a specified interface in the exercise application. The user interface 400 includes a list of "available devices" currently scanned by the mobile phone. The user may tap a "bind" option 401, so that a Bluetooth communication connection is established between the mobile phone and the watch (that is, M3, where M3 is a device name such as a default model of the watch at delivery).

It should be noted that communication between the mobile phone and the watch is not limited to Bluetooth communication. For example, the communication may be another wired or wireless connection manner such as near field communication (near Field Communication, NFC) or wireless fidelity (Wireless Fidelity, Wi-Fi). This is not limited in the embodiments of this application.

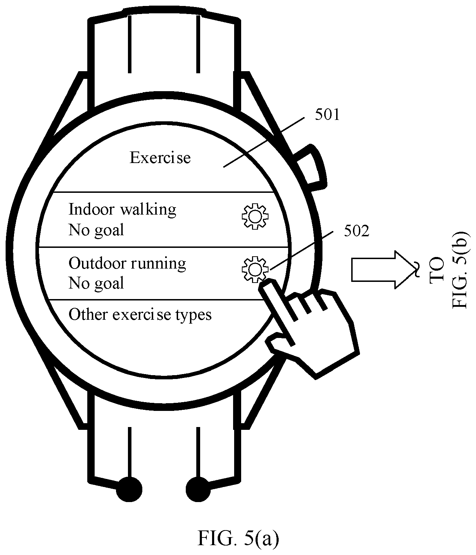

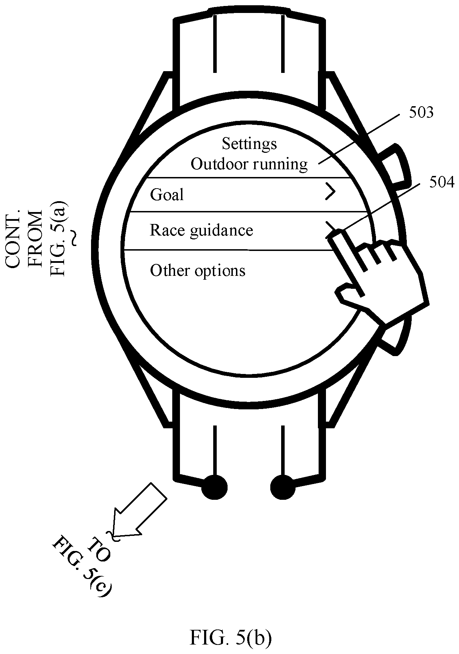

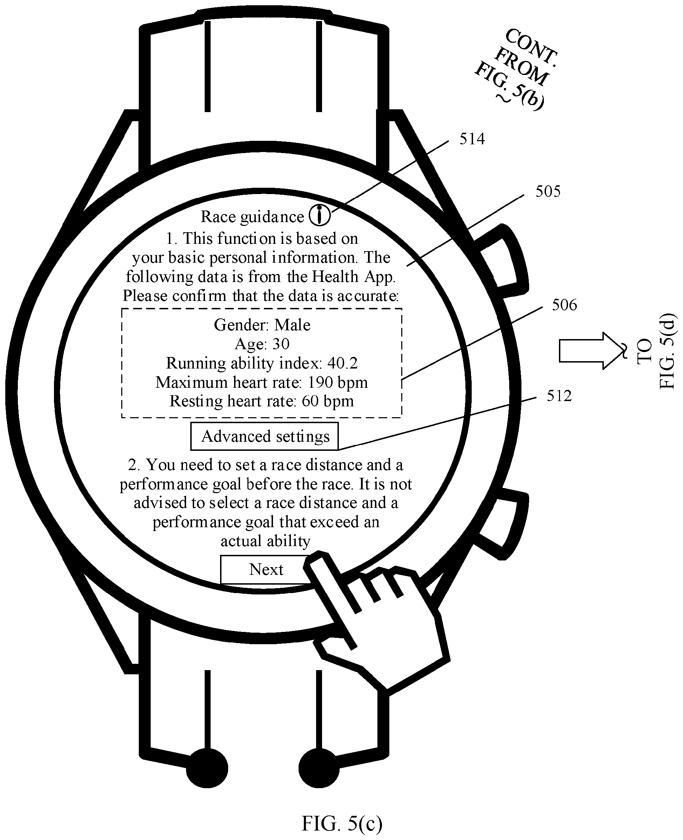

For example, as shown in FIG. 5(a), the watch displays an interface 501, and the interface 501 includes a setting option 502. After detecting an operation, for example, tapping the setting option 502 by the user, the watch may jump to a setting interface 503 of "Running" shown in FIG. 5(b). The interface 503 may include a "race guidance" option 504. After detecting that the user taps the race guidance option 504, the watch may jump to an interface 505 shown in FIG. 5(c).

The interface 505 includes a first parameter 506 of the user, and the first parameter includes but is not limited to a gender, an age, a running ability index, a maximum heart rate, and a resting heart rate. The running ability index may be used to represent a running ability, and may also be referred to as another name such as a "running ability value" or a running ability index. In some examples, a higher value of the running ability index indicates a stronger running ability, and on the contrary, a lower value of the running ability index indicates a weaker running ability. Alternatively, in some other examples, a higher value of the running ability index indicates a weaker running ability, and on the contrary, a lower value of the running ability index indicates a stronger running ability. The first parameter may be used to calculate an exercise goal.

The first parameter has a corresponding parameter value range. Optionally, a value range of the running ability index may be but is not limited to 20.0 to 89.9. Optionally, the running ability index in the watch may be obtained from another device.

Optionally, a value range of the maximum heart rate may be but is not limited to 155 to 220 bpm. In an example, if the watch does not obtain the maximum heart rate, a value of the maximum heart rate may be 220 to x, where x indicates the age of the user.

Optionally, a value range of the resting heart rate may be but is not limited to 30 to 110 bpm. In some examples, if the electronic device does not obtain the resting heart rate, the resting heart rate may be a preset value (for example, 60 bpm) by default.

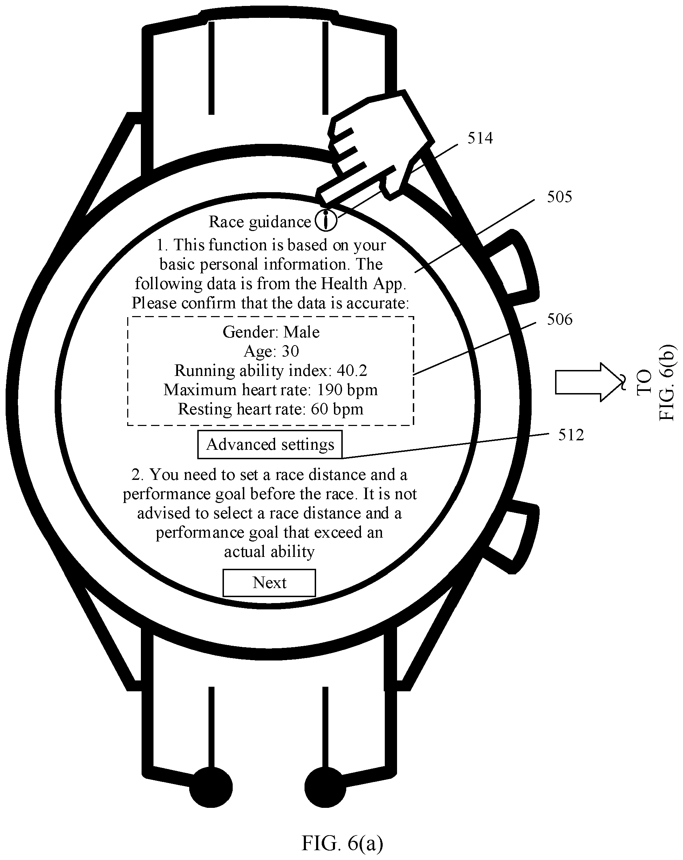

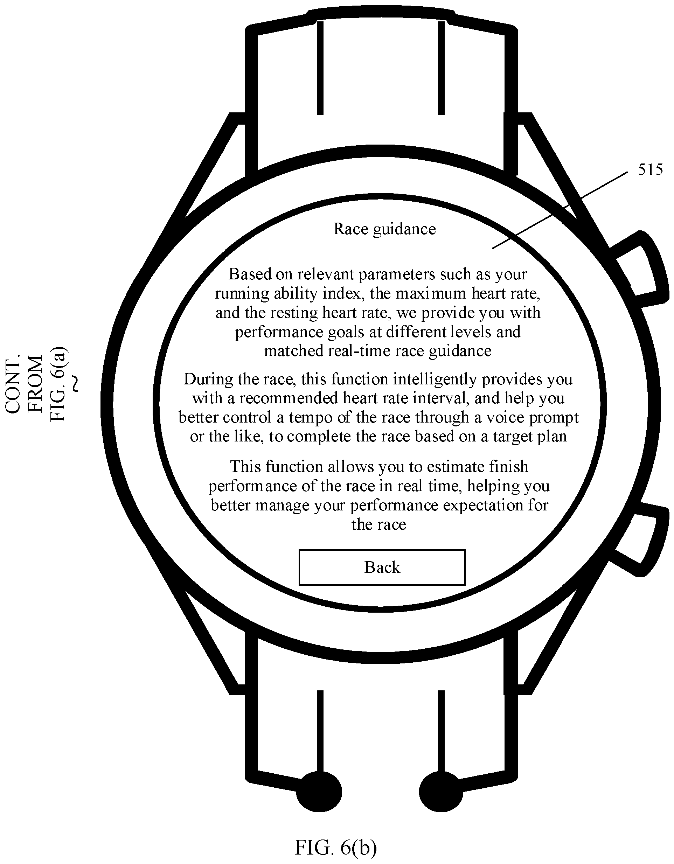

Optionally, the interface 505 may further include a control 514. As shown in FIG. 6(a), when detecting that the user taps the control 514, the watch may jump to an interface 515 shown in FIG. 6(b), to browse details of a "race guidance" function.

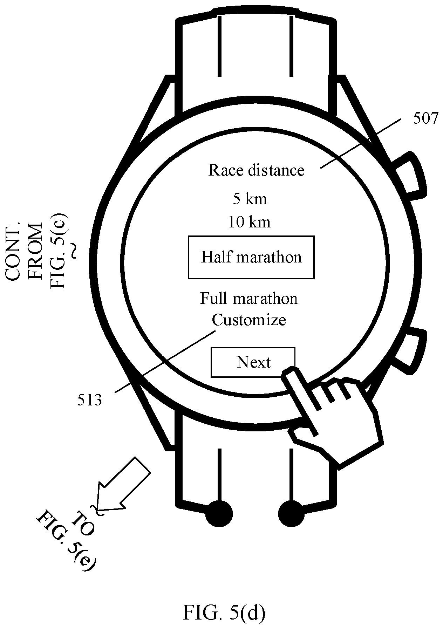

In some scenarios, if the user determines that the first parameter 506 displayed on the interface 505 is accurate, the user may tap a "next" option on the interface 505. After detecting the operation of the user, the watch may jump to an interface 507 shown in FIG. 5(d). The interface 507 may display different race distances. The user may set a race distance through the interface 507. For example, the interface 507 may display four race distances: 5 km, 10 km, a half marathon, and a full marathon, and the user may select this race as a "half marathon".

Optionally, the interface 507 may further include a custom option 513 that is used by the user to customize a race distance. For example, the customized race distance is within a range of 3 to 42 km. In a possible implementation, after the user taps the custom option 513, a race distance setting similar to a setting interface shown on an interface 603 is accessed, and one or more adjustable options are displayed for the user to set a race distance. In another possible implementation, the interface 507 does not display options such as "5 km", "10 km", "half marathon", and "full marathon", but displays only one or more adjustable options for setting a race distance, so that the user sets a desired race distance.

Alternatively, in some other scenarios, if the watch does not obtain a part of parameters in the first parameter 506, the electronic device may prompt the user to set this part of parameters.

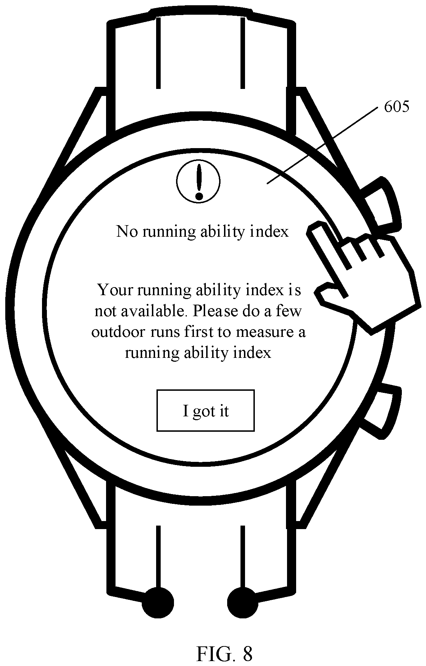

For example, as shown in FIG. 8, if the watch does not obtain a running ability index, the watch may display an interface 605, to prompt the user to run first to measure the running ability index.

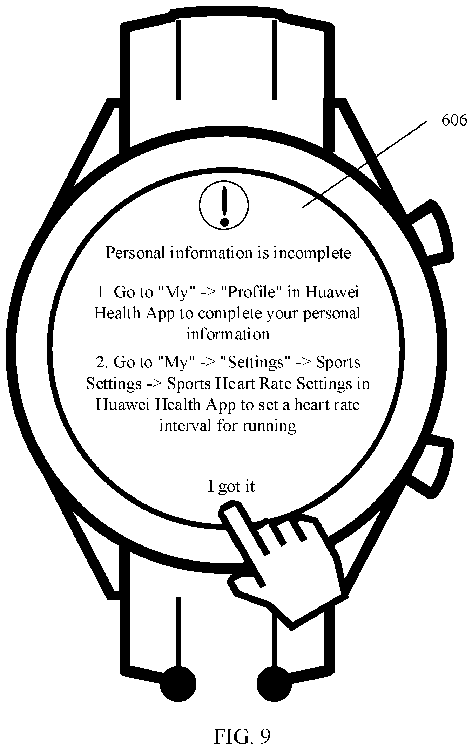

For another example, as shown in FIG. 9, if the watch does not obtain a gender, an age, or a heart rate, the watch may display an interface 606, to prompt the user to set this part of parameters.

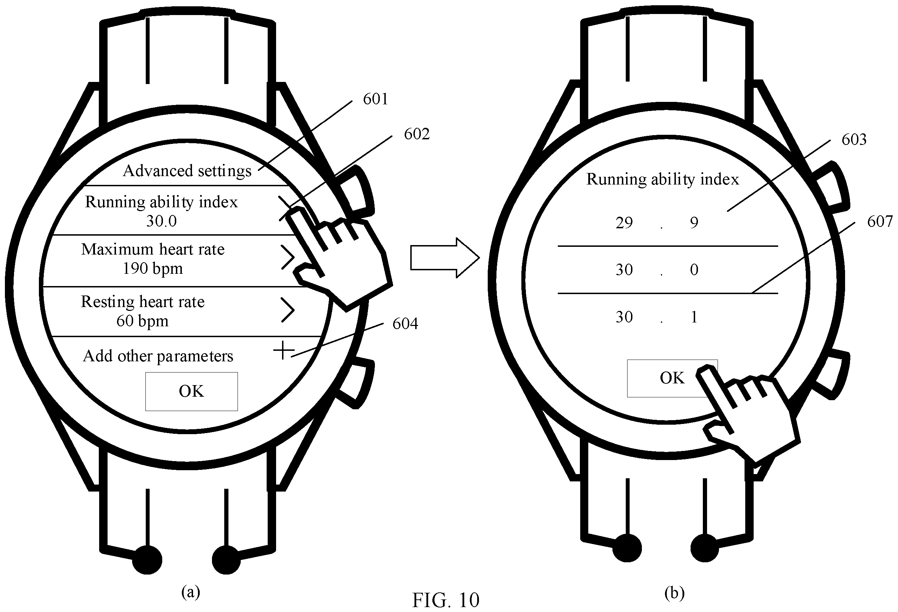

Alternatively, in some other scenarios, if the user determines that the first parameter 506 displayed on the interface 505 is inaccurate, or the user wants to set the first parameter, the user may tap an "advanced setting" option 512 on the interface 505. After detecting the operation of the user, the watch may jump to an interface 601 shown in (a) in FIG. 10. The interface includes setting options of a part or all of parameters in the first parameter, for example, setting options of the running ability index, the maximum heart rate, and the resting heart rate. Optionally, the interface 601 may further include a control 604, and the user may add and set another parameter by using control 604.

For example, after detecting that the user taps a setting option 602 of the running ability index, the watch may jump to an interface 603 shown in (b) in FIG. 10. The user may set a value of the running ability index through the interface 603. For example, after detecting an operation of tapping an "OK" option by the user on the interface 603, the watch may jump back to the interface 601. The user may set other parameters (for example, the maximum heart rate and the resting heart rate) through the interface 601. After completing the setting on the interface 601, the user may tap an "OK" option, and after detecting the operation of the user, the watch may jump to the interface shown in FIG. 5(d), so that the user sets a race distance. Alternatively, after completing the setting on the interface 601, the user may tap an "OK" option, and after detecting the operation of the user, the watch may jump to the interface 505 shown in FIG. 5(c), so that the user can determine, through the interface 505, whether the parameter value is successfully modified. Then, after detecting that the user taps the "next" button on the interface 505, the watch jumps to the interface 507 shown in FIG. 5(d).

It should be noted that, in this embodiment of this application, a manner of switching to a specified interface is not limited. The user may trigger, through one operation instruction, the electronic device to switch from a current interface to a target interface, or the user may trigger, through a plurality of operation instructions, the electronic device to switch from a current interface to a target interface. For example, for switching from the interface 601 shown in (a) in FIG. 10 to the interface 507 shown in FIG. 5(d), the user may tap the "OK" button on the interface 601 to trigger the interface switching. Alternatively, the user may first tap the "OK" button on the interface 601 shown in (a) in FIG. 10, to trigger the electronic device to first switch the interface 601 to the interface 505 shown in FIG. 5(c), and when detecting that the user taps the "next" button on the interface 505, the electronic device switches the interface 505 to the target interface 507 shown in FIG. 5(d).

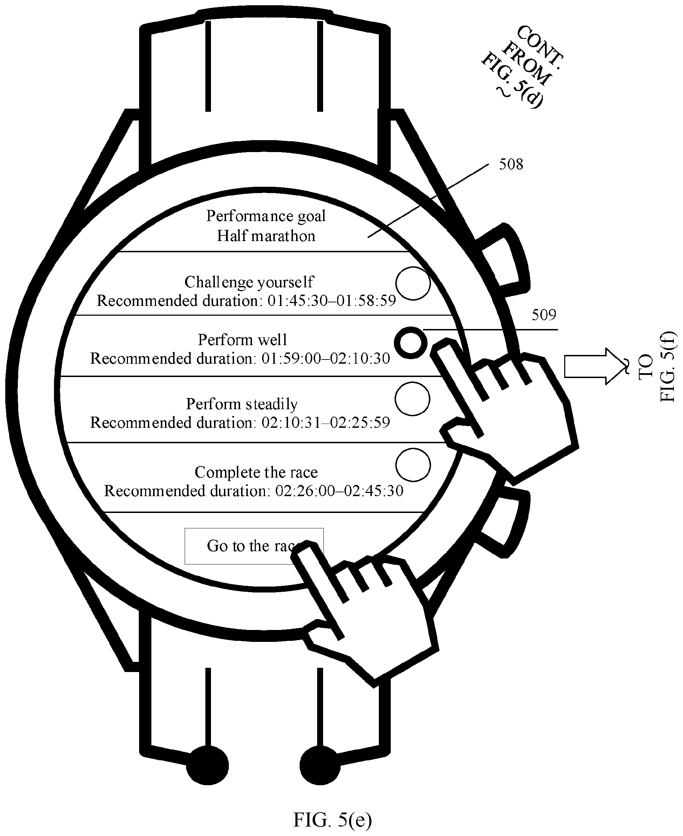

Then, on the interface 507 shown in FIG. 5(d), after the user sets a race distance, if the watch detects an operation of tapping the "next" option by the user, the watch jumps to an interface 508 shown in FIG. 5(e). The interface 508 may be used to set an exercise strategy, for example, set an exercise level.

For example, the interface 508 shown in FIG. 5(e) includes four exercise levels: challenge yourself, perform well, perform steadily, and complete the race. Each exercise level corresponds to one segment of recommended duration, that is, time for completing exercise at this level. Different levels of exercise usually have different intensity, difficulty, and time. For example, as shown in FIG. 5(e), the watch recommends that time for the user to complete a half marathon at a level "challenge yourself" is 01:45:30 to 01:58:59, and recommends that time for the user to complete a half marathon at a level "perform well" is 01:59:00 to 02:10:30. The half marathon at the level "challenge yourself" takes less time and is more intense than the half marathon at the level "perform well".

That the watch calculates recommended duration for exercise at a specified level may be: calculating the recommended duration for the exercise at the specified level based on the first parameter. For example, if a performance goal (that is, an exercise level) is "challenge yourself", a marathon at this level is the most difficult and takes the shortest time in the foregoing levels. The watch may predict, based on a highest running ability index (assuming that the highest running ability index corresponds to a strongest running ability) and the maximum heart rate of the user, shortest time for the user to complete the marathon at this level.

It should be noted that in FIG. 5(e), classification of four exercise levels is used as an example for description. In some other embodiments, more or fewer exercise levels may be further classified. An exercise level classification manner and a quantity of classified exercise levels are not limited in this embodiment of this application.

Optionally, considering that there may be a specific deviation or a physical status of the user may be unstable in the exercise process when the electronic device calculates recommended time for exercise at a specified level, the recommended time for the exercise at this level may be within a specified duration range. Still taking a performance goal (that is, an exercise level) being "challenge yourself" as an example, as shown in FIG. 5(e), recommended time for a marathon at this level is within a range of 01 :45:30 to 01 :58:59. When actual time used by the user to complete the half marathon is within this range, it is considered that the user gives full play to an optimal exercise level.

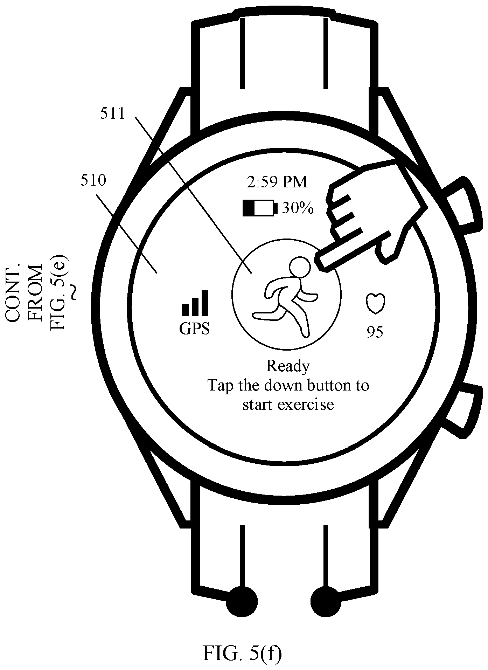

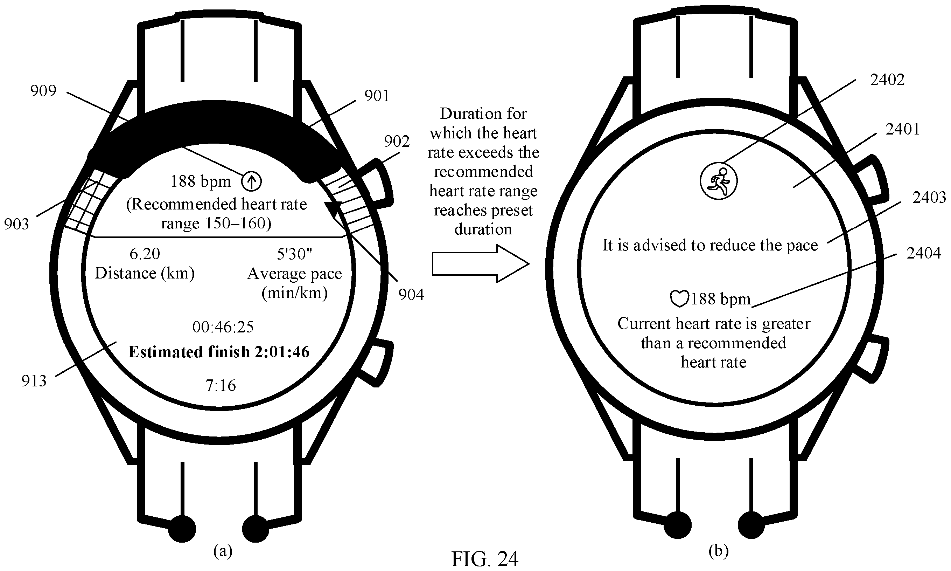

Still as shown in FIG. 5(e), the user may select one exercise level from the four exercise levels (challenge yourself, perform well, perform steadily, and complete the race) on the interface 508. For example, the user taps an option 509 corresponding to an exercise level "perform well", and taps an option "go to the race". After detecting the operation of the user, the watch jumps to an interface 510 shown in FIG. 5(f), and the user may tap a control 511 to start the race, and the watch starts to collect statistics on an exercise parameter during the race. Alternatively, the user may trigger starting of the race in another manner. For example, after the watch displays the interface 510 shown in FIG. 5(f), the user inputs a preset operation (for example, tapping a down button), to trigger the watch to start executing a current exercise strategy. For example, the user taps the down button or double-taps the control 511 in FIG. 5(f), and the watch may display an interface 913 shown in (a) in FIG. 24.

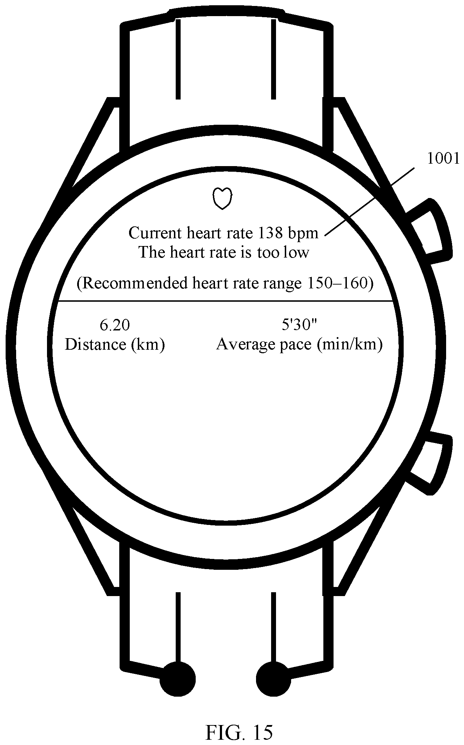

In some other examples, after detecting that the user taps the control 511, the watch may alternatively display another exercise interface similar to the interface 913. For example, after detecting that the user taps the control 511, the watch may alternatively display an exercise interface 1001 shown in FIG. 15.

Exercise parameters include but are not limited to a step quantity, a stride length, a stride rate, a distance, time, a pace, a heart rate, a body temperature, blood oxygen, blood pressure, a heart rate variability (heart rate variability, HRV, which may also be referred to as a heart rate fluctuation), a respiratory rate, and the like. Optionally, there is an association relationship between a heart rate and a pace. Generally, within a specific range, a higher pace indicates a higher heart rate.

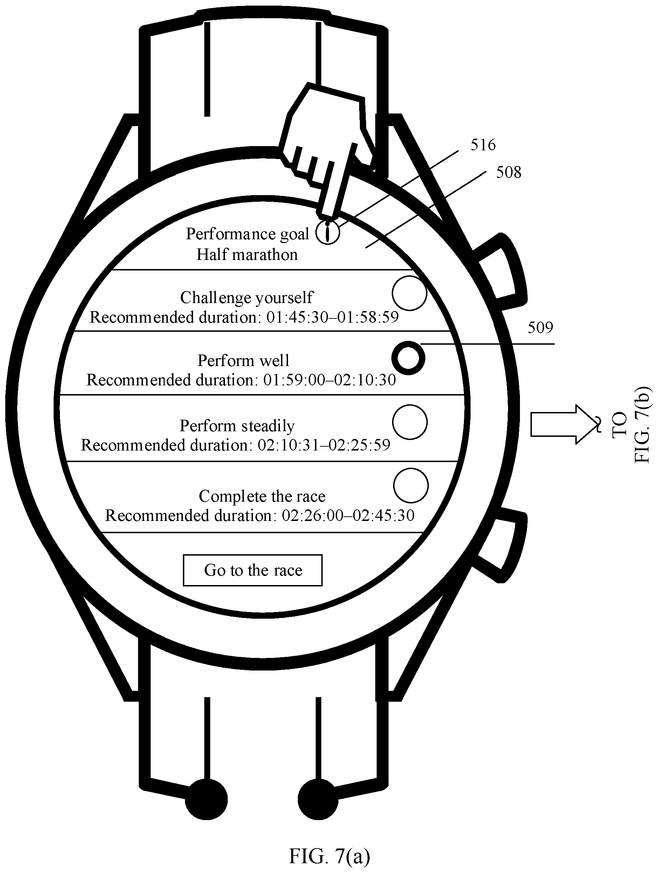

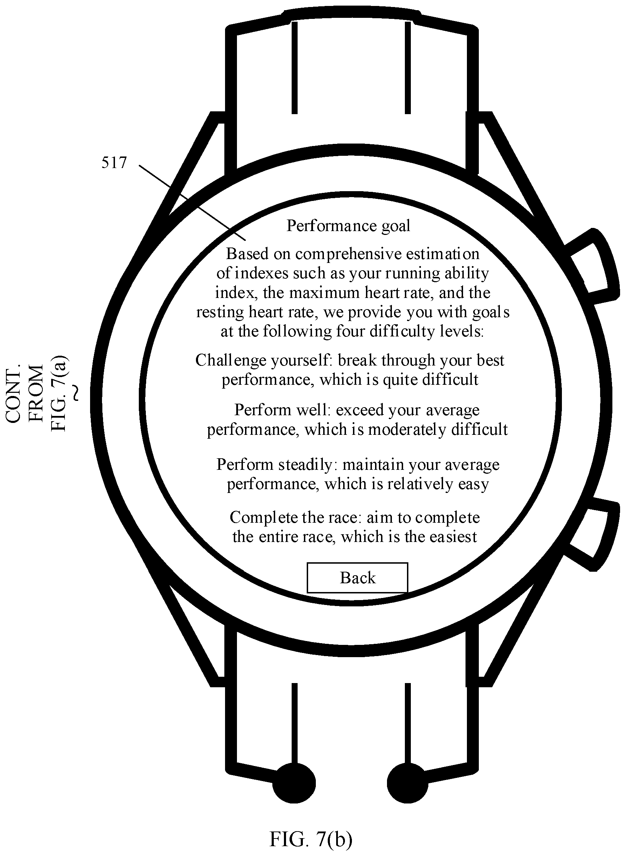

Optionally, the interface 508 may further include a control 516 that is used by the user to browse details of exercise at each level included in a "performance goal". For example, as shown in FIG. 7(a), after detecting an operation of tapping the control 516 by the user, the watch may jump to an interface 517 shown in FIG. 7(b), and the interface 517 is an interface of details of the performance goal.

In a possible implementation, the watch may calculate, based on a level of exercise, ranges of one or more exercise parameters required for completing the exercise. Still taking the four exercise levels shown in FIG. 5(e) as an example, if a half marathon at a level "challenge yourself" takes the shortest time and has the highest intensity, a pace and a heart rate required for completing the half marathon at this level are the highest. If time for a half marathon at a level "perform well" is in the middle, a pace and a heart rate required for completing the half marathon at this level are in the middle. The rest may be deduced by analogy. Exercise at each level in descending order of pace requirements is successively: challenge yourself, perform well, perform steadily, and complete the race. Similarly, exercise at each level in descending order of heart rate requirements is successively: challenge yourself, perform well, perform steadily, and complete the race.

Optionally, considering factors that there may be a specific deviation or a physical status of the user may be unstable in the exercise process when the electronic device calculates a recommended heart rate for exercise at a specified level, the recommended heart rate for the exercise at this level may be within a specified heart rate range. For example, if a performance goal (an exercise level) is "perform well", a recommended heart rate for a half marathon at this level is within a range of 150 to 160 bpm. When an actual heart rate at which the user completes the half marathon is within this range, it is considered that the user has reached exercise difficulty of this level.

Optionally, a recommended pace may be within a specified pace range. For example, if a performance goal (an exercise level) is "perform well", a recommended pace for a half marathon at this level is within a range of 5'30" to 7'30". When an actual pace at which the user completes the half marathon is within this range, it is considered that the user has reached exercise difficulty of this level.

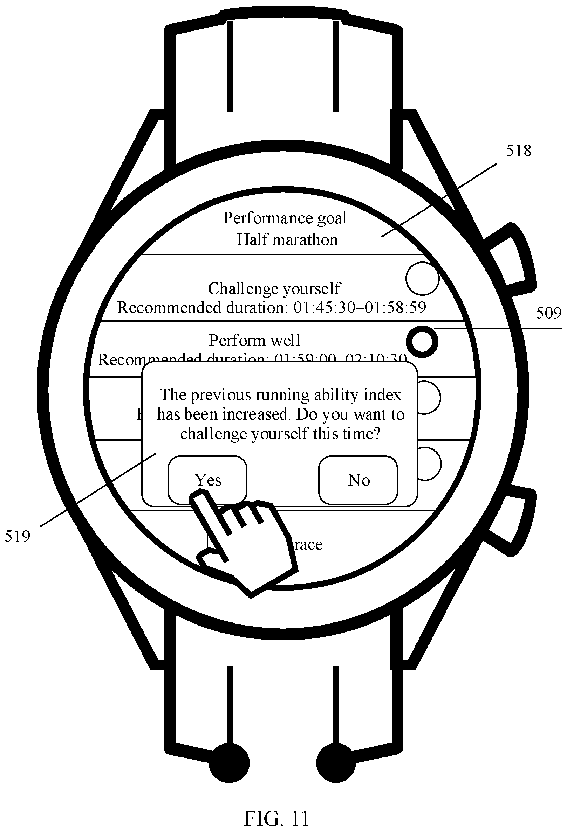

In some embodiments, it is considered that a running ability index may generally increase with a quantity of exercise times, exercise load, and the like. When detecting that the running ability index of the user is increased, the electronic device may recommend higher-level exercise to the user. For example, as shown in FIG. 11, on an interface 518, when detecting that the user taps a control 509, the watch may pop up a prompt box 519, to recommend a higher exercise level (that is, challenge yourself) to the user. After detecting that the user taps a "Yes" button in the prompt box 519, the watch sets a level of this exercise to "challenge yourself".

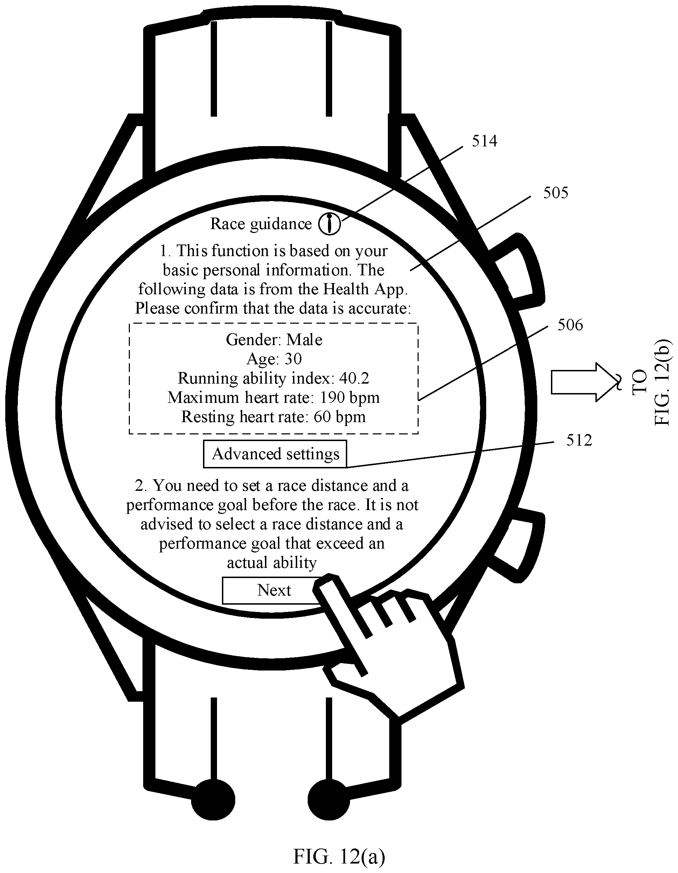

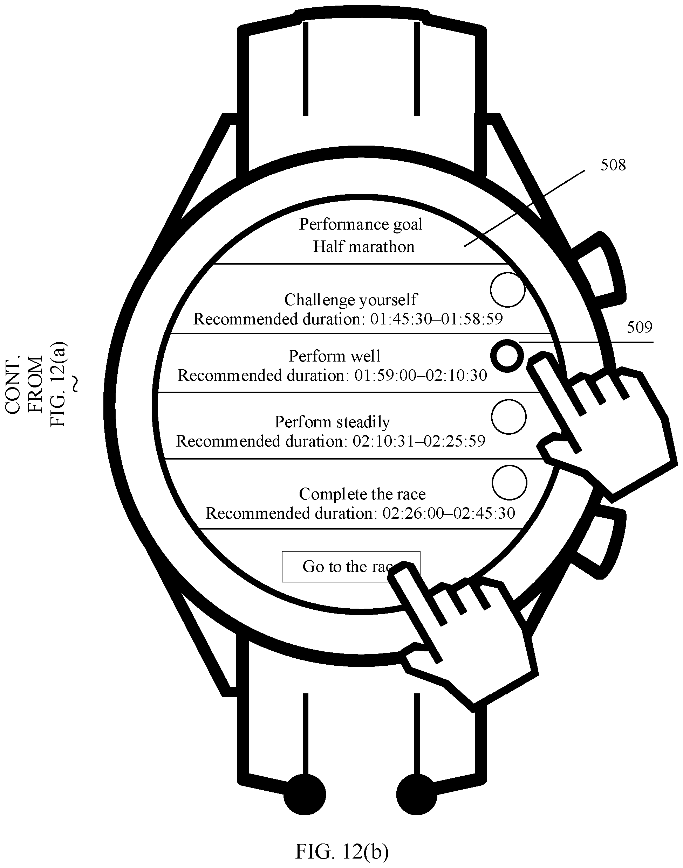

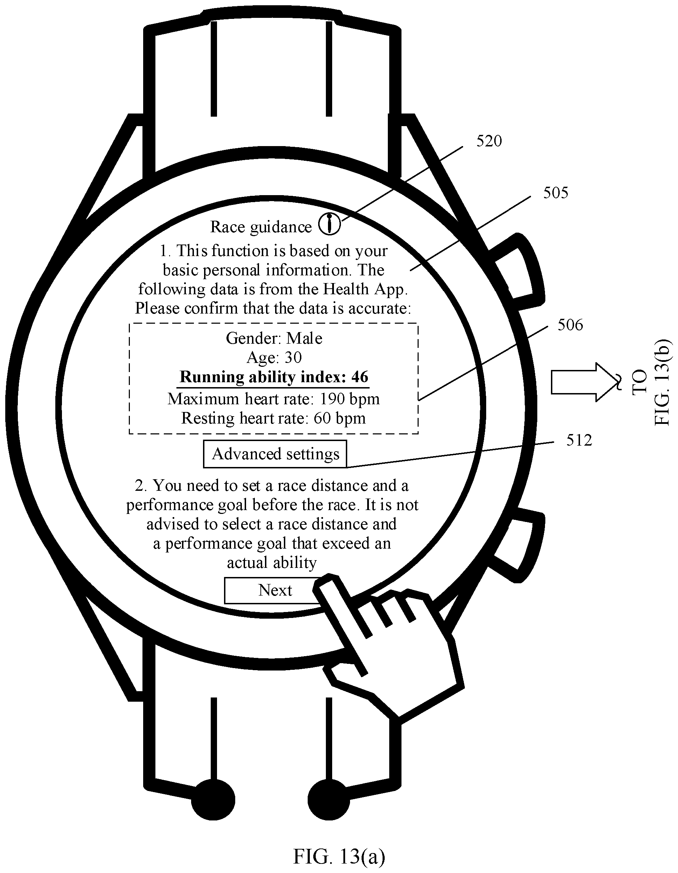

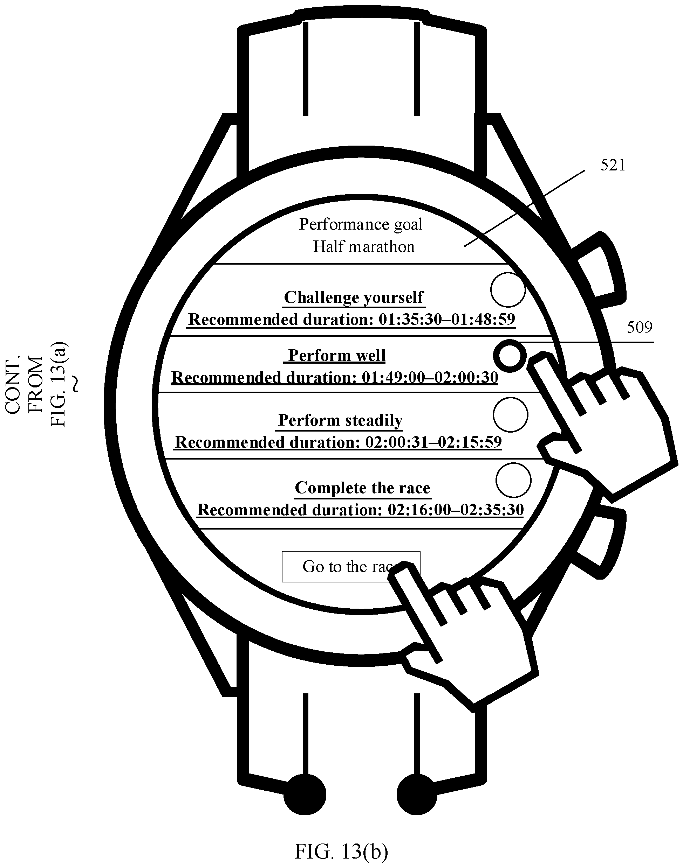

In addition, after the running ability index changes, a performance goal (recommended time) of exercise at a corresponding level changes accordingly. For example, (a) and FIG. 12(b) are setting interfaces before the user starts exercise. Then, the user increases a quantity of running times or increases exercise load, resulting in an increase in the running ability index. When the user sets race guidance by using the watch, the watch displays a race guidance prompt control 520 shown in FIG. 13(a), where a running ability index in the control 520 is increased compared with a running ability index in a control 514 shown in FIG. 12(a). Then, when the user sets a performance goal by using the watch, the watch displays an interface 521 shown in FIG. 13(b), and a performance goal (recommended duration) of exercise at each level on the interface 521 is shortened.

Optionally, the electronic device displays a changed running ability index and/or a changed performance goal by using specific UI effect. Specific UI effect includes but is not limited to: a specific color, specific animation effect (for example, blinking), a specific text prompt, and an increase in a text display size. For example, in FIG. 13(a), the changed running ability index is displayed in a bold and underline manner. In FIG. 13(b), the changed performance goal is displayed in a bold and underline manner.

Optionally, after the running ability index changes, in addition to the change in the performance goal of exercise at each level, recommended parameter value ranges such as a recommended heart rate range and/or a recommended pace range corresponding to each performance goal also change accordingly.

In a possible implementation, a part of parameters in the first parameter (used to calculate recommended time of exercise at different levels (that is, performance goals of exercise at different levels)) may be read from an application. For example, the electronic device may obtain a part of parameters (for example, a heart rate) in the first parameter from an exercise application periodically or through trigger based on another condition. For another example, an exercise application may actively report a part of parameters in the first parameter to the electronic device periodically or according to another strategy. It should be noted that the application used to obtain the first parameter is not limited to the exercise application, and may be another application. This is not limited in this application.

Alternatively, the electronic device may determine the first parameter based on perception data collected by an internal component. The internal component includes but is not limited to at least one of the following components: a sensor and a positioning component (for example, a GPS module). The sensor may include but is not limited to one or more of the following: an acceleration sensor and a gyroscope, configured to collect data of a corresponding type. The positioning component may be configured to collect location information. In some cases, the positioning component may also be considered as a sensor.

For example, the electronic device is a watch, and a status of the user or a status of the terminal may be determined based on data of a sensor such as an acceleration sensor or a gyroscope, for example, the user is walking, running, or driving. For another example, during outdoor exercise, the watch may calculate a movement distance based on data collected by a GPS module, and calculate a pace based on the movement distance. For another example, during indoor exercise, a pace may be obtained from an external device (for example, a treadmill).

Alternatively, the electronic device may have a low battery, or the corresponding first parameter obtained by using application data or an internal component of the electronic device is not accurate enough, or based on other considerations, for a purpose of data accuracy or the like, the electronic device may alternatively obtain the corresponding first parameter by using an external device. The external device may be an extended device of the electronic device, for example, an external positioning component or a sensor; or the external device may be another device connected to the electronic device; or the external device may be a sensor in another device connected to the electronic device, for example, an acceleration sensor or a gyroscope.

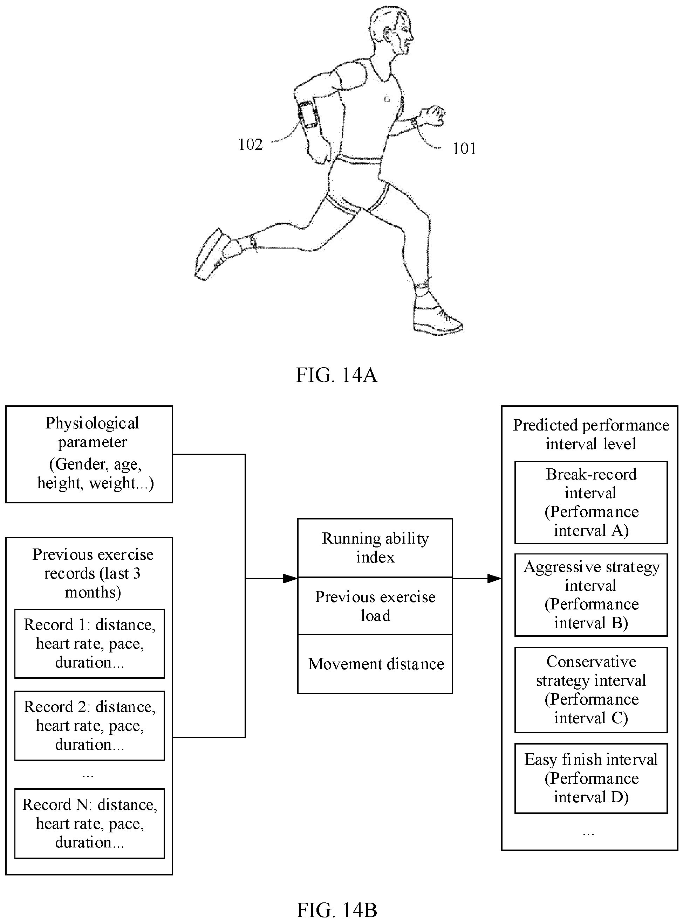

For example, as shown in FIG. 14A, the user wears the first electronic device 102 (for example, a mobile phone) and the second electronic device 101 (for example, a watch) in the exercise process. The first electronic device 102 is configured to collect a part of parameters in the first parameter, and the second electronic device 101 is configured to collect a part of parameters in the first parameter, that is, the first electronic device 102 and the second electronic device 101 jointly collect the first parameter. Alternatively, the first electronic device 102 collects the first parameter, or the second electronic device 101 collects the first parameter.

Alternatively, a part of parameters (for example, the running ability index) in the first parameter are calculated based on other parameters (for example, the heart rate and the pace) in the first parameter.

FIG. 14B shows a procedure of calculating a performance goal of exercise at each level. The electronic device obtains exercise-related data. Exercise-related parameters include but are not limited to a physiological parameter and an exercise record of the user. The physiological parameter includes but is not limited to any one or more of the following: an age, a gender, a height, and a weight. The exercise record may be an exercise record in a preset time period (for example, within three months).

The electronic device may calculate the running ability index and/or the exercise load based on the physiological parameter and the exercise record of the user. The exercise load indicates a frequency, a quantity of times, intensity, and the like of exercise. High exercise load indicates that the user exercises more frequently. In this case, when the user subsequently performs the same exercise, exercise performance is usually more stable. In some examples, the exercise load may be used to calculate an exercise ability.

As a previous exercise record is updated, the running ability index changes to guide a next time of exercise.

In a possible implementation, the electronic device may calculate one running ability index based on each exercise record, and separately calculate a plurality of running ability indexes based on a plurality of exercise records. The electronic device may perform weighting on the plurality of running ability indexes to obtain a latest running ability index. Optionally, the latest running ability index in the plurality of running ability indexes corresponds to a largest weight. It may be learned that, as a quantity of exercise times increases and an exercise record increases, a running ability index may be continuously updated.

Optionally, the user may set a movement distance, for example, select a half marathon. The electronic device may predict a performance goal of exercise based on the running ability index (and/or the exercise load) and the movement distance set by the user. The performance goal may be classified into different levels, for example, including a break-record interval A, an aggressive strategy interval B, a conservative strategy interval C, and an easy finish interval D. In an example, the break-record interval A corresponds to the foregoing performance range "challenge yourself", the aggressive strategy interval B corresponds to the performance range "perform well", the conservative strategy interval C corresponds to the performance range "perform steadily", and the easy finish interval D corresponds to the performance range "complete the race".

In this solution, the electronic device may recommend a plurality of performance intervals to the user based on a stable exercise ability, exercise load, and the like of the user in a period of time (the user may have exercised many times in this period of time). The performance interval is more in line with an exercise level of the user in this period of time, so that more accurate exercise guidance can be provided for the user. The user may select a performance interval of this exercise from the plurality of performance intervals, and the performance interval selected by the user matches an exercise ability, an exercise item, and a training status of the user.

It may be understood that after the user sets an exercise strategy, the electronic device provides guidance on exercise intensity in the exercise process for the user.

For example, as shown in (e) and FIG. 5(f), after the user sets a performance goal by using the watch and taps the control 511, the watch may display the exercise interface 913 shown in (a) in FIG. 24. In some other examples, after detecting that the user taps the control 511, the watch may alternatively display another exercise interface similar to the interface 913.

In some embodiments, the watch may detect a real-time exercise parameter of the user. If a value of the exercise parameter exceeds a recommended value range of exercise at a corresponding level, the watch may prompt the user to adjust the exercise parameter, so that the user completes the exercise based on the preset performance goal by adjusting the exercise parameter.

Optionally, the electronic device may prompt the user to adjust the exercise parameter in an interface manner, or in a voice and/or vibration manner, or through a combination of a plurality of manners, for example, playing a voice while displaying an exercise interface. A prompt manner is not limited in this embodiment of this application.

For example, assuming that a range of a recommended heart rate is 150 to 160, and if a current heart rate of the user is 138 bpm, the watch may play a voice "the current heart rate is 138 times per minute and is lower than the recommended heart rate, so it is advised to increase the pace", to prompt the user to adjust a current pace. In this solution, a heart rate may represent exercise intensity. Therefore, actual exercise intensity of the user may be detected by detecting a real-time heart rate of the user. When an actual heart rate of the user exceeds the recommended heart rate range, it indicates that the exercise intensity of the user does not meet exercise intensity required by the performance goal. In this case, the electronic device may prompt the user, so that the user adjusts the exercise intensity according to the prompt, thereby helping the user achieve the performance goal.

Optionally, the electronic device may prompt, by using any one or more pieces of information, the user that an exercise parameter exceeds a parameter range, and the one or more pieces of information include a prompt card, a voice, a vibration, and an exercise interface.

For example, it is assumed that a recommended heart rate range is 150 to 160, and if a current heart rate of the user is 138 bpm, the watch may display the exercise interface 1001 shown in FIG. 15, to prompt the user to adjust a current pace. The exercise interface 1001 includes the recommended heart rate range and the current actual heart rate. The user may adjust a pace according to a prompt on the exercise interface 1001, so that the actual heart rate is within the recommended heart rate range. Optionally, the exercise interface 1001 may further include a current movement distance (6.20 km) and a current average pace (5'30" min/km, indicating that 1 km takes 5 minutes and 30 seconds). Optionally, before displaying the exercise interface 1001, the watch may further vibrate for a period of time (for example, vibrate for 8 seconds), to prompt the user to view the interface of the watch.

In this solution, the electronic device may present prompt information, so that the user adjusts exercise intensity according to the prompt, and the user give full play to an optimal state during exercise and safely complete the exercise, thereby achieving a corresponding desired result of the user.

Optionally, if an exercise parameter of the user exceeds a recommended value range for a short time, the electronic device may not present prompt information (for example, not display an interface used to prompt a pace adjustment, or not vibrate), to prevent ping-pong effect from frequently presenting prompt information to the user and disturbing the user. On the contrary, if the exercise parameter of the user exceeds the recommended value range for a long time, for example, for more than preset duration, the watch presents the prompt information.

For example, the preset duration is 5 minutes. For example, at 7:11 to 7:16, if it is detected that the heart rate of the user is continuously less than a minimum value of the recommended heart rate range within 5 minutes, the watch displays the exercise interface. Then, it is assumed that the heart rate of the user is within the recommended heart rate range within 7:17 to 7:26, and the heart rate of the user is continuously less than the minimum value of the recommended heart rate range again within 7:27 to 7:30. It may be learned that before 7:27 to 7:30, the watch has displayed the exercise interface, and may prompt the user 4 minutes in advance. In other words, the preset duration may be dynamically adjusted. If the heart rate of the user is less than the recommended heart rate range for a plurality of times in a specific time interval (for example, within 30 minutes), the watch is triggered to prompt the user that the preset duration may gradually become shorter.

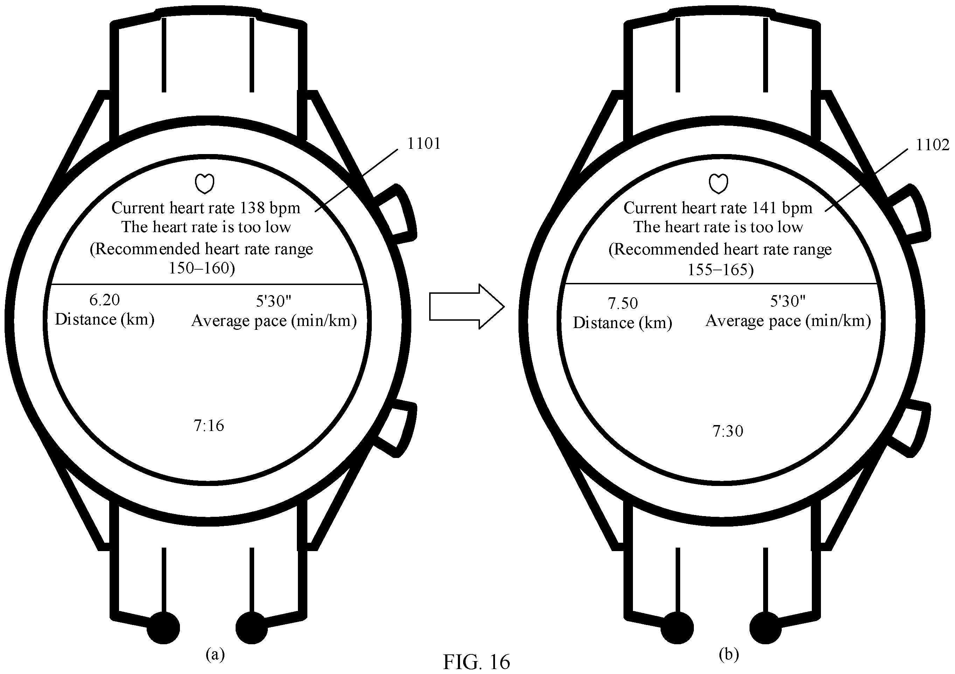

Optionally, the electronic device dynamically monitors an exercise parameter of the user in real time, and may dynamically calculate a recommended parameter range based on a current physical status of the user. FIG. 16 shows exercise interfaces displayed by the watch twice in the exercise process of the user. At 7:16, the watch detects that an actual heart rate of the user is 138 bpm, and a recommended heart rate range is 150 to 160 in this case. As shown in an interface 1101 in (a) in FIG. 16, the current actual heart rate of the user is lower than the recommended heart rate range 150 to 160. The watch monitors and calculates the exercise parameter of the user in real time. At 7:30, the watch detects that the actual heart rate of the user is 141 bpm, and the recommended heart rate range has changed to 155 to 165 in this case. As shown in an interface 1102 in (b) in FIG. 16, the current actual heart rate of the user is lower than the recommended heart rate range.

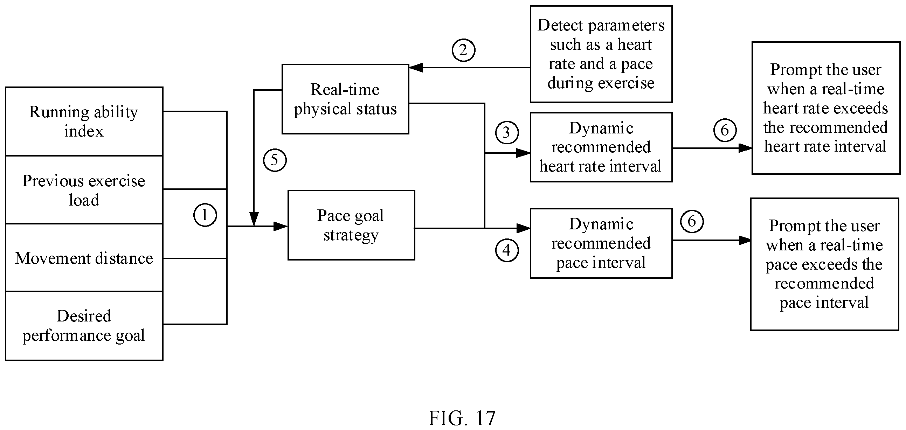

FIG. 17 shows a procedure of calculating a recommended pace range and a recommended heart rate range. The electronic device obtains one or more parameters such as a running ability index, previous exercise load, a movement distance, and a performance goal, and calculates a pace goal strategy (for example, a pace curve) based on the one or more parameters. In addition, the electronic device may obtain a real-time exercise parameter (for example, a real-time heart rate and a real-time pace shown in FIG. 17) of the user in the exercise process, and comprehensively evaluate a current physical status of the user based on each exercise parameter of the user. The electronic device may calculate and update the recommended heart rate range and/or the recommended pace range in real time during the race based on the pace goal strategy corresponding to a performance goal expected by the user and with reference to the real-time physical status of the user.

Optionally, the exercise parameter used to calculate the physical status may be but is not limited to parameters such as a slope, blood oxygen, blood pressure, a respiratory rate, a body temperature, an HRV, training load, a step quantity, a stride length, a stride rate, a distance, and time.

In some examples, if it is detected that the real-time heart rate of the user exceeds the recommended heart rate range, the electronic device may prompt the user to adjust exercise intensity; and/or if it is detected that the real-time pace of the user exceeds the recommended pace range, the electronic device may prompt the user to adjust exercise intensity.

Optionally, in FIG. 17, the real-time physical status of the user may be further used to calculate the pace goal strategy, so that the pace goal strategy is more in line with an exercise status of the user.

In this solution, the electronic device may recommend an appropriate heart rate range and/or an appropriate pace range to the user based on a current exercise parameter (for example, a pace, a heart rate, a target distance, a physical status, and a performance goal) of the user, to guide the user to better achieve the performance goal.

Optionally, to indicate and quickly identify different heart rate ranges, indication regions of a plurality of heart rate ranges may be set on a display, and different indication regions have different user interface (user interface, UI) effect. The UI effect includes but is not limited to any one or more of the following effect: color effect and animation effect.

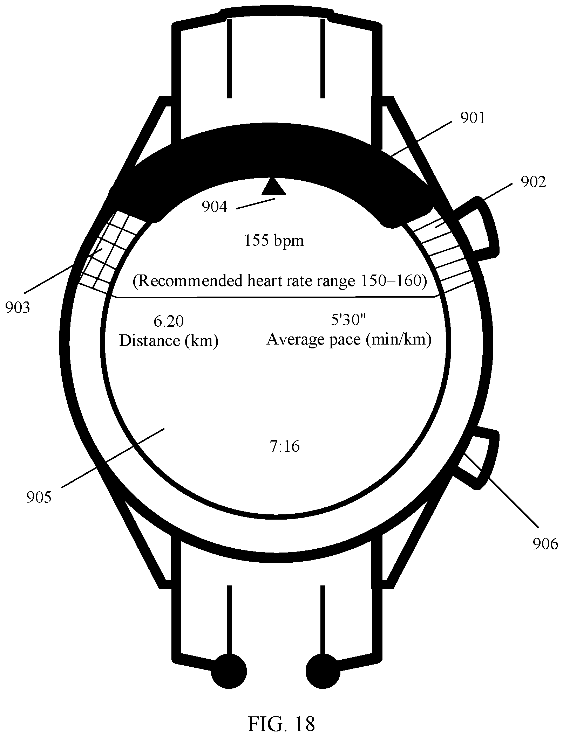

For example, as shown in FIG. 18, when the user presses a down button 906 in a running process, the watch may display an interface 905. The interface 905 may include a current heart rate (155 bpm) of the user. Optionally, the interface 905 may further include a recommended heart rate range (for example, 150 to 160).

In a possible implementation, a display edge (that is, an edge of a watch face) of the interface 905 may include three indication regions: an indication region 901, an indication region 902, and an indication region 903. Different indication regions represent different heart rate ranges. Optionally, the indication region 901 may represent a recommended heart rate range (for example, 150 to 160 bpm), a heart rate value in a heart rate range represented by the indication region 903 is less than a minimum value (for example, < 150 bpm) of the recommended heart rate range, and a heart rate value in a heart rate range represented by the indication region 902 is greater than a maximum value (for example, > 160 bpm) of the recommended heart rate.

Optionally, colors of the three indication regions may be different, or colors of the indication region 903 and the indication region 902 are the same, and a color of the indication region 901 is different from that of the indication region 903, or another UI design manner is used, so that the user can easily distinguish between different indication regions. A specific UI style of the indication region is not limited in this embodiment of this application.

As shown in FIG. 18, the interface 905 further includes a pointer 904, and a location at which the pointer 904 points to the edge of the watch face represents a current actual heart rate of the user. In FIG. 18, the electronic device obtains a current actual heart rate 155 bpm of the user through measurement, and the pointer 904 points to a location corresponding to 155 bpm in the indication region 901 (for example, the recommended heart rate range is 150 to 160 bpm, and the pointer 904 points to a middle location in the indication region 901). Through a pointing direction of the pointer 904, the user may quickly identify that the current heart rate is within the recommended heart rate range, or the current heart rate is greater than or less than the recommended heart rate range, to perform exercise adjustment according to the foregoing indication.

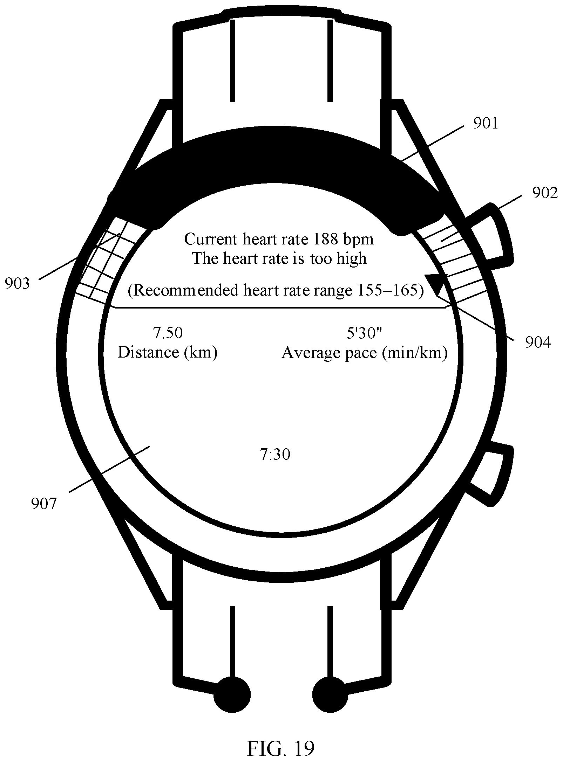

For another example, as shown in FIG. 19, if the heart rate exceeds the recommended heart rate range in the exercise process of the user, the watch may display an interface 907. In the interface 907, an actual heart rate of the user is 188 bpm, and the pointer 904 points to a location corresponding to 188 bpm in the indication region 902. The user may quickly determine, based on the pointing direction of the pointer 904, that the current heart rate is too high. Therefore, the user may reduce a pace, to successfully complete the exercise based on a specified performance goal.

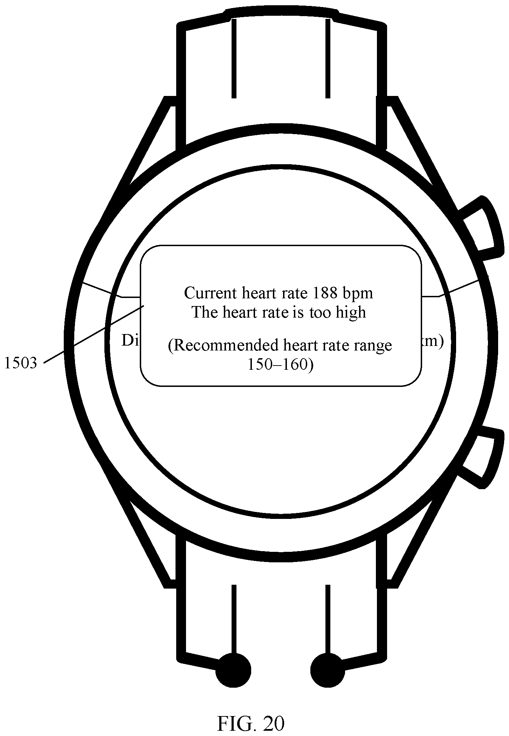

Optionally, the electronic device may further prompt, in a card pop-up manner, the user to adjust the exercise parameter. For example, as shown in FIG. 20, if the heart rate exceeds the recommended heart rate range in the exercise process of the user, the watch may pop up a prompt card 1503. The interface 1503 includes an actual heart rate (188 bpm) of the user and a recommended heart rate range (150 to 160). Optionally, when popping up the prompt card, the electronic device may further play a voice and/or vibration.

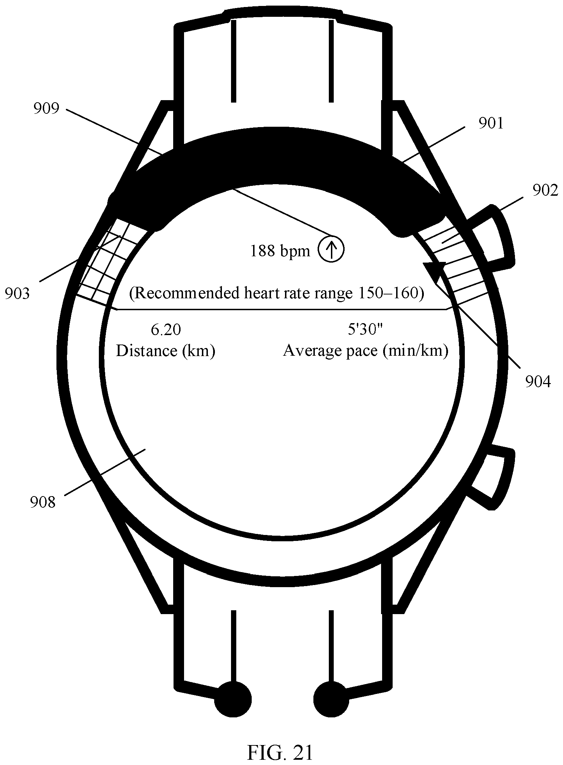

Optionally, to help the user identify the current exercise parameter, the watch may further display an icon used to represent an exercise parameter value. For example, if the heart rate is greater than the recommended heart rate range in the exercise process of the user, the watch may display an interface 908 shown in FIG. 21. The interface 908 includes a current heart rate 188 bpm of the user. The interface 908 further includes an icon 909, and the icon 909 is used to represent that the current heart rate is greater than a maximum value of the recommended heart rate range. For meanings of other interface elements on the interface 908, refer to the related descriptions in the foregoing embodiment. Details are not described again.

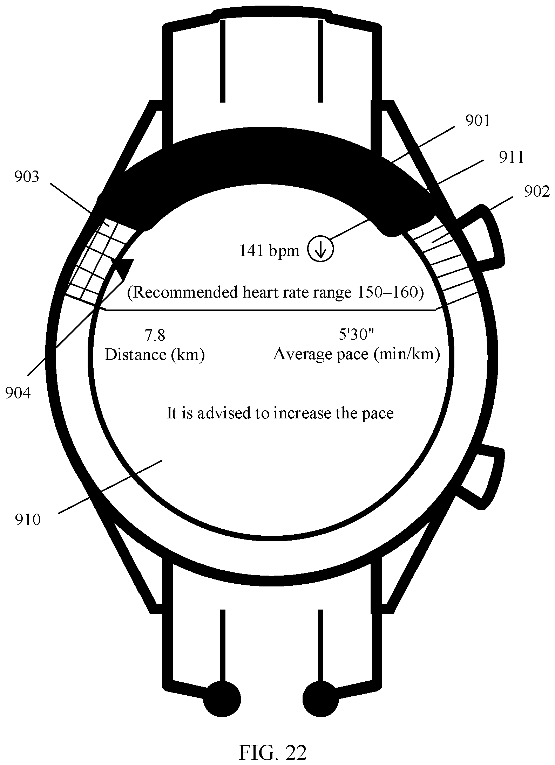

For another example, if the heart rate is less than the recommended heart rate range in the exercise process of the user, the watch may display an interface 910 shown in FIG. 22. The interface 910 includes a current heart rate 141 bpm of the user. The interface 910 further includes an icon 911, and the icon 911 is used to represent that the current heart rate is less than a minimum value of the recommended heart rate range. For meanings of other interface elements on the interface 910, refer to the related descriptions in the foregoing embodiment. Details are not described again.

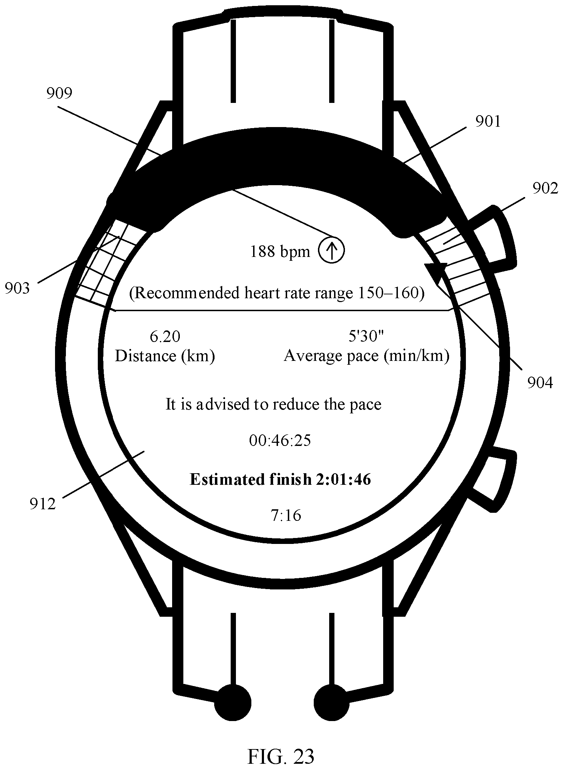

The foregoing interface used to prompt that the current heart rate exceeds the recommended heart rate range may be collectively referred to as an exercise interface. It should be noted that, the exercise interface may include other information in addition to the recommended heart rate range and/or the recommended pace range of the user. For example, as shown in FIG. 23, an exercise interface 912 may further include current exercise duration (exercise duration, for example, 00:46:25) and estimated finish duration (for example, 2:01:46). Optionally, the exercise interface may further include a current time point (for example, 7:16).

Optionally, a display interface of the watch may be switched from the current exercise interface to another interface. Interface switching may be automatically performed by the watch, or may be performed by the watch according to an instruction of the user.

For example, in the exercise process of the user, the watch may display the exercise interface 913 shown in (a) in FIG. 24. For descriptions of interface elements included in the exercise interface 913, refer to the related descriptions of the interface 912 shown in FIG. 23. When a trigger condition is met, the watch may switch to another interface. For example, if duration for which the heart rate exceeds the recommended heart rate range 150 to 160 reaches specified duration (for example, 5 minutes), the watch may jump to an interface 2401 shown in (b) in FIG. 24. The interface 2401 includes an exercise guidance recommendation 2403 (it is advised to reduce the pace), so that the user adjusts the exercise parameter (for example, reduces the pace) based on the exercise guidance recommendation 2403.

Optionally, the interface 2401 may further include current heart rate information 2404 (for example, 188 bpm). Optionally, the interface 2401 may further include an icon 2402. Optionally, the icon 2402 may be an outdoor running icon or another icon.

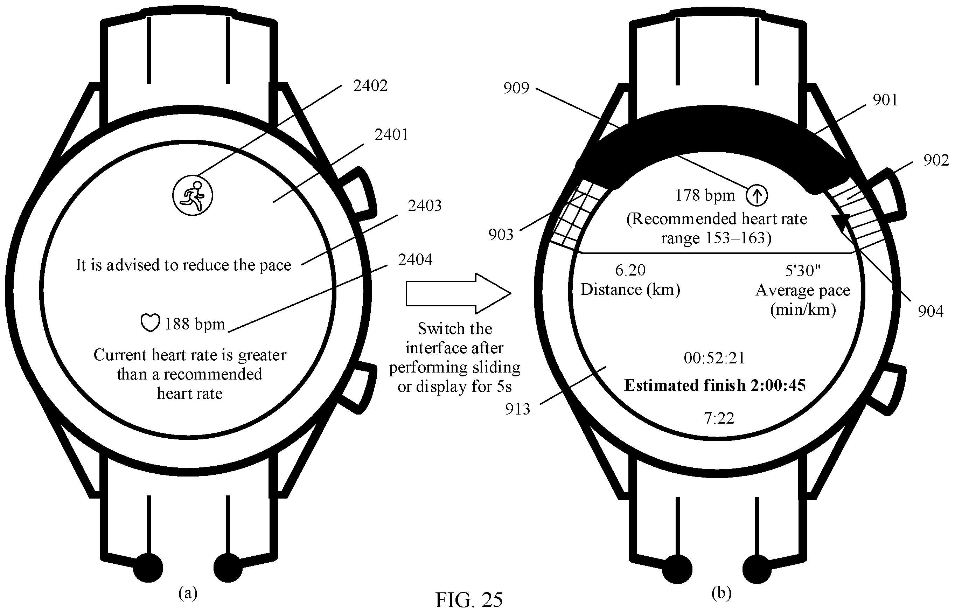

As shown in (a) in FIG. 25, the watch displays the interface 2401, to prompt the user to adjust the exercise parameter. In some examples, if a preset operation (for example, a rightward slide operation) of the user is detected, the watch may jump back to the exercise interface 913 shown in (b) in FIG. 25. Alternatively, in some other embodiments, if duration for which the watch displays the interface 2401 reaches specific duration (for example, 5 seconds), the watch may automatically jump back to the exercise interface 913 shown in (b) in FIG. 25.

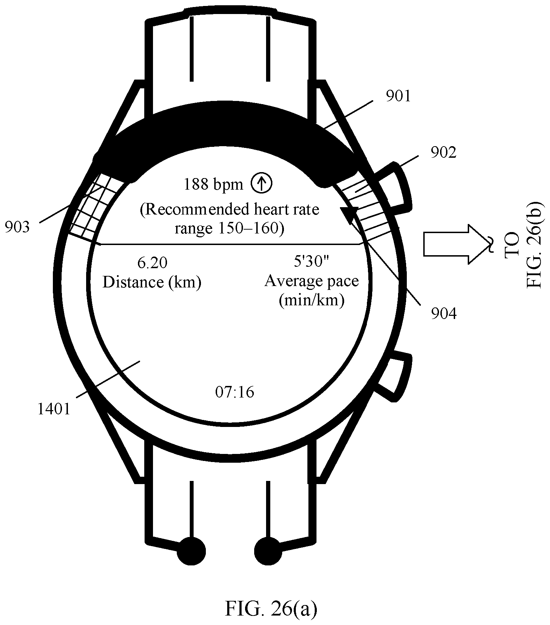

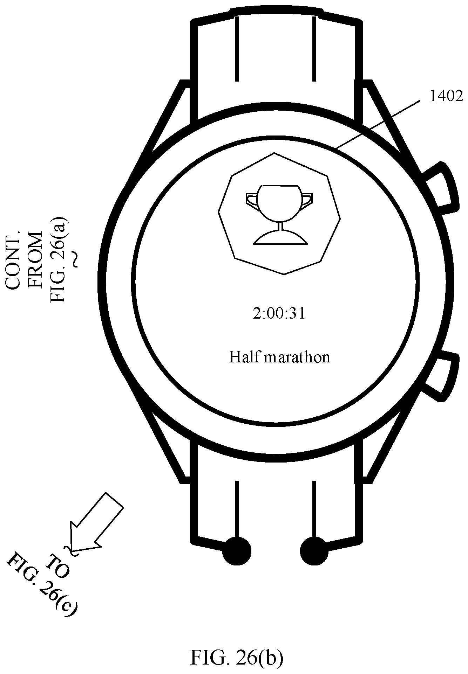

For example, in the exercise process of the user, the watch may display an interface 1401 shown in FIG. 26(a). Then, the user continues to exercise until a target distance is reached. For example, when the user finishes running a half marathon selected by the user shown in FIG. 5(d), the watch may automatically switch to an interface 1402 shown in FIG. 26(b), to prompt the user that this exercise ends. Optionally, the interface 1402 includes exercise duration (2:00:31) of this exercise (half marathon). Optionally, when displaying the interface 1402, or before or after displaying the interface 1402, the watch may vibrate for first preset duration (for example, 300 milliseconds). Optionally, duration for which the watch displays the interface 1402 is second preset duration (for example, 5 seconds).

Optionally, after this exercise ends, a race guidance function is disabled. Then, UI effect of the interface displayed by the watch is restored to UI effect before the race guidance is enabled. For example, after this exercise (for example, the half marathon selected by the user shown in FIG. 5(d)) ends, the race guidance function is disabled, and the interface displayed by the watch no longer displays an indication region having different UI effect (for example, no longer displays the indication region 901). When doing exercise next time, the user may enable the race guidance function through a related interface (for example, the interface 505 shown in FIG. 6(a)).

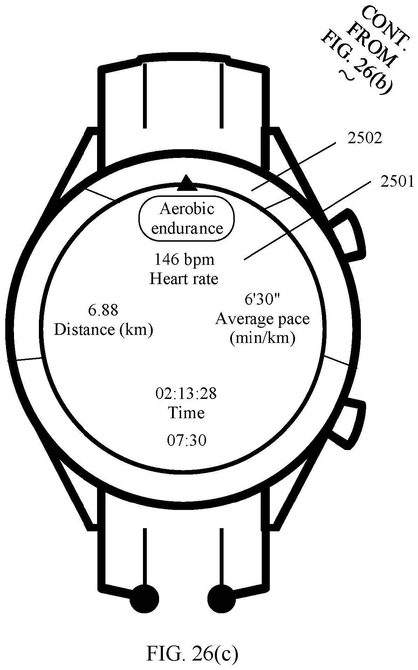

Alternatively, in some other examples, the watch displays the interface 1402 shown in FIG. 26(b), to prompt the user that this exercise (for example, the half marathon selected by the user shown in FIG. 5(d)) ends. After this exercise ends, the watch disables the race guidance function, and UI effect of an annular region on an edge of a display of the watch is no longer displayed. For example, after disabling the race guidance, the watch displays an interface 2501 shown in FIG. 26(c). Compared with an annular region 901 shown in FIG. 26(a), an annular region 2502 on the interface 2501 is no longer displayed with specific UI effect.

Optionally, after the exercise interface is displayed, the watch may switch to another interface in response to a preset operation input by the user. Optionally, the preset operation includes but is not limited to any of the following operations: sliding left or right on the interface, sliding up or down on the interface, and tapping a down button. Alternatively, after the display of the exercise interface reaches preset duration, the watch may switch to another interface.

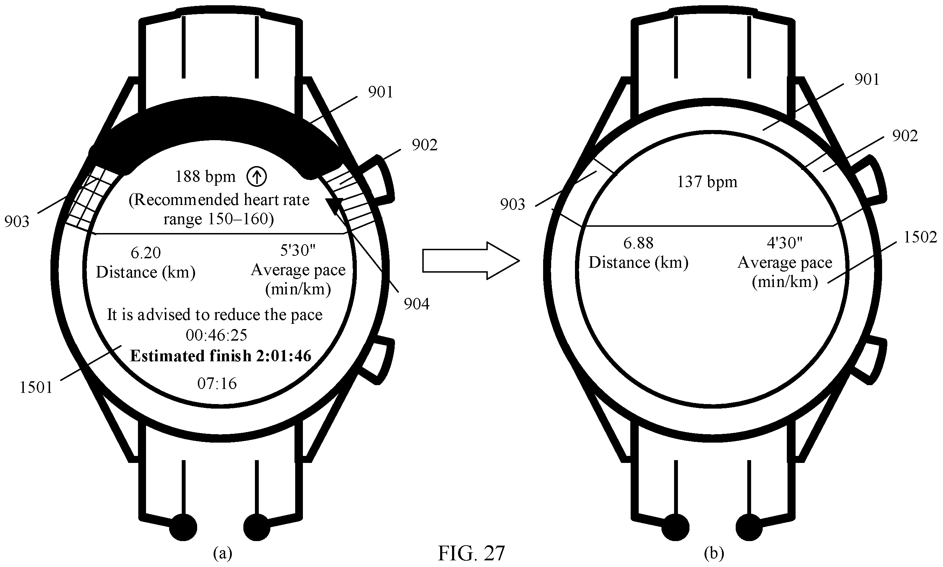

For example, if the heart rate is greater than a range of a recommended heart rate in the exercise process of the user, the watch may display an interface 1501 shown in (a) in FIG. 27. The interface 1501 is used to prompt the user that the current heart rate is greater than the recommended heart rate, and the user may adjust a pace based on the prompt on the interface 1501. Then, in response to an operation of tapping a down button by the user, the watch may switch to a step counting interface 1502 shown in (b) in FIG. 27. Optionally, duration for which the watch displays the interface 1502 is third preset duration (for example, 5 seconds).

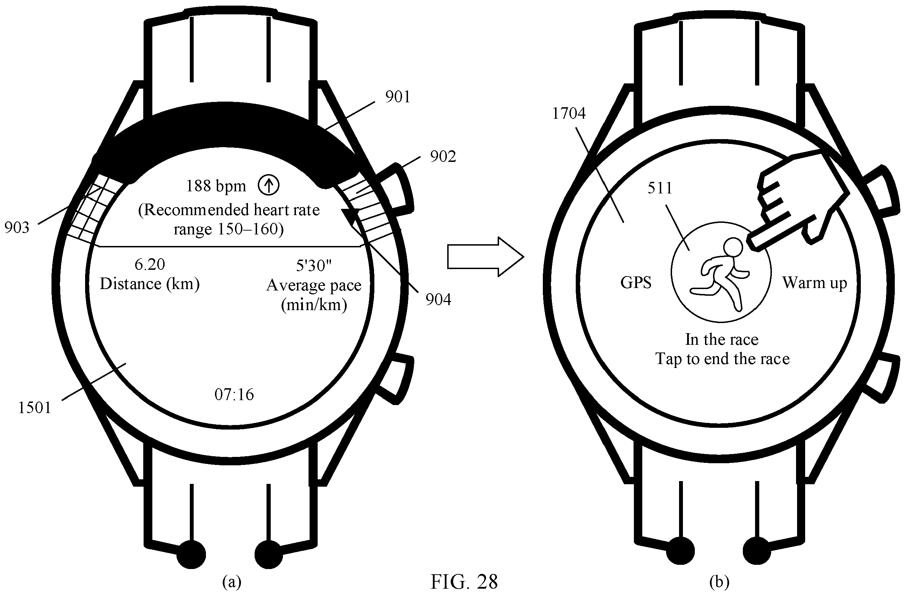

For another example, as shown in (a) in FIG. 28, the watch displays an exercise interface 1501, to prompt the user that the current heart rate is too high, so that the user adjusts exercise intensity based on the prompt. When duration of displaying the exercise interface 1501 reaches 5 seconds or a rightward slide operation of the user is detected, the watch jumps to an interface 1704 shown in (b) in FIG. 28. The interface 1704 includes a control 511, and the user may end the current exercise by tapping the control 511.

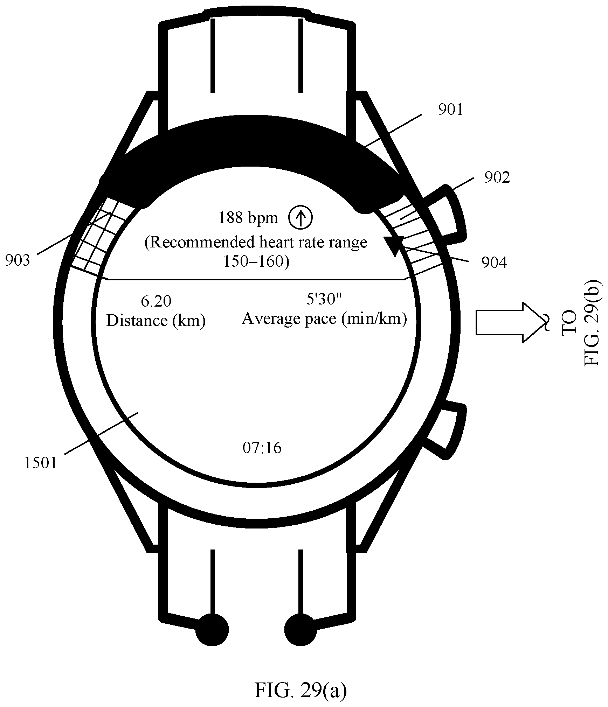

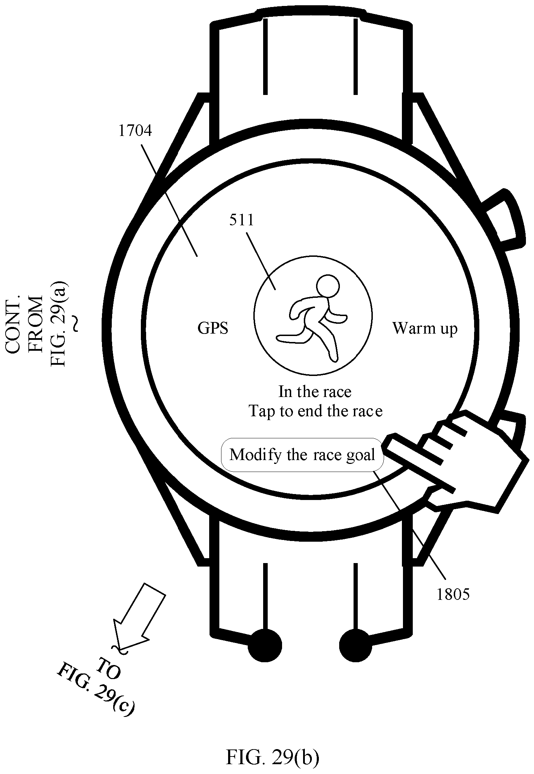

Optionally, the electronic device may further display a control used to modify a performance goal, so that the user can dynamically adjust a performance goal of this exercise in the exercise process. For example, as shown in FIG. 29(a), the watch displays an exercise interface 1501, to prompt the user that the current heart rate is too high. In some examples, if a rightward slide operation of the user is detected, the watch jumps to an interface 1704 shown in FIG. 29(b), and the interface 1704 includes a control 1805.

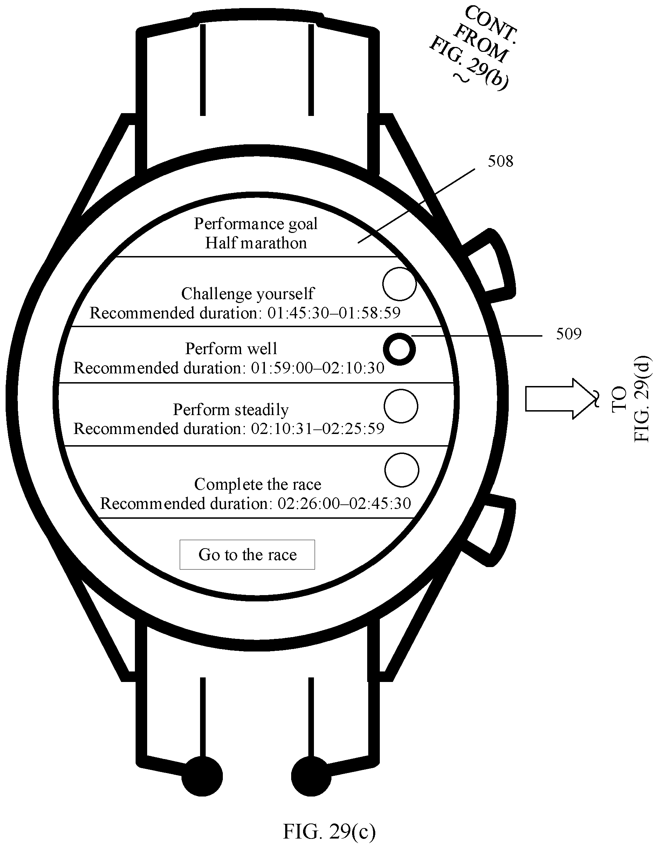

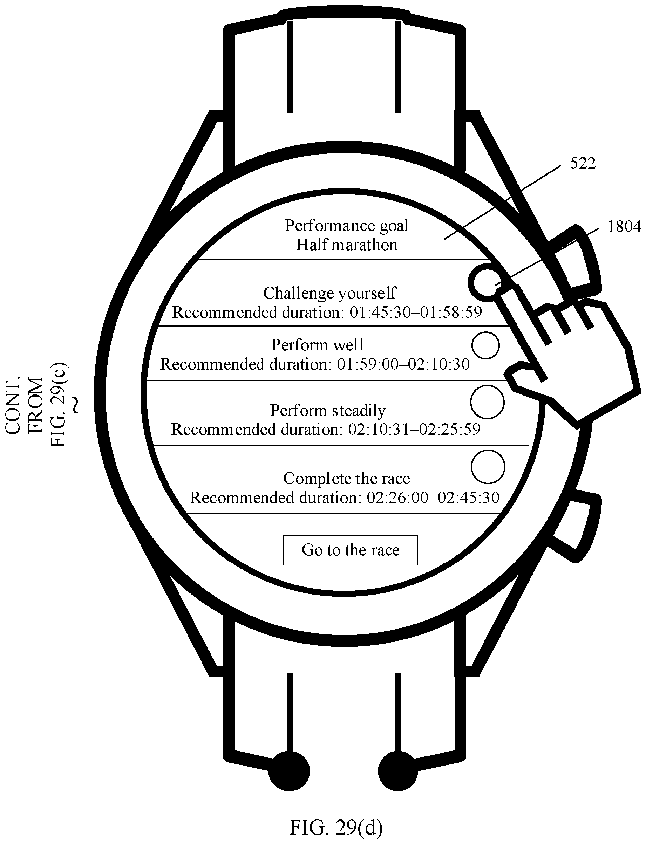

In some examples, if it is detected that the user taps the control 1805, the watch may jump to an interface 508 for setting a performance goal shown in FIG. 29(c). As shown in FIG. 29(c), an exercise level previously selected by the user is "perform well". Then, as shown in FIG. 29(d), after it is detected that an option 1804 corresponding to an exercise level "challenge yourself" is tapped, the watch modifies a current exercise goal. Subsequently, the watch may determine a recommended heart rate range and/or a recommended pace range based on the modified performance goal. In some examples, the watch may further dynamically estimate finish time of the user based on the modified performance goal.

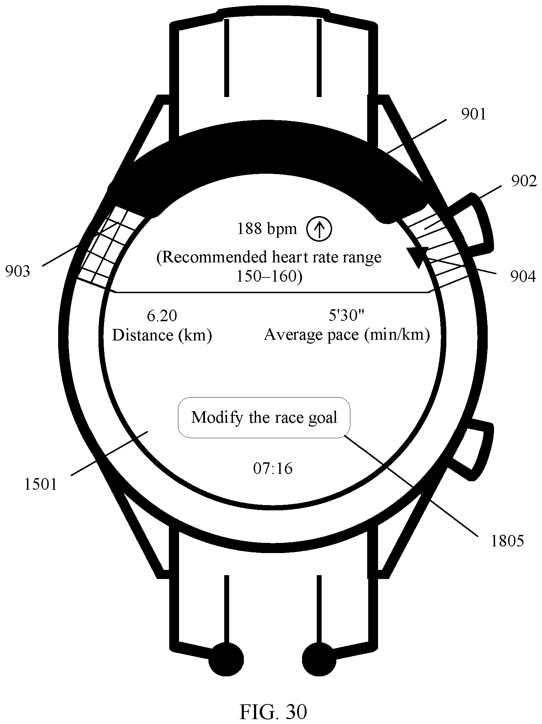

In some other embodiments, the control 1805 used to modify a race goal may be alternatively displayed on another interface. For example, as shown in FIG. 30, the watch may display the control 1805 on the exercise interface 1501, to help the user modify a race goal.

Based on a process of prompting the user of the heart rate when the heart rate exceeds the recommended heart rate range, in some other embodiments, the watch may further detect other exercise parameters. When values of one or more exercise parameters exceed recommended value ranges, the watch may prompt the user that the values of the one or more exercise parameters exceed the recommended value ranges and display the recommended value ranges of the one or more exercise parameters. For a specific implementation in which the watch prompts the user of the values of the one or more exercise parameters and displays the recommended value ranges of the one or more exercise parameters, refer to the related descriptions in which the watch prompts the user of the heart rate and displays the recommended range of the heart rate.

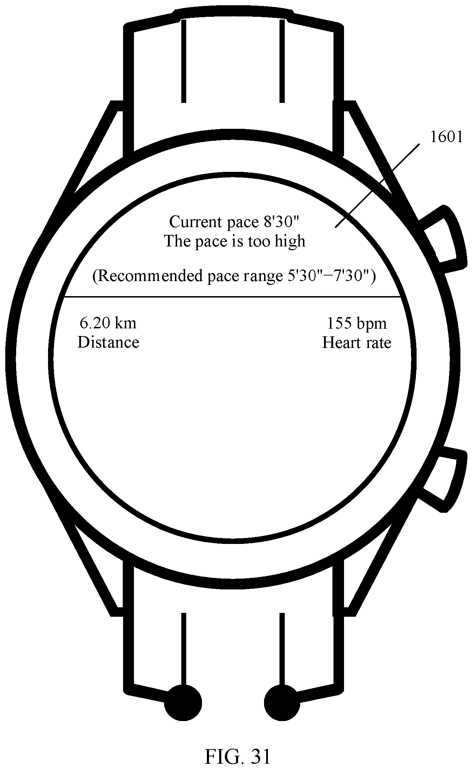

For example, an exercise parameter is a pace. For example, if a pace is greater than a recommended pace range in the exercise process of the user, the watch may display an interface 1601 shown in FIG. 31, and the interface 1601 is used to prompt the user that the current pace is too high. The interface 1601 may include the current pace of the user and the recommended pace range (5'30" to 7'30"), and the user may adjust the pace based on the prompt on the interface 1601.

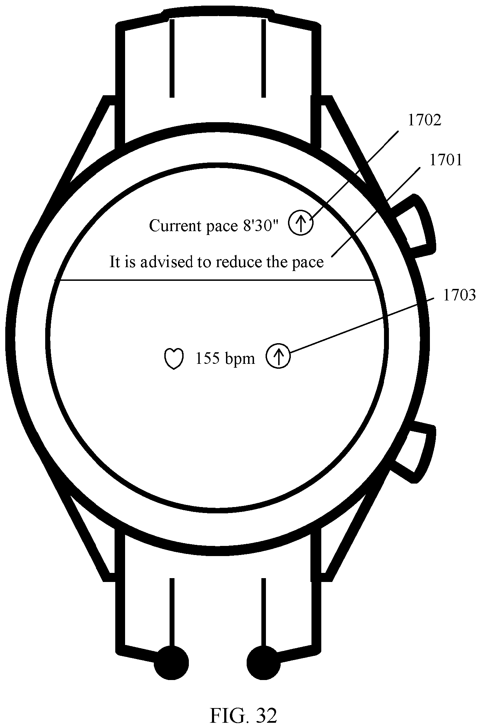

Optionally, when the watch prompts the user of the values of the one or more exercise parameters through the interface, different indication regions may be set on the interface, and the value of the exercise parameter is indicated through a pointer, and/or a specific icon (used to indicate that the value of the exercise parameter is too high or too low) is displayed on the interface, so that the user can quickly identify whether the value of the exercise parameter meets the recommended range. For example, if the pace is greater than the recommended pace range and the heart rate is greater than the recommended heart rate range in the exercise process of the user, the watch may display an interface 1701 shown in FIG. 32. The interface 1701 may include a current pace of the user and an icon 1702, and the icon 1702 is used to prompt the user that the current pace is too high. The interface 1701 further includes a current heart rate of the user and an icon 1703, and the icon 1703 is used to prompt the user that the current heart rate is too high. Optionally, the interface 1701 may further include an exercise guidance recommendation (it is advised to reduce the pace).

In some embodiments, after prompt information A indicating that a parameter exceeds a parameter range is displayed, if the user does not adjust the exercise parameter, the exercise parameter still exceeds the parameter range, and if duration for which the exercise parameter exceeds the parameter range reaches specific duration, the electronic device may again display prompt information B indicating that the parameter exceeds the parameter range. Optionally, the prompt information A and the prompt information B may be any one or more of the foregoing prompt card, exercise interface, voice, and vibration. Optionally, the prompt information A and the prompt information B may be prompt information in a same manner or in different manners.

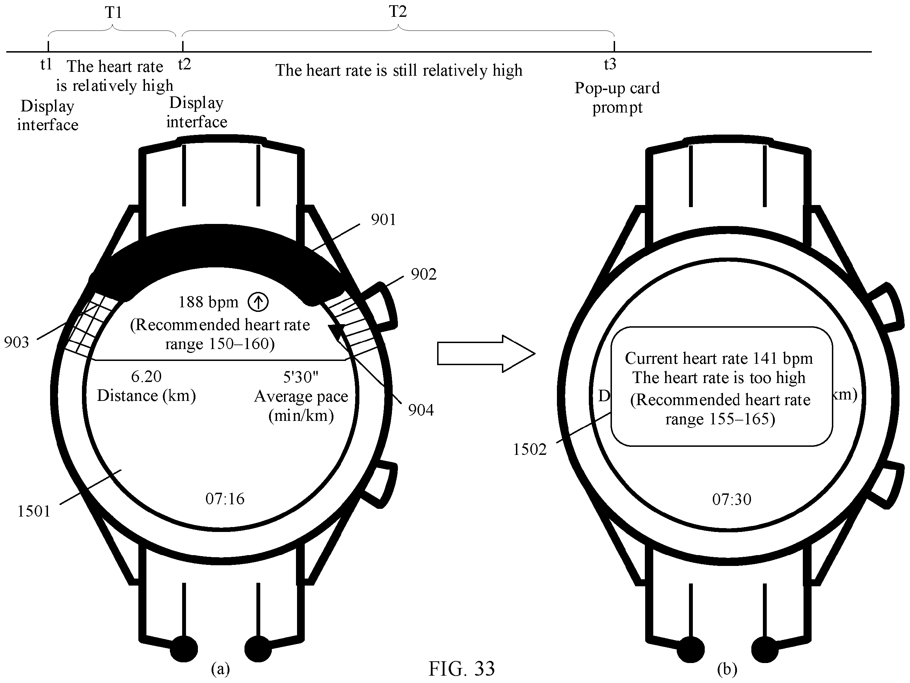

For example, as shown in FIG. 33, the watch displays an exercise interface at a moment t1. Through measurement, if the watch determines that a heart rate of the user is continuously relatively high in a time period T1 (t1-t2), the watch may display, at a moment t2, an exercise interface 1501 shown in (a) in FIG. 33, to prompt that a real-time heart rate exceeds a recommended heart rate range. Then, the watch continues to detect the real-time heart rate of the user. If it is detected that the real-time heart rate of the user is still continuously relatively high in a T2 period (t2-t3), the watch may prompt, at a moment t3, the user again that the heart rate is relatively high.

In some examples, to attract attention of the user, the watch may pop up a pop-up window 1502 shown in (b) in FIG. 33, to prompt the user that a real-time heart rate is relatively high.

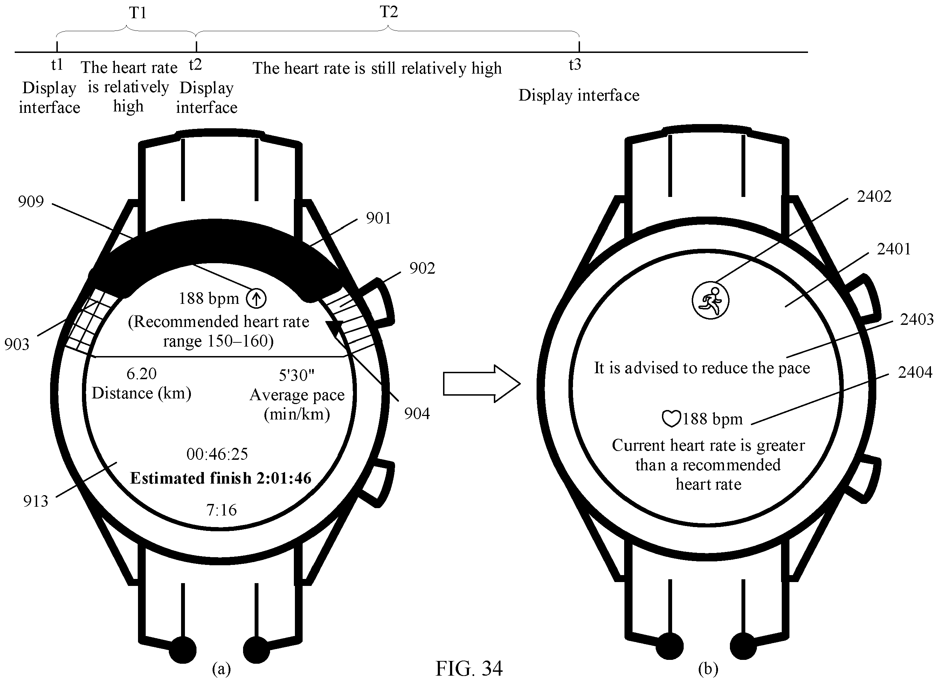

For another example, as shown in FIG. 34, the watch displays an exercise interface at a moment t1. Through measurement, if the watch determines that a heart rate of the user is continuously relatively high in a time period T1 (t1-t2), the watch may display, at a moment t2, an exercise interface 913 shown in (a) in FIG. 34. Then, the watch continues to detect the real-time heart rate of the user. If it is detected that the real-time heart rate of the user is still continuously relatively high in a time period T2 (t2-t3), the watch may display, at the moment t3, an interface 2401 shown in (b) in FIG. 34.

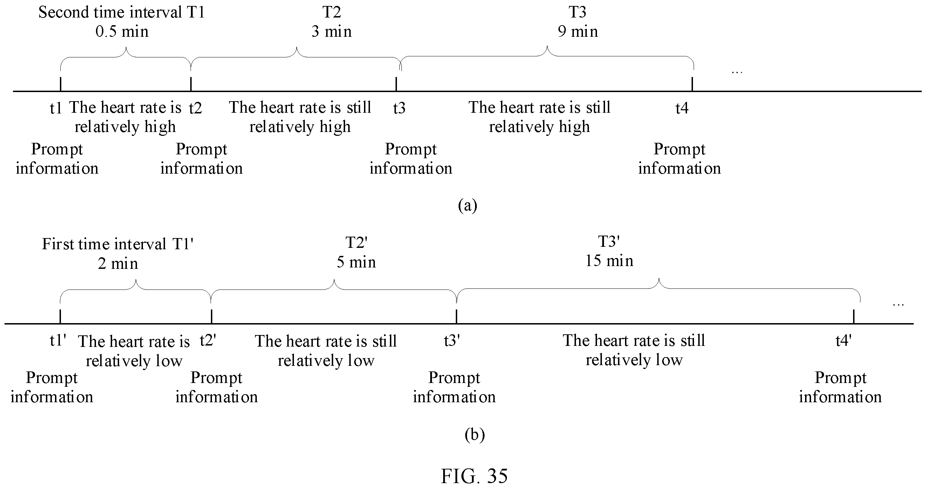

In some embodiments, in a relatively long period of time, if all exercise parameters of the user exceed parameter ranges, a time interval for presenting prompt information may be shortened. For example, as shown in (a) in FIG. 35, if the electronic device detects that the heart rate of the user is continuously relatively high in the time period T1, the prompt information may be presented to prompt the user that the heart rate is continuously relatively high. Then, because the user may not adjust the exercise parameter or for another reason, the electronic device detects that the heart rate of the user is still continuously relatively high, and the prompt information may be presented again at the moment t3. A time interval for presenting the prompt information again is T2 (T2 is greater than the time interval T1). Similarly, then, if the electronic device still detects that the heart rate of the user is continuously relatively high, the prompt information is presented again at a moment t4, and a time interval for presenting the prompt information again is T3 (T3 is greater than T2).

In some embodiments, in a scenario in which an exercise parameter is continuously less than a minimum value of a parameter range, the electronic device may present prompt information to the user at a first time interval. In a scenario in which an exercise parameter is continuously greater than a maximum value of a parameter range, the electronic device may present prompt information to the user at a second time interval. The first time interval is different from the second time interval. Optionally, the first time interval is greater than the second time interval. For example, as shown in (a) in FIG. 35, the second time interval is represented by Tn. As shown in (b) in FIG. 35, the first time interval is represented by Tn'. In some examples, Tn is less than Tn'.

In some embodiments, when a recommended parameter range changes greatly, the electronic device may present prompt information to the user. For example, as shown in (a) in FIG. 16, the watch displays an interface 1101, and the interface 1101 includes a current recommended heart rate range (150 to 160). Then, the watch updates the recommended parameter range in real time based on an exercise parameter and the like in the exercise process of the user. When determining that a difference between the recommended parameter range and a previous recommended parameter range is greater than a threshold, the watch may display the interface 1102 shown in (b) in FIG. 16, and the interface 1102 includes a changed recommended heart rate range (155 to 165).

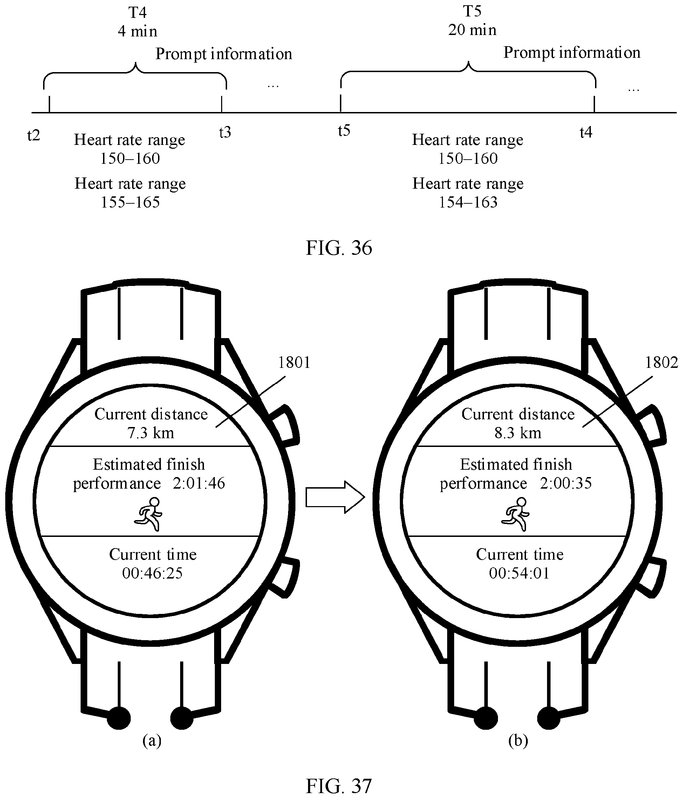

In some embodiments, if duration for which a variation value of the recommended parameter range is greater than or equal to a first threshold reaches second duration, the electronic device presents prompt information to the user. If duration for which the variation value of the recommended parameter range is greater than or equal to a second threshold reaches third duration, the electronic device presents prompt information to the user. In some examples, the second duration is less than the third duration, and the first threshold is greater than the second threshold. It is assumed that the first duration is 4 minutes, the second duration is 20 minutes, the first threshold is 10, and the second threshold is 7. For example, as shown in FIG. 36, at a moment t2, the recommended heart rate range of the user is 150 to 160, and then the recommended heart rate range changes to 155 to 165. In this case, a variation value of the recommended heart rate range is |165-160|+|155-150|=10, and the variation value is greater than or equal to the first threshold 10. If duration for which the variation value of the recommended heart rate range is greater than or equal to 10 reaches 4 minutes, the watch presents prompt information when the duration reaches 4 minutes (for example, at a moment t3).

For another example, as shown in FIG. 36, at a moment t5, the recommended heart rate range of the user is 150 to 160, and then the recommended heart rate range changes to 154 to 163. In this case, a variation value of the recommended heart rate range is 1163-1601+1154-1501=7, and the variation value is greater than or equal to the second threshold 7. If duration for which the variation value of the recommended heart rate range is greater than or equal to 7 reaches 20 minutes, the watch presents prompt information when the duration reaches 20 minutes (for example, at a moment t4).

An embodiment of this application further provides an exercise guidance method. In this method, an electronic device may predict, based on an exercise parameter obtained in real time, duration for completing this exercise. For example, at a specified moment in an exercise process of a user, in response to a preset operation performed by the user, the watch may calculate, based on the exercise parameter obtained in real time, estimated duration for completing this exercise, and display an interface 1801 shown in (a) in FIG. 37. The interface 1801 includes estimated finish performance (for example, 2:01:46). Optionally, the interface 1801 further includes exercise duration (for example, current time is 00:46:25). Optionally, the interface 1801 further includes an exercise distance (for example, a current distance is 7.3 kilometers).

Optionally, the preset operation may be that the user taps a screen of the electronic device, or taps a specific button of the electronic device, or another operation that is used to trigger the electronic device to predict and display information about finish performance.

Optionally, the interface 1801 further includes a real-time heart rate and/or a real-time pace of the user.

Optionally, the interface used to present estimated finish performance to the user may further include a recommended heart rate range and/or a recommended pace range. In other words, the electronic device may present the recommended parameter range and the estimated finish performance to the user on a same interface. For example, as shown in FIG. 23, the interface 912 includes a recommended heart rate range (150 to 160) and estimated finish duration (2:01:46). Optionally, the interface 912 may further include other information.

For example, at another moment in the exercise process of the user, in response to a preset operation performed by the user, the watch may calculate, based on the exercise parameter obtained in real time, estimated duration for completing this exercise, and display an interface 1802 shown in (b) in FIG. 37. The interface 1802 includes estimated finish performance (for example, 2:00:35). Optionally, the interface 1802 further includes exercise duration (for example, current time is 00:54:01). Optionally, the interface 1802 further includes an exercise distance (for example, a current distance is 8.3 kilometers).

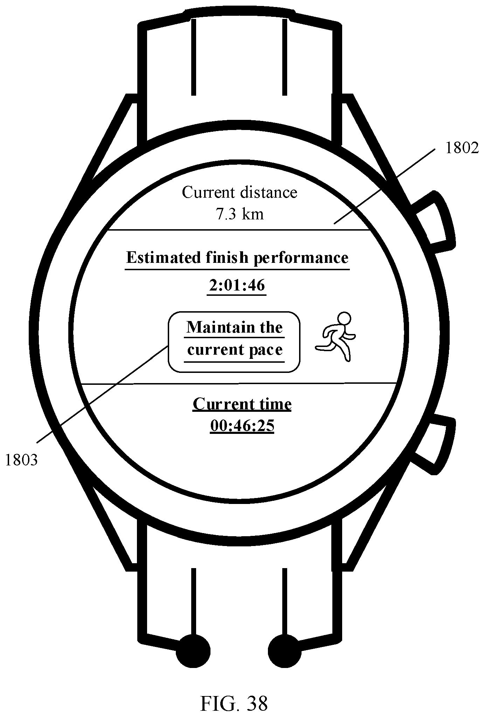

In some embodiments, to attract attention of the user to some important information, this part of important information may be displayed based on specific UI effect. For example, as shown in FIG. 38, the watch displays an interface 1802, and estimated finish performance and current time are presented in a bold and underline manner on the interface 1802. Optionally, the interface 1802 may further include an exercise guidance recommendation 1803 (maintaining the current pace).

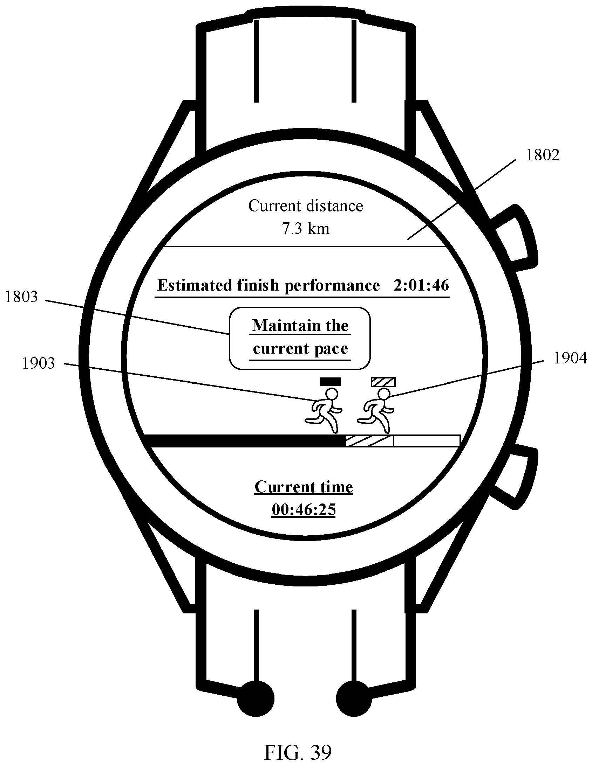

Optionally, the electronic device may present, to the user through a progress bar, an actual movement distance and a movement distance moved at a recommended pace. For example, as shown in FIG. 39, an interface 1802 includes a figure icon 1903 and a figure icon 1904. The figure icon 1903 represents a user who is exercising, and the figure icon 1904 represents a figure (which may be referred to as a pacemaker or a pacer) who exercises at a recommended pace. It should be noted that the foregoing pacemaker may be a person that is virtualized by the electronic device and who exercises at the recommended pace, that is, in an actual exercise process, there may be no pacemaker who exercises at the recommended pace.

In FIG. 39, a black progress bar represents a current actual movement distance (for example, 7.3 kilometers) of the user, and a black and slash progress bar represents a movement distance of the pacemaker. Through different progress bars, the user may intuitively learn about a current exercise progress and a distance difference between the user and the pacemaker. This helps the user adjust exercise intensity of the user based on the progress bar, to narrow the distance difference between the user and the pacemaker.

Optionally, a specific movement speed of the pacemaker is related to an exercise level set by the user. For example, if the user selects an exercise level "challenge yourself", the electronic device may configure the movement speed of the pacemaker as a maximum value of a recommended pace range of the user. In this way, the user may be guided to complete a race at a relatively high pace through the exercise progress bar of the pacemaker moving at a high speed.

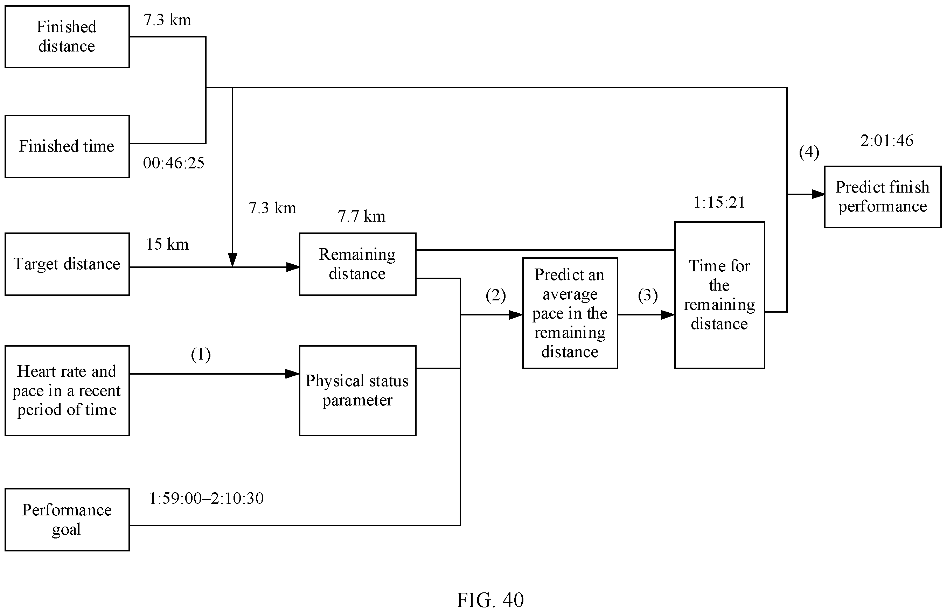

In a possible implementation, FIG. 40 shows a procedure of predicting finish performance. The procedure includes the following steps:
(1). The electronic device obtains a heart rate and a pace of the user in a recent time period, and calculates a physical status of the user based on the heart rate and the pace.
(2). The electronic device calculates an average pace of an unfinished distance (that is, a remaining distance) based on the unfinished distance and a performance goal.

For example, as shown in FIG. 5(e), it is assumed that a mileage of a half marathon selected by the user this time is 15 kilometers, a performance goal (perform well) is 01:59:00 to 02: 10:30, a distance that the user has currently moved is 7.3 kilometers, and exercise duration is 00:46:25. In this case, an unfinished distance is 7.7 kilometers, and the electronic device may calculate a recommended pace range A based on the unfinished distance (7.7 kilometers), the performance goal (01:59:00-02:10:30), and the exercise duration (00:46:25), and determine an average pace of the unfinished distance based on the recommended pace range A.

Optionally, the recommended pace range A=(t1-t2)/L. (Formula 1)

L represents the unfinished distance, t1 represents the performance goal, a value range of t1 is 01:59:00 to 02:10:30, and t2 represents the exercise duration. Optionally, a value of the average pace of the unfinished distance is within the recommended pace range. For example, the electronic device uses an intermediate value of the recommended pace range as the average pace of the unfinished distance, or uses a minimum value of the recommended pace range as the average pace of the unfinished distance; or the electronic device may select another value within the recommended pace range as the average pace of the unfinished distance.

Optionally, the electronic device may further correct the average pace of the unfinished distance based on the physical status of the user in the recent period of time. This means that when the average pace of the unfinished distance is being calculated, not only an exercise parameter such as a target pace required for achieving the performance goal is considered, but also the physical status of the user in the recent period of time needs to be considered, so that the target pace is properly adjusted based on the physical status of the user, to achieve the performance goal as much as possible without exceeding physical load of the user.

In addition, because a latest physical status of the user is calculated based on exercise parameters such as the heart rate and the pace in the recent period of time, a relatively stable physical status of the user in the recent period of time can be represented, and the average pace of the unfinished distance is calculated based on the stable physical status, thereby improving calculation precision.

For example, the electronic device may calculate the recommended pace range of the unfinished distance based on the foregoing formula 1. It is assumed that the recommended pace range is [s1, s2], where s1 is a minimum value of the recommended pace range, and s2 is a maximum value of the recommended pace range. The electronic device may obtain the physical status of the user. In some examples, if the physical status of the user is not good, the electronic device may use the minimum value s1 of the recommended pace range as the average pace of the unfinished distance, to achieve the performance goal as much as possible without exceeding exercise load. On the contrary, if the physical status of the user is relatively good, the electronic device may use s2 as the average pace of the unfinished distance, thereby improving exercise performance of the user.

(3). The electronic device calculates exercise duration of the unfinished distance based on the unfinished distance and the average pace of the unfinished distance.

(4). The electronic device calculates total duration of this exercise (that is, finish performance) based on exercise duration of a finished distance and the exercise duration of the unfinished distance.

For example, assuming that the exercise duration of the finished distance is 00:46:25, and the exercise duration of the unfinished distance calculated by the electronic device is 1:15:21, the finish performance is a sum of the two, that is, 2:01:46.

In the foregoing exercise guidance method, in the exercise process (for example, a running race) of the user, finish performance of the user may be dynamically estimated (the finish performance of the user may be updated in real time) based on an actual exercise parameter of the user (which may be used to represent a physical status of the user) and a preset performance goal. In some cases, if the physical status of the user is stable, the user may complete the current exercise based on the preset performance goal. In some other cases, if it is detected that the physical status of the user is unstable or a requirement of a specified goal is not met, the electronic device recommends, to the user based on the physical status of the user, a recommended parameter value range that is suitable for the current physical status and that minimizes a difference from the performance goal. In this way, finish performance that conforms to the physical status of the user can be obtained.

The foregoing is mainly described by using an example in which the second electronic device is a watch, and the second electronic device calculates a corresponding parameter and displays a corresponding interface. In some other embodiments, all the foregoing steps performed by the second electronic device may be alternatively performed by the first electronic device or another device; or the first electronic device and the second electronic device perform the foregoing steps together (with another device), where the first electronic device performs a part of the foregoing steps, and the second electronic device performs the other part of the steps; or the first electronic device, the second electronic device, and a third electronic device may perform the foregoing steps together. In some other embodiments, a server may alternatively complete a related step such as parameter calculation. An execution body of each method step is not limited in this embodiment of this application.

For example, the exercise guidance method in this embodiment of this application may be further applied to any of the following scenarios.

Scenario 1: A user wears a heart rate band and a watch (or a mobile phone) in an exercise process. The heart rate band is configured to measure a real-time heart rate of the user. The watch may obtain the real-time heart rate of the user from the heart rate band, and prompt the user when the real-time heart rate exceeds a recommended heart rate. The prompt may be performed in a voice, vibration, and/or display manner on the heart rate band and/or the watch (or the mobile phone).

Scenario 2: A user runs indoors on a treadmill, and the user wears an apparatus (for example, a watch) for measuring a heart rate. When detecting that a heart rate of the user exceeds a recommended heart rate range, the watch sends an instruction to the treadmill, and the treadmill displays a real-time heart rate of the user and the recommended heart rate range. The user may clearly observe heart rate prompt information through a large display of the treadmill, and adjust a pace accordingly.

Optionally, in this scenario, the treadmill may measure a real-time pace of the user, and may send the pace to the watch. When detecting that the pace of the user exceeds a recommended pace range, the watch sends an instruction to the treadmill, and the treadmill displays the real-time pace of the user and the recommended pace range.

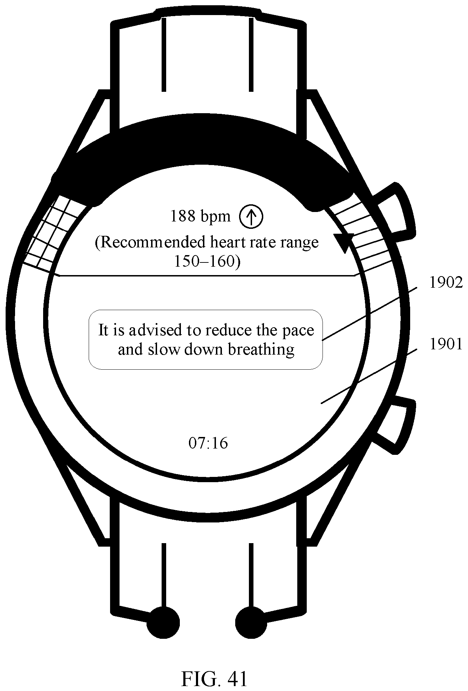

It should be noted that the foregoing embodiments are mainly described by using an example in which the electronic device guides the user to adjust a heart rate and/or a pace. In some other embodiments, the electronic device may alternatively guide the user to adjust other exercise parameters. For example, the electronic device may alternatively guide a breathing adjustment manner of the user. For example, as shown in FIG. 41, if it is detected that a heart rate of the user is greater than 160, the watch displays an interface 1901. The interface 1901 includes an exercise guidance advice 1902 that is used to prompt the user to reduce the pace and slow down a breathing rhythm through adjustment.

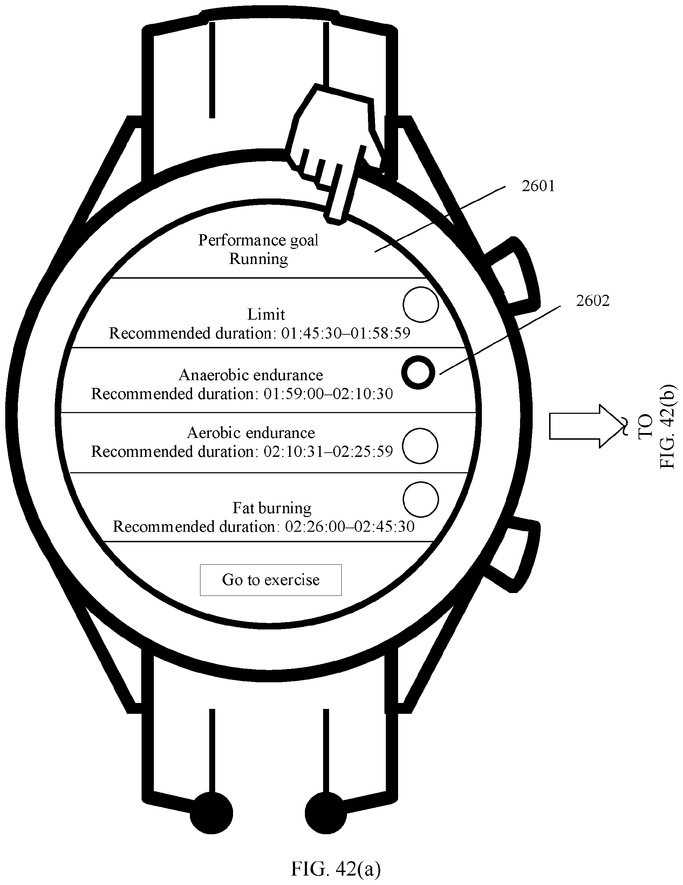

The foregoing embodiments are mainly described by using an exercise level such as "challenge yourself" as an example. In some other embodiments, the electronic device may alternatively set other exercise levels. For example, as shown in FIG. 42(a), the electronic device may display an exercise level setting interface 2601, and the interface 2601 includes information about a plurality of exercise levels. For example, the exercise levels may include a limit, anaerobic endurance, aerobic endurance, fat burning, and the like. Each exercise level corresponds to one performance interval (or referred to as a performance range). The performance interval is determined based on a stable exercise status (including an exercise ability and/or exercise load) of the user in a recent period of time.

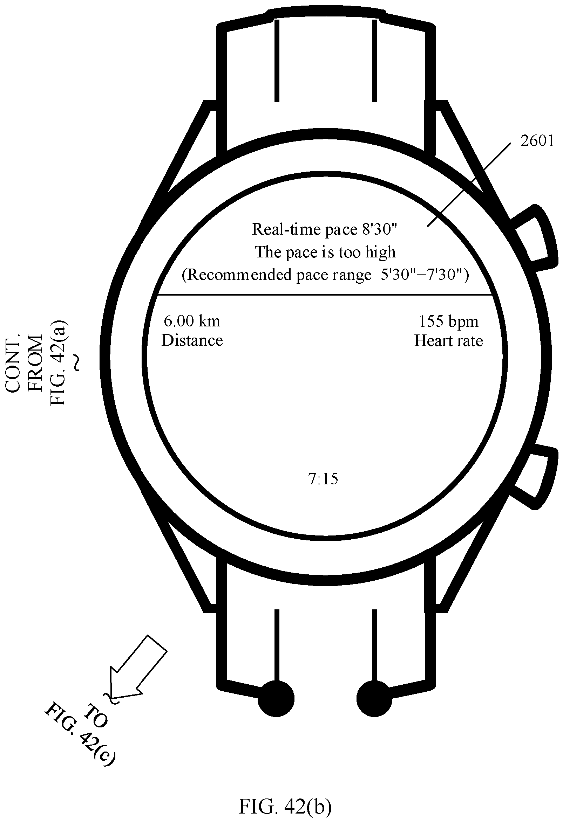

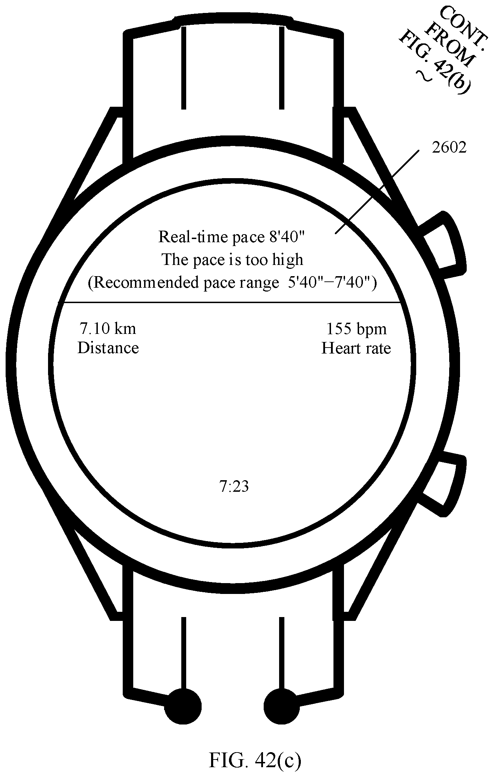

In response to an operation of tapping an anaerobic endurance option 2602 by the user, the electronic device determines that a target exercise level of this exercise is anaerobic endurance. Subsequently, in the exercise process, the electronic device may adjust a recommended parameter range based on the target exercise level (anaerobic endurance), the exercise status of the user in the recent period of time, and a real-time physical status of the user. For example, as shown in FIG. 42(b), at 7:15, the electronic device displays an interface 2601, and the interface 2601 includes a corresponding real-time pace (8'30") of the user at 7:15 and a recommended pace range (5'30" to 7'30"). Then, as shown in FIG. 42(c), at 7:23, the electronic device displays an interface 2602, and the interface 2602 includes a corresponding real-time pace (8'40") of the user at 7:23 and a recommended pace range (5'40" to 7'40").

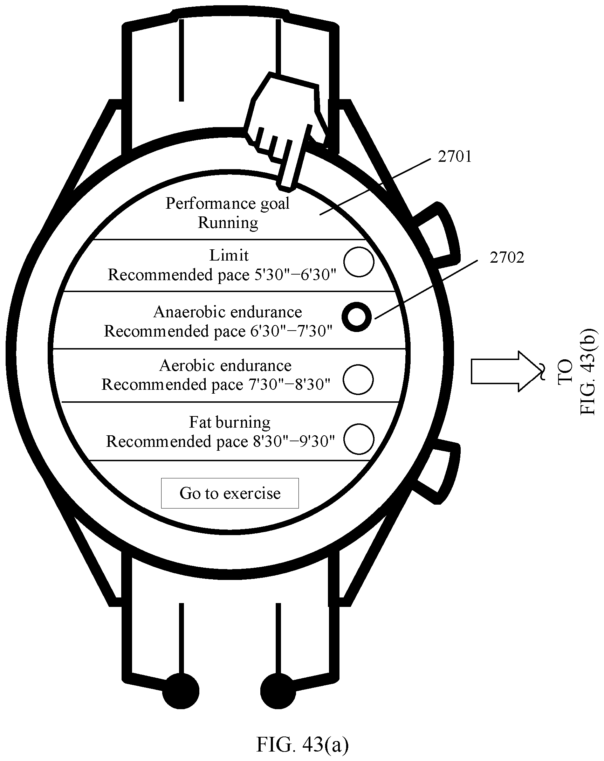

The foregoing is mainly described by using an example in which the performance interval is a duration interval. In some other embodiments, the performance interval may be another interval. For example, the performance interval may be a pace interval. For example, as shown in FIG. 43(a), the electronic device may display an exercise level setting interface 2701, and the interface 2701 includes information about a plurality of exercise levels. For example, the exercise levels may include a limit, anaerobic endurance, aerobic endurance, fat burning, and the like. Each exercise level corresponds to one pace interval (or referred to as a pace range). The pace interval is determined based on a stable exercise status (including an exercise ability and/or exercise load) of the user in a recent period of time.

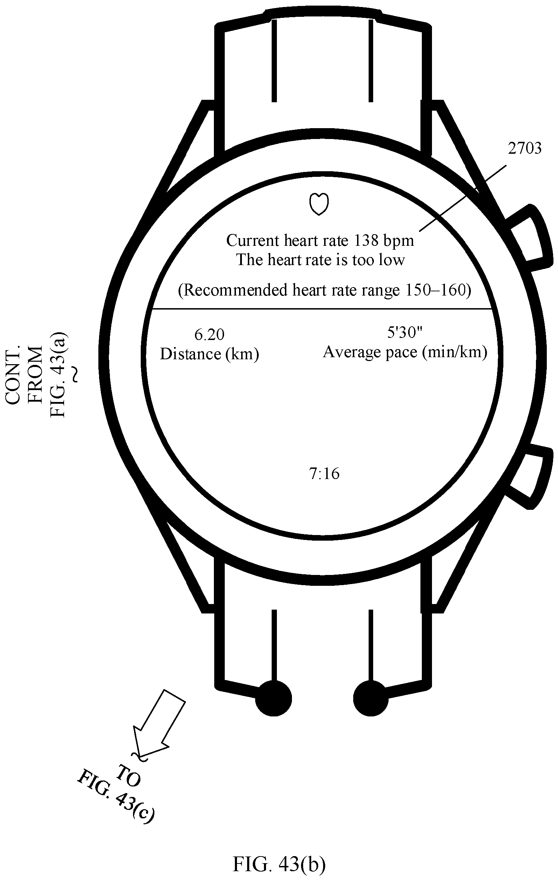

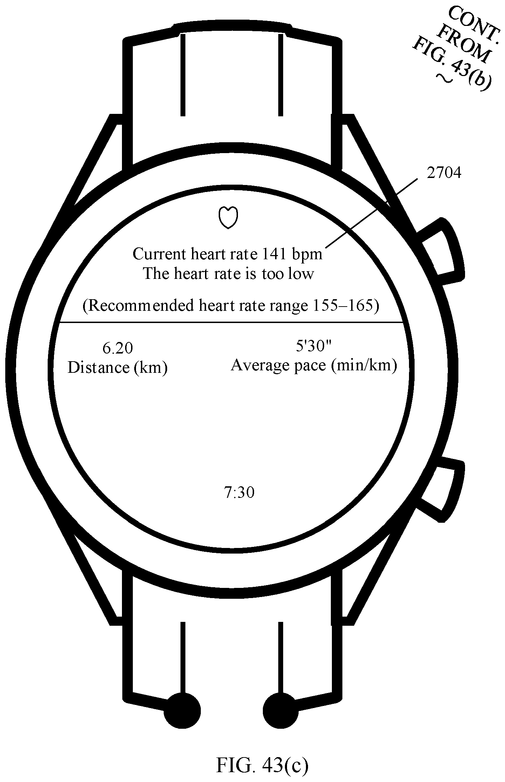

In response to an operation of tapping an anaerobic endurance option 2702 by the user, the electronic device determines that a target exercise level of this exercise is anaerobic endurance. Subsequently, in the exercise process, the electronic device may adjust a recommended parameter range based on the target exercise level (anaerobic endurance), the exercise status of the user in the recent period of time, and a real-time physical status of the user. For example, as shown in FIG. 43(b), at 7:16, the electronic device displays an interface 2703, and the interface 2703 includes a corresponding real-time heart rate (138 bpm) of the user at 7:16 and a recommended heart rate range (150 to 160). Then, as shown in FIG. 43(c), at 7:30, the electronic device displays an interface 2704, and the interface 2704 includes a corresponding real-time heart rate (141 bpm) of the user at 7:30 and a recommended heart rate range (155 to 165).

The foregoing embodiments are mainly described by using running exercise as an example. It may be understood that the exercise guidance method in this embodiment of this application may be further applied to another type of exercise, for example, applied to guidance on exercise such as an elliptical trainer, cycling, walking, and swimming. The exercise guidance may be race guidance, training guidance, or another type of exercise guidance.

In some other types of exercise, a heart rate may be used to represent exercise intensity. The electronic device may collect a real-time heart rate of the user. When the real-time heart rate exceeds a recommended heart rate range, the electronic device may prompt the user to adjust the exercise intensity. For example, in a process in which the user performs fat burning exercise, if it is detected that the real-time heart rate of the user is less than a lower limit of a recommended heart rate, the electronic device may prompt the user to increase the exercise intensity, to achieve fat burning effect.

The foregoing is mainly described by using an example in which a running ability index is used as an exercise ability in running exercise. In another type of exercise, an exercise ability may be determined based on an exercise record of the corresponding type of exercise performed by the user. For example, in swimming exercise, an exercise ability of the swimming exercise may be determined based on a previous exercise record (for example, time spent on each time of swimming, a heart rate, and a frequency of the swimming exercise) of the user.

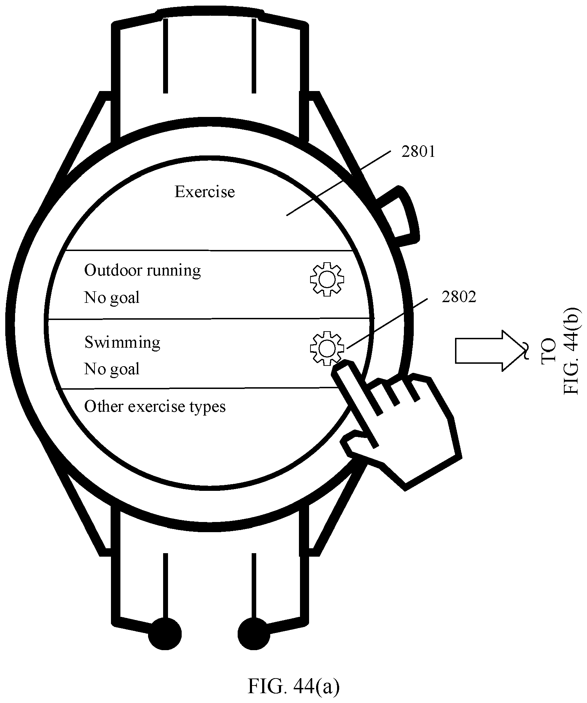

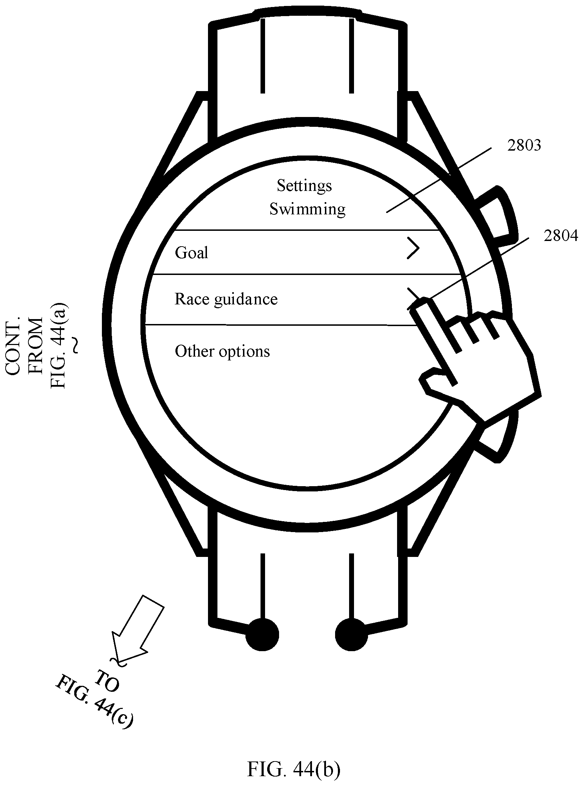

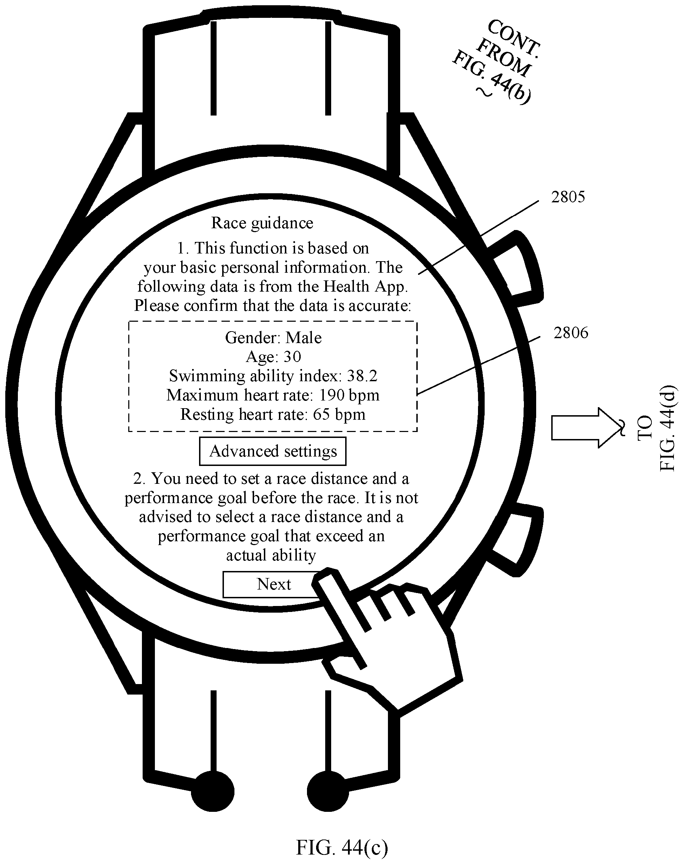

For example, the user may set a performance interval of the swimming exercise. As shown in FIG. 44(a), the electronic device displays an interface 2801, and the interface 2801 includes a swimming exercise option 2802. After detecting that the user taps the option 2802, the electronic device jumps to an interface 2803 shown in FIG. 44(b), and the interface 2803 includes a race guidance option 2804. After detecting that the user taps the option 2804, the electronic device may jump to an interface 2805 shown in FIG. 44(c), and the interface 2805 includes a plurality of parameters 2806, for example, including a swimming ability index. The swimming ability index may be used as an exercise ability of the user in the swimming exercise.

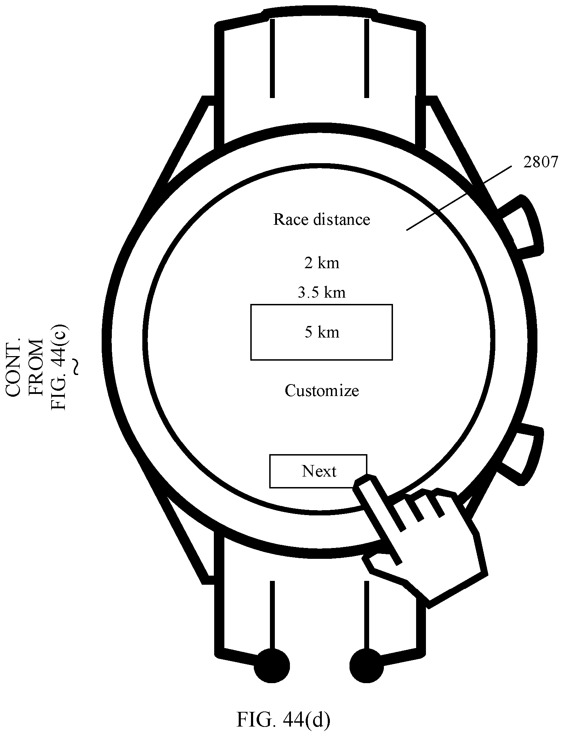

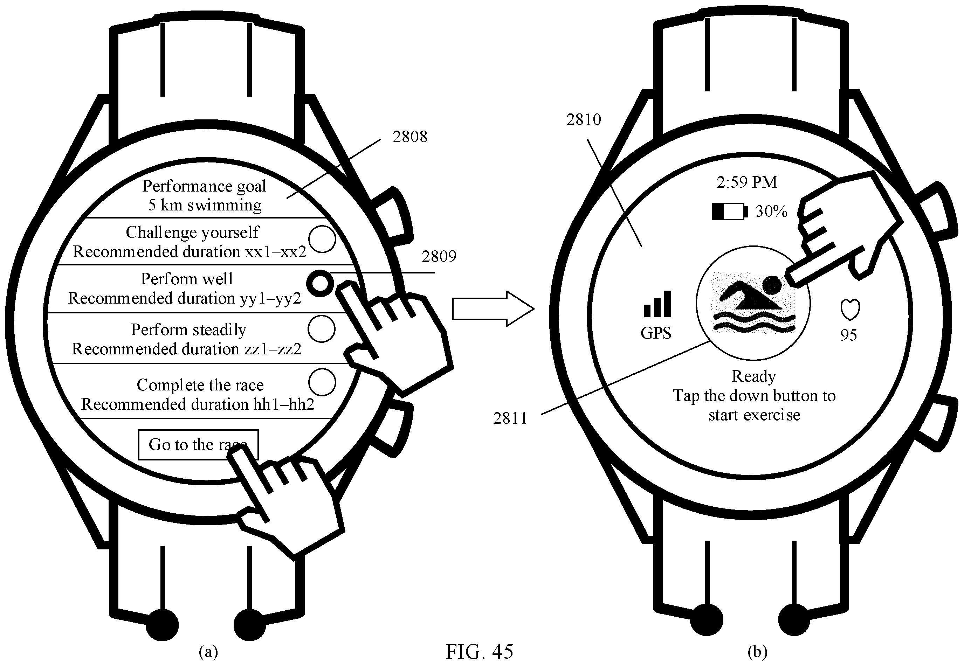

After detecting an operation of tapping a "next" button on the interface 2805 by the user, the electronic device may jump to an interface 2807 shown in FIG. 44(d). The user may set a target distance of a swimming race through the interface 2807. After detecting an operation in which the user selects a race distance 5 km and taps the "next" button, the electronic device may jump to an interface 2808 shown in (a) in FIG. 45. The user may tap an option 2809 "perform well" on the interface 2808, to trigger the electronic device to jump to an interface 2810 shown in (b) in FIG. 45. The interface 2810 includes a control 2811, and the user may tap the control 2811 to start a swimming race.

Optionally, the electronic device may alternatively determine a prompt manner of an exercise guidance recommendation based on an exercise type of the user. For example, if the user is currently performing cycling exercise, the user is prompted to adjust an exercise parameter and/or is prompted of finish duration preferentially through a voice prompt.

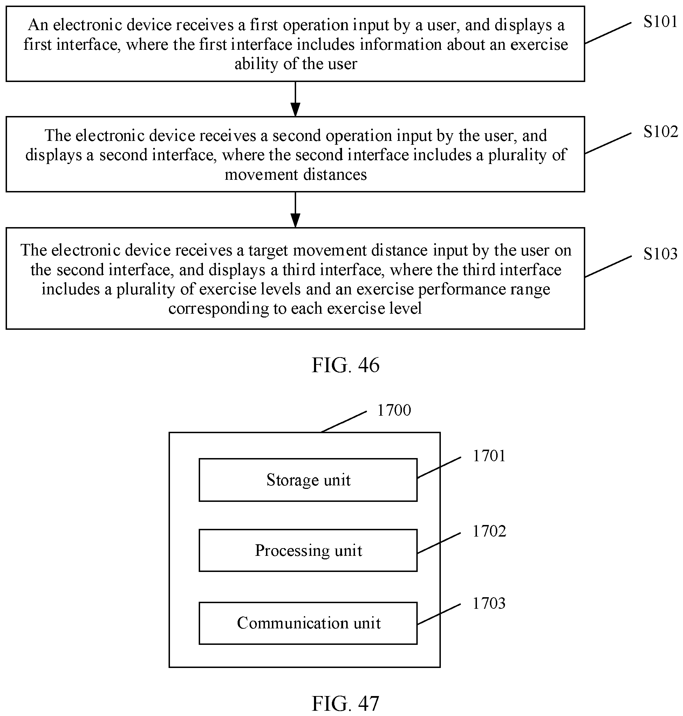

FIG. 46 shows an example procedure of an exercise guidance method according to an embodiment of this application. As shown in FIG. 46, the method includes the following steps:

S101: An electronic device receives a first operation input by a user, and displays a first interface, where the first interface includes information about an exercise ability of the user.

For example, as shown in FIG. 5(b), the electronic device displays an interface 503, and the interface 503 includes a race guidance option 504. The electronic device receives a tap operation (an example of the first operation) performed by the user on the race guidance option 504, and displays an interface 505 (the first interface) shown in FIG. 5(c). The interface 505 includes a running ability index (that is, the exercise ability) of the user.

S102: The electronic device receives a second operation input by the user, and displays a second interface, where the second interface includes a plurality of movement distances.

For example, still as shown in FIG. 5(c), the electronic device receives an operation (an example of the second operation) of tapping a "Next" button by the user on the interface 505, and displays an interface 507 (the second interface). The interface 507 includes a plurality of movement distances such as 5 km and 10 km.

S103: The electronic device receives a target movement distance input by the user on the second interface, and displays a third interface.

Still as shown in FIG. 5(d), the user may select the target movement distance (for example, select a half marathon) on the interface 507 (the second interface).

The third interface includes a plurality of exercise levels and an exercise performance range corresponding to each exercise level, and the exercise performance range is determined based on the corresponding exercise level, the target movement distance, and the exercise ability.

For example, as shown in FIG. 5(e), the electronic device displays an interface 508 (an example of the third interface), and the interface 508 includes exercise levels such as challenge yourself and perform well, and an exercise performance range corresponding to each exercise level. For example, an exercise performance range of the exercise level challenge yourself is 01:45:30 to 01:58:59.

In a possible design, the method further includes: receiving a first target exercise level input by the user on the third interface, and displaying a first exercise interface, where the first target exercise level corresponds to a first exercise performance range.

For example, still as shown in FIG. 5(e), the electronic device receives the exercise level perform well (the first target exercise level) input by the user on the interface 508 (the third interface), and may jump to an interface 510 (an example of the first exercise interface) shown in FIG. 5(f). A first exercise performance range corresponding to the exercise level perform well is 01:59:00 to 02:10:30.

In a possible design, after the displaying a first interface, the method further includes:
displaying a fourth interface, where the fourth interface includes a first control used to modify the first exercise ability; and
receiving an operation performed by the user on the first control, and displaying a fifth interface, where the fifth interface includes an option used to modify the first exercise ability.

For example, as shown in (a) in FIG. 10, a watch displays an interface 601 (an example of the fourth interface), and the interface 601 includes a control 602 (an example of the first control) used to modify a running ability index (an example of the first exercise ability). An operation performed by the user on the control 602 is received. The watch displays an interface 603 (an example of the fifth interface) shown in (b) in FIG. 10, and the interface 603 includes an option used to modify the running ability index.

In a possible design, as shown in FIG. 5(c), the interface 505 may further include heart rate information.

As shown in (a) in FIG. 10, the interface 601 may include a control (an example of a second control) used to modify the heart rate information (for example, a maximum heart rate or a resting heart rate).

In a possible design, the method further includes: displaying a second exercise interface in response to a third operation input by the user, where
the second exercise interface includes a current exercise parameter of the user, an annular indication region located on an interface edge, and a pointer; and the pointer points to the annular indication region, the annular indication region includes a first indication region, a second indication region, and a third indication region, a first end of the first indication region is connected to a second end of the second indication region, and a second end of the first indication region is connected to a first end of the third indication region, where
the first indication region includes an annular region corresponding to a first parameter range, and the first parameter range is a recommended range of the electronic device for the exercise parameter; and the second indication region includes an annular region greater than a maximum value of the first parameter range, the third indication region includes an annular region less than a minimum value of the first parameter range, and a location at which the pointer points to the annular indication region indicates whether the current exercise parameter of the user exceeds the first parameter range.

For example, as shown in FIG. 5(f), in response to an operation (an example of the third operation) of tapping a control 511 or tapping a down button by the user, the watch displays an interface 905 (an example of the second exercise interface) shown in FIG. 18.

The interface 905 includes a current exercise parameter (a heart rate 155 bpm) of the user, an annular indication region located at an interface edge, and a pointer 904, and the pointer 904 points to the annular indication region. The annular indication region includes a first indication region 901, a second indication region 903, and a third indication region 902. A first end (for example, a left end) of the first indication region 901 is connected to a second end (for example, a right end) of the second indication region 903, and a second end (for example, a right end) of the first indication region 901 is connected to a first end (for example, a left end) of the third indication region 902.

The first indication region 901 includes an annular region corresponding to a first parameter range (for example, a recommended heart rate range 150 to 160), the second indication region 903 includes an annular region greater than a maximum value of the first parameter range (for example, a maximum value 160 of the recommended heart rate range), the third indication region 902 includes an annular region less than a minimum value of the first parameter range (for example, a minimum value 150 of the recommended heart rate range), and a location at which the pointer 904 points to the annular indication region indicates whether the current exercise parameter of the user exceeds the first parameter range (for example, the location at which the pointer 904 points to the annular indication region indicates whether the current exercise parameter of the user exceeds the recommended heart rate range 150 to 160).

Whether an exercise parameter exceeds a parameter range means whether the exercise parameter is greater than a maximum value of the parameter range or less than a minimum value of the parameter range. As shown in FIG. 18, when a real-time heart rate is greater than the maximum value 160 of the recommended heart rate range, the real-time heart rate exceeds the recommended heart rate range. When the real-time heart rate is less than the minimum value 150 of the recommended heart rate range, the real-time heart rate also exceeds the recommended heart rate range.

The foregoing is mainly described by using an example in which an operation of the user triggers display of an exercise interface. In some other embodiments, display of the exercise interface in this embodiment of this application may not be triggered based on the operation of the user. The electronic device may automatically display the exercise interface when detecting that a specific condition is met.

In a possible design, when the current exercise parameter of the user exceeds the first parameter range, the pointer points to the second indication region or the third indication region, the second exercise interface further includes first prompt information, and the first prompt information is used to prompt the user that the current exercise parameter exceeds the first parameter range.

For example, FIG. 19 shows another example of the second exercise interface. A second exercise interface 907 may further include first prompt information (a heart rate is too high), the first prompt information is used to prompt the user that the current exercise parameter exceeds the first parameter range (for example, a prompt that a real-time heart rate exceeds a recommended heart rate range 155 to 165), and the pointer points to the third indication region 902.

For example, FIG. 22 shows another example of the second exercise interface. A second exercise interface 910 may further include first prompt information 911 (a heart rate is too high), the first prompt information is used to prompt the user that the current exercise parameter exceeds the first parameter range (for example, a prompt that a real-time heart rate exceeds a recommended heart rate range 150 to 160), and the pointer points to the second indication region 903.

In a possible design, the second exercise interface further includes the first parameter range.

For example, as shown in FIG. 22, the interface 910 includes the recommended heart rate range 150 to 160.

In a possible design, the method further includes:
obtaining a first exercise parameter of the user in an exercise process; and
displaying a third exercise interface when the first exercise parameter exceeds the first parameter range for more than preset duration, where the third exercise interface is used to recommend the user to adjust a second exercise parameter. The first exercise parameter and the second exercise parameter may be the same or different. In a possible design, the first exercise parameter includes a heart rate, and the second exercise parameter includes a pace.

For example, the first exercise parameter is a heart rate, and the second exercise parameter is a pace. For example, as shown in FIG. 23, when duration for which the heart rate is greater than the maximum value (for example, 160 bpm) of the recommended heart rate range exceeds preset duration, an interface 912 (an example of the third exercise interface) is displayed, and the interface 912 is used to recommend the user to reduce the pace.

For another example, as shown in FIG. 22, when duration for which the heart rate is less than the minimum value (for example, 150 bpm) of the recommended heart rate range exceeds preset duration, the watch displays the interface 910, and the interface 910 is used to recommend the user to increase the pace.

In some other examples, the third exercise interface may be alternatively presented in a form of a card or the like.

In a possible design, the method further includes:
displaying a second exercise interface, where the second exercise interface includes a first parameter range, and the first parameter range is related to a first physical status of the user at a first moment in an exercise process; and
displaying a fourth exercise interface, where the fourth exercise interface includes a second parameter range, and the second parameter range is related to a second physical status of the user at a second moment in the exercise process, where the first parameter range is different from the second parameter range, and the first moment is different from the second moment.

For example, in the exercise process, the watch displays an interface 905 (an example of the second interface) shown in FIG. 18, the interface 905 includes a recommended heart rate range 150 to 160 (an example of the first parameter range), and the recommended heart rate range is related to a first physical status of the user at a first moment 7:16 in the exercise process. In a possible design, the first physical status is determined based on an exercise parameter of the user at the first moment 7:16, or is determined based on an exercise parameter of the user in a time period around 7:16.

For another example, the watch displays an interface 907 (an example of the fourth exercise interface) shown in FIG. 19, the interface 907 includes a recommended heart rate range 155 to 165 (an example of the second parameter range), and the second parameter range is related to a second physical status of the user at a second moment 7:30 in the exercise process. The second physical status is determined based on an exercise parameter of the user at the second moment 7:30.

In a possible design, the exercise parameter includes one or more of a heart rate, a pace, and a respiratory rate.

In a possible design, second prompt information is presented when a difference between the second parameter range and the first parameter range is greater than a threshold.

Optionally, the difference between the second parameter range and the first parameter range may be determined based on a maximum value of the second parameter range, a maximum value of the first parameter range, a minimum value of the second parameter range, and a minimum value of the first parameter range. For example, the first parameter range is a recommended heart rate range 150 to 160, and the second parameter range is a recommended heart rate range 155 to 165. A difference between the recommended heart rate range 150 to 160 and the recommended heart rate range 155 to 165 may be |165-160|+|155-150|=10, where ∥ is an absolute value symbol.

In a possible design, the second exercise interface includes estimated duration for completing the target movement distance, and the estimated duration is determined based on the first physical status of the user, the target movement distance, and the first exercise performance range.

For example, as shown in FIG. 23, the interface 912 (an example of the second exercise interface) includes estimated duration 2:01:46. The estimated duration is determined based on the first physical status of the user at the moment 7:16, the target movement distance (for example, a distance corresponding to the half marathon shown in FIG. 5(d)), and the first exercise performance range (for example, a performance range 01:59:00 to 02:10:30 shown in FIG. 5(c)).

In a possible design, the second exercise interface includes one or more of the following: a current finished movement distance, current exercise duration, and a current exercise parameter.

For example, as shown in FIG. 23, the interface 912 (an example of the second exercise interface) includes a current finished movement distance 6.2 km, current exercise duration (for example, 00:46:25), and a current exercise parameter (for example, 188 bpm).

In a possible design, the second exercise interface further includes a first icon, a second icon, and a progress bar, the first icon indicates, on the progress bar, a location of the current finished movement distance of the user in the target movement distance, and the second icon indicates, on the progress bar, a location that is of the user in the target movement distance and that is expected based on the first exercise performance range.

For example, as shown in FIG. 39, an interface 1802 (an example of the second exercise interface) further includes an icon 1904 (an example of the first icon), an icon 1903 (an example of the second icon), and a progress bar. The icon 1904 indicates, on the progress bar, a location of the current finished movement distance of the user in the target movement distance, and the icon 1903 indicates, on the progress bar, a location that is of the user in the target movement distance and that is expected based on the first exercise performance range (for example, a performance range corresponding to an exercise level perform well).

In a possible design, the method further includes:
displaying a sixth interface, where the sixth interface includes third prompt information, and the third prompt information is used to prompt the user to do exercise to obtain the first exercise ability.

For example, as shown in FIG. 8, the watch displays an interface 605 (an example of the sixth interface), the interface 605 includes third prompt information, and the third prompt information is used to prompt the user to do exercise, to obtain the first exercise ability (for example, the running ability index).

In a possible design, the method further includes:
displaying a seventh interface, where the seventh interface includes the first target exercise level, and the first target exercise level corresponds to a second exercise performance range; the second exercise performance range is determined based on the first target exercise level, the target movement distance, and a second exercise ability of the user; and the second exercise performance range is different from the first exercise performance range, and the first exercise ability is different from the second exercise ability.

After the running ability index changes, a performance goal (recommended duration) of exercise at a corresponding level changes accordingly. For example, (a) and FIG. 12(b) are setting interfaces before the user exercises. Then, the user increases a quantity of running times or increases exercise load, resulting in an increase in the running ability index. When the user sets race guidance by using the watch, the watch displays a race guidance prompt control 520 shown in FIG. 13(a), where a running ability index in the control 520 is increased compared with a running ability index in a control 514 shown in FIG. 12(a). Then, when the user sets a performance goal by using the watch, the watch displays an interface 521 (an example of the seventh interface) shown in FIG. 13(b), and a performance goal (recommended duration) of exercise at each level on the interface 521 is shortened. The seventh interface includes the first target exercise level (for example, an exercise level perform well), the first target exercise level corresponds to a second exercise performance range (01:49:00 to 02:00:30), the second exercise performance range is determined based on the first target exercise level, the target movement distance, and the second exercise ability of the user. The second exercise performance range is different from the first exercise performance range (01:59:00 to 02:10:30), and the first exercise ability is different from the second exercise ability (for example, the second exercise ability is greater than the first exercise ability).

In a possible design, the displaying a third exercise interface includes:
displaying the third exercise interface within first duration, and displaying a fifth exercise interface after the first duration, where UI effect of the fifth exercise interface is different from UI effect of the third exercise interface.

For example, duration for which the watch displays the third exercise interface 912 shown in FIG. 23 is 4s. After 4s, the watch automatically switches to another interface.

In a possible design, after the displaying a third exercise interface, a sixth exercise interface is displayed in response to a fourth operation input by the user, where UI effect of the sixth exercise interface is different from UI effect of the third exercise interface.

For example, after the watch displays the third exercise interface 912 shown in FIG. 23, if an operation of the user such as performing leftward or rightward sliding or tapping a down button, the watch may switch to another exercise interface.

In a possible design, the method further includes:
displaying a seventh exercise interface in the exercise process of the user in response to a fifth operation input by the user, where the seventh exercise interface includes a third control used to modify an exercise level.

For example, as shown in FIG. 29(a), the watch displays an exercise interface 1501, to prompt the user that the current heart rate is too high. In some examples, if a rightward slide operation (an example of the fifth operation) of the user is detected, the watch jumps to an interface 1704 (an example of the seventh exercise interface) shown in FIG. 29(b), and the interface 1704 includes a control 1805 (an example of the third control).

In a possible design, after the displaying a seventh exercise interface, the method further includes:
receiving an operation performed by the user on the third control, and displaying an eighth exercise interface, where the eighth exercise interface includes information about a plurality of exercise levels; and
receiving a second target exercise level input by the user on the eighth exercise interface, and displaying a ninth exercise interface, where the second target exercise level is different from the first target exercise level.

In some examples, if it is detected that the user taps the control 1805, the watch may jump to a performance goal setting interface 508 (an example of the eighth exercise interface) shown in FIG. 29(c). As shown in FIG. 29(c), an exercise level previously selected by the user is "perform well". Then, after detecting that an option 1804 corresponding to an exercise level "challenge yourself" (the second target exercise level input by the user) is tapped, the watch modifies a current exercise goal, as shown on an interface 522 (an example of the ninth exercise interface) shown in FIG. 29(d).

Subsequently, the watch may determine a recommended heart rate range and/or a recommended pace range based on the modified performance goal. In some examples, the watch may further dynamically estimate finish time of the user based on the modified performance goal.

In a possible design, the method further includes:
displaying fourth prompt information on the third interface, where the fourth prompt information is used to prompt the user to select a third target exercise level, and the third target exercise level is different from the first target exercise level.

For example, an exercise level previously selected by the user is an exercise level perform well. As shown in FIG. 11, the watch pops up a pop-up window 519 (an example of the fourth prompt information) on an interface 518, to prompt the user to select an exercise level challenge yourself (an example of the third target exercise level).

In a possible design, before the receiving a first operation input by a user, the method further includes:
displaying an eighth interface, where the eighth interface includes a plurality of exercise types; and
receiving a target exercise type input by the user on the eighth interface, and displaying a ninth interface, where
the receiving a first operation input by a user includes receiving the first operation that is input by the user and that is performed on the ninth interface.

For example, as shown in FIG. 5(a), the watch may display an interface 501 (an example of the eighth interface), and the interface 501 includes a plurality of exercise types. A target exercise type (for example, running) input by the user on the interface 501 is received, and an interface 503 (an example of the ninth interface) shown in FIG. 5(b) is displayed.

The receiving a first operation input by a user includes receiving the first operation that is input by the user and that is performed on the interface 503.

In a possible design, the exercise includes any one of the following types of exercise: running, swimming, and cycling.

In a possible design, the exercise includes running, the first exercise ability is a running ability index, and the running ability index is related to an exercise record of the user.

In a possible design, the electronic device includes a wearable device or a mobile terminal device.

The foregoing one or more interfaces are all examples, and there may be another interface design manner. A specific interface design manner and a switching manner between interfaces (for example, switching between interfaces may be performed by perform sliding upward and downward on an interface, or interface switching may be implemented by pressing a specific button) are not limited in this application.

Optionally, elements on some interfaces may be combined with each other to form a new interface. For example, that it is advised to increase a pace on the interface 912 in FIG. 23 is combined with an interface element in FIG. 19.

The foregoing mainly uses an example in which a display of the watch is in a circular (oval) shape. In some other examples, the display of the watch may be alternatively square, odd-form, or the like. Taking a square display as an example, the square display may also have an annular region at an edge.

In the foregoing method, a unit of each exercise parameter is an example, and other units may be used. For example, a unit of a pace is (min/km). In some other embodiments, there may be another unit (for example, km/h) of a pace.

It should be noted that the foregoing embodiments may be combined, and a combined solution is implemented. Optionally, some operations in the procedures of the method embodiments are optionally combined, and/or a sequence of some operations is optionally changed. In addition, an execution sequence between the steps of each procedure is merely an example, and does not constitute a limitation on an execution sequence between the steps. The steps may be performed in another execution sequence. It is not intended to indicate that the execution sequence is the only sequence in which these operations may be performed. A person of ordinary skill in the art may figure out various manners of re-arranging the operations described in this specification. In addition, it should be noted that process details related to an embodiment in this specification are also applicable to other embodiments in a similar manner, or different embodiments may be combined.

In addition, some steps in the method embodiments may be equivalently replaced with other possible steps. Alternatively, some steps in the method embodiments may be optional, and may be deleted in some use scenarios. Alternatively, other possible steps may be added in the method embodiments.

In addition, the foregoing method embodiments may be implemented separately or in combination.

For example, in the procedures of the algorithms in FIG. 14B, FIG. 40, and the like, some steps may be equivalently replaced with other possible steps. Alternatively, some steps in the method embodiments may be optional, and may be deleted in some use scenarios. Alternatively, other possible steps may be added in the method embodiments, or there are other implementations. A specific algorithm is not limited in the embodiments of this application.

It may be understood that, to implement the foregoing functions, the electronic device provided in the embodiments of this application includes corresponding hardware structures and/or software modules for performing the functions. With reference to the units and algorithm steps described in the embodiments disclosed in this application, the embodiments of this application can be implemented in a form of hardware or hardware and computer software. Whether a function is performed by hardware or hardware driven by computer software depends on particular applications and design constraints of the technical solution. A person skilled in the art may use different methods to implement the described functions for each particular application, but it should not be considered that the implementation falls beyond the scope of the technical solutions in embodiments of this application.

In the embodiments of this application, function unit division may be performed on the device based on the foregoing method example. For example, each function unit may be obtained through division based on each corresponding function, or two or more functions may be integrated into one processing unit. The integrated unit may be implemented in a form of hardware, or may be implemented in a form of a software function unit. It should be noted that, in the embodiments of this application, unit division is an example, and is merely logical function division. In actual implementation, another division manner may be used.

FIG. 47 is a schematic block diagram of an apparatus according to an embodiment of this application. The apparatus may be the foregoing receiving device or the foregoing sending device. The apparatus 1700 may exist in a form of software, or may be a chip that can be used in a device. The apparatus 1700 includes a processing unit 1702 and a communication unit 1703. Optionally, the communication unit 1703 may be further divided into a sending unit (not shown in FIG. 47) and a receiving unit (not shown in FIG. 47). The sending unit is configured to support the apparatus 1700 in sending information to another device, and the receiving unit is configured to support the apparatus 1700 in receiving information from another device.

Optionally, the apparatus 1700 may further include a storage unit 1701, configured to store program code and data of the apparatus 1700. The data may include but is not limited to original data, intermediate data, or the like.

The processing unit 1702 may be configured to support the receiving device in performing a control-related operation. The communication unit 1703 is configured to support communication between the receiving device and another device.

The apparatus 1700 may further include a display unit (not shown in FIG. 47) and an input unit (not shown in FIG. 47).

In a possible manner, the processing unit 1702 may be a controller or the processor 501 shown in FIG. 3, for example, may be a central processing unit (Central Processing Unit, CPU), a general-purpose processor, a digital signal processor (Digital Signal Processor, DSP), an application-specific integrated circuit (Application-Specific Integrated Circuit, ASIC), a field programmable gate array (Field Programmable Gate Array, FPGA) or another programmable logic device, a transistor logic device, a hardware component, or any combination thereof. The processing unit may implement or execute various example logical blocks, modules, and circuits described with reference to content disclosed in this application. Alternatively, the processor may be a combination of processors implementing a computing function, for example, a combination of one or more microprocessors, or a combination of a DSP and a microprocessor.

In a possible manner, the communication unit 1703 may be the transceiver 503 shown in FIG. 3, or may be a transceiver circuit, a radio frequency component, or the like.

In a possible manner, the storage unit 1701 may be the memory 502 shown in FIG. 3.

An embodiment of this application further provides an electronic device, including one or more processors and one or more memories. The one or more memories are coupled to the one or more processors. The one or more memories are configured to store computer program code, and the computer program code includes computer instructions. When the one or more processors execute the computer instructions, the electronic device is enabled to perform the foregoing related method steps, to implement the method in the foregoing embodiment.

An embodiment of this application further provides a chip system, including a processor. The processor is coupled to a memory. The memory is configured to store a program or instructions. When the program or the instructions is/are executed by the processor, the chip system is enabled to implement the method according to any one of the foregoing method embodiments.

Optionally, there may be one or more processors in the chip system. The processor may be implemented through hardware, or may be implemented through software. When the processor is implemented by using the hardware, the processor may be a logic circuit, an integrated circuit, or the like. When the processor is implemented by using the software, the processor may be a general-purpose processor, and is implemented by reading software code stored in the memory.

Optionally, there may also be one or more memories in the chip system. The memory may be integrated with the processor, or may be disposed separately from the processor. This is not limited in this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in this application.

For example, the chip system may be a field programmable gate array (field programmable gate array, FPGA), an application-specific integrated chip (application-specific integrated circuit, ASIC), a system on chip (system on chip, SoC), a central processing unit (central processing unit, CPU), a network processor (network processor, NP), a digital signal processor (digital signal processor, DSP), a micro controller unit (micro controller unit, MCU), a programmable controller (programmable logic device, PLD), or another integrated chip.

It should be understood that the steps in the foregoing method embodiments may be completed by using a hardware integrated logic circuit or instructions in a form of software in the processor. The steps of the methods disclosed with reference to embodiments of this application may be directly performed by a hardware processor, or may be performed by a combination of hardware in the processor and a software module.

An embodiment of this application further provides a computer-readable storage medium. The computer-readable storage medium stores computer instructions. When the computer instructions are run on an electronic device, the electronic device is enabled to perform the related method steps, to implement the method in the foregoing embodiment.

An embodiment of this application further provides a computer program product. When the computer program product is run on a computer, the computer is enabled to perform the foregoing related steps to implement the method in the foregoing embodiment.

In addition, an embodiment of this application further provides an apparatus. The apparatus may be specifically a component or a module. The apparatus may include a processor and a memory that are connected to each other. The memory is configured to store computer-executable instructions. When the apparatus runs, the processor may execute the computer-executable instructions stored in the memory, so that the apparatus is enabled to perform the method in the foregoing method embodiments.

The electronic device, the computer-readable storage medium, the computer program product, or the chip provided in the embodiments of this application is configured to perform the corresponding method provided above. Therefore, for beneficial effects that can be achieved, refer to the beneficial effects of the corresponding method provided above. Details are not described herein again.

It may be understood that, to implement the foregoing functions, the electronic device includes a corresponding hardware and/or software module for performing each function. In combination with example algorithm steps described in embodiments disclosed in this specification, this application may be implemented by hardware or a combination of hardware and computer software. Whether a function is performed by hardware or hardware driven by computer software depends on particular applications and design constraints of the technical solution. A person skilled in the art may use different methods to implement the described functions for each particular application with reference to embodiments, but it should not be considered that the implementation goes beyond the scope of this application.

In this embodiment, the electronic device may be divided into function modules based on the foregoing method examples. For example, each function module corresponding to each function may be obtained through division, or two or more functions may be integrated into one processing module. The integrated module may be implemented in a form of hardware. It should be noted that, in this embodiment, division into the modules is an example, is merely logical function division, and may be other division during actual implementation.

Based on the foregoing descriptions of the implementations, a person skilled in the art may clearly understand that for the purpose of convenient and brief descriptions, division into the foregoing function modules is merely used as an example for description. During actual application, the foregoing functions can be allocated to different function modules for implementation based on a requirement, that is, an inner structure of an apparatus is divided into different function modules to implement all or some of the functions described above. For a specific working process of the foregoing system, apparatus, and unit, refer to a corresponding process in the foregoing method embodiments, and details are not described herein again.

In the several embodiments provided in this application, it should be understood that the disclosed methods may be implemented in other manners. For example, the described terminal device embodiment is merely an example. For example, the module or unit division is merely logical function division, and may be other division during actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the modules or units may be implemented in electronic, mechanical, or other forms.

The units described as separate parts may or may not be physically separate, and parts displayed as units may or may not be physical units, that is, may be located in one location, or may be distributed on a plurality of network units. Some or all of the units may be selected depending on actual requirements to achieve the objectives of the solutions in the embodiments.

In addition, function units in embodiments of this application may be integrated into one processing unit, each of the units may exist alone physically, or two or more units are integrated into one unit. The integrated unit may be implemented in a form of hardware, or may be implemented in a form of a software function unit.

When the integrated unit is implemented in the form of the software function unit and sold or used as an independent product, the integrated unit may be stored in a computer-readable storage medium. Based on such an understanding, the technical solutions of this application essentially, or the part contributing to the current technology, or all or some of the technical solutions may be implemented in the form of a software product. The computer software product is stored in a storage medium and includes several instructions for instructing a computer device (which may be a personal computer, a server, or a network device) or a processor to perform all or some of the steps of the methods described in embodiments of this application. The foregoing storage medium includes any medium that can store program instructions, for example, a flash memory, a removable hard disk, a read-only memory, a random access memory, a magnetic disk, or an optical disc.

The foregoing description is merely a specific implementation of this application, but is not intended to limit the protection scope of this application. Any variation or replacement within the technical scope disclosed in this application shall fall within the protection scope of this application. Therefore, the protection scope of this application shall be subject to the protection scope of the claims.

## Claims

1. An exercise guidance method, wherein the method is applied to an electronic device, and the method comprises:
receiving a first operation input by a user, and displaying a first interface, wherein the first interface comprises information about a first exercise ability of the user;
receiving a second operation input by the user, and displaying a second interface, wherein the second interface comprises one or more movement distances;
receiving a target movement distance input by the user on the second interface; and
displaying a third interface, wherein the third interface comprises a plurality of exercise levels and an exercise performance range corresponding to each exercise level, and the exercise performance range is determined based on the corresponding exercise level, the target movement distance, and the first exercise ability.

2. The method according to claim 1, wherein the method further comprises: receiving a first target exercise level input by the user on the third interface, and displaying a first exercise interface, wherein the first target exercise level corresponds to a first exercise performance range.

3. The method according to claim 1 or 2, wherein after the displaying a first interface, the method further comprises:
displaying a fourth interface, wherein the fourth interface comprises a first control used to modify the first exercise ability; and
receiving an operation performed by the user on the first control, and displaying a fifth interface, wherein the fifth interface comprises an option used to modify the first exercise ability.

4. The method according to claim 3, wherein the first interface comprises heart rate information of the user, and the fourth interface comprises a second control used to modify the heart rate information.

5. The method according to any one of claims 1 to 4, wherein the method further comprises:
displaying a second exercise interface in response to a third operation input by the user, wherein the second exercise interface comprises a current exercise parameter of the user, an annular indication region located on an interface edge, and a pointer; and the pointer points to the annular indication region, the annular indication region comprises a first indication region, a second indication region, and a third indication region, a first end of the first indication region is connected to a second end of the second indication region, and a second end of the first indication region is connected to a first end of the third indication region, wherein
the first indication region comprises an annular region corresponding to a first parameter range, and the first parameter range is a recommended range of the electronic device for the exercise parameter; and the second indication region comprises an annular region greater than a maximum value of the first parameter range, the third indication region comprises an annular region less than a minimum value of the first parameter range, and a location at which the pointer points to the annular indication region indicates whether the current exercise parameter of the user exceeds the first parameter range.

6. The method according to claim 5, wherein when the current exercise parameter of the user exceeds the first parameter range, the pointer points to the second indication region or the third indication region, the second exercise interface further comprises first prompt information, and the first prompt information is used to prompt the user that the current exercise parameter exceeds the first parameter range.

7. The method according to claim 5 or 6, wherein the second exercise interface further comprises the first parameter range.

8. The method according to any one of claims 1 to 7, wherein the method further comprises:
obtaining a first exercise parameter of the user in an exercise process; and
displaying a third exercise interface when the first exercise parameter exceeds the first parameter range for more than preset duration, wherein the third exercise interface is used to recommend the user to adjust a second exercise parameter, and the first parameter range is a recommended range of the electronic device for the first exercise parameter.

9. The method according to claim 8, wherein the first exercise parameter comprises a heart rate, and the second exercise parameter comprises a pace; and
the displaying a third exercise interface when the first exercise parameter exceeds the first parameter range for more than preset duration comprises:
displaying the third exercise interface when the heart rate is greater than the maximum value of the first parameter range, wherein the third exercise interface is used to recommend the user to reduce the pace; or
displaying the third exercise interface when the heart rate is less than the minimum value of the first parameter range, wherein the third exercise interface is used to recommend the user to increase the pace.

10. The method according to claim 8 or 9, wherein the displaying a third exercise interface comprises:
displaying the third exercise interface within first duration, and displaying a fifth exercise interface after the first duration, wherein user interface UI effect of the fifth exercise interface is different from UI effect of the third exercise interface.

11. The method according to claim 8 or 9, wherein after the displaying a third exercise interface, the method further comprises:
displaying a sixth exercise interface in response to a fourth operation input by the user, wherein UI effect of the sixth exercise interface is different from UI effect of the third exercise interface.

12. The method according to any one of claims 1 to 4, wherein the method further comprises:
displaying a second exercise interface, wherein the second exercise interface comprises a first parameter range, and the first parameter range is related to a first physical status of the user at a first moment in an exercise process; and
displaying a fourth exercise interface, wherein the fourth exercise interface comprises a second parameter range, and the second parameter range is related to a second physical status of the user at a second moment in the exercise process, wherein the first parameter range is different from the second parameter range, and the first moment is different from the second moment.

13. The method according to claim 12, wherein the first physical status is determined based on an exercise parameter of the user at the first moment, and the second physical status is determined based on an exercise parameter of the user at the second moment.

14. The method according to claim 13, wherein the exercise parameter comprises a heart rate and/or a pace.

15. The method according to any one of claims 12 to 14, wherein the method further comprises: presenting second prompt information when a difference between the second parameter range and the first parameter range is greater than a threshold.

16. The method according to any one of claims 12 to 15, wherein the second exercise interface comprises estimated duration for completing the target movement distance, and the estimated duration is determined based on the first physical status of the user, the target movement distance, and the first exercise performance range.

17. The method according to any one of claims 5 to 16, wherein the second exercise interface comprises one or more of the following: a current finished movement distance, current exercise duration, and a current exercise parameter.

18. The method according to any one of claims 5 to 17, wherein the second exercise interface further comprises a first icon, a second icon, and a progress bar, the first icon indicates, on the progress bar, a location of the current finished movement distance of the user in the target movement distance, and the second icon indicates, on the progress bar, a location that is of the user in the target movement distance and that is expected based on the first exercise performance range.

19. The method according to any one of claims 1 to 18, wherein the method further comprises:
displaying a sixth interface, wherein the sixth interface comprises third prompt information, and the third prompt information is used to prompt the user to do exercise to obtain the first exercise ability.

20. The method according to any one of claims 2 to 19, wherein the method further comprises:
displaying a seventh interface, wherein the seventh interface comprises the first target exercise level, and the first target exercise level corresponds to a second exercise performance range; the second exercise performance range is determined based on the first target exercise level, the target movement distance, and a second exercise ability of the user; and the second exercise performance range is different from the first exercise performance range, and the first exercise ability is different from the second exercise ability.

21. The method according to any one of claims 2 to 20, wherein the method further comprises:
displaying a seventh exercise interface in the exercise process of the user in response to a fifth operation input by the user, wherein the seventh exercise interface comprises a third control used to modify an exercise level.

22. The method according to claim 21, wherein after the displaying a seventh exercise interface, the method further comprises:
receiving an operation performed by the user on the third control, and displaying an eighth exercise interface, wherein the eighth exercise interface comprises information about a plurality of exercise levels; and
receiving a second target exercise level input by the user on the eighth exercise interface, and displaying a ninth exercise interface, wherein the second target exercise level is different from the first target exercise level.

23. The method according to any one of claims 2 to 22, wherein after the receiving a first target exercise level input by the user on the third interface, the method further comprises:
presenting fourth prompt information on the third interface, wherein the fourth prompt information is used to prompt the user to select a third target exercise level, and the third target exercise level is different from the first target exercise level.

24. The method according to any one of claims 1 to 23, wherein before the receiving a first operation input by a user, the method further comprises:
displaying an eighth interface, wherein the eighth interface comprises a plurality of exercise types; and
receiving a target exercise type input by the user on the eighth interface, and displaying a ninth interface, wherein
the receiving a first operation input by a user comprises receiving the first operation that is input by the user and that is performed on the ninth interface.

25. The method according to any one of claims 1 to 24, wherein the exercise comprises any one of the following types of exercise: running, swimming, and cycling.

26. The method according to any one of claims 1 to 25, wherein the exercise comprises running, the first exercise ability is a running ability index, and the running ability index is related to an exercise record of the user.

27. The method according to any one of claims 1 to 26, wherein the electronic device comprises a wearable device or a mobile terminal device.

28. An electronic device, comprising a memory and a processor, wherein
the memory is configured to store computer instructions; and
the processor is coupled to the memory and is configured to invoke the computer instructions in the memory, to perform the method according to any one of claims 1 to 27 by using the transceiver.

29. A computer-readable storage medium, wherein the computer-readable storage medium stores computer-executable instructions, and the computer-executable instructions are invoked by a computer to perform the method according to any one of claims 1 to 27.

30. A computer program product, comprising instructions, wherein when the instructions are run on a computer, the computer is enabled to perform the method according to any one of claims 1 to 27.

31. A chip, wherein the chip is coupled to a memory and is configured to read and execute program instructions stored in the memory, to implement the method according to any one of claims 1 to 27.
